# EUROPEAN PATENT APPLICATION

(11) **EP 3 808 763 A1**
(43) Date of publication of application: **21.04.2021**
(21) Application number: 19382910.8
(22) Date of filing: 17.10.2019
(51) Int. Cl.: C07K 14/47, A61K 38/17

(54) **COMPOUNDS FOR IMMUNOMODULATION**

(71) Applicant: Fundació Institut d'Investigació Biomèdica de Bellvitge (IDIBELL), 08908 L'Hospitalet de Llobregat (ES)
(72) Inventor: ARAN PERRAMON, Josep M., E-08100 Mollet del Vallès, Barcelona (ES); LUQUE GÓMEZ, Ana, E-08016 Barcelona (ES); SERRANO SANTACRUZ, Inmaculada, E-08020 Barcelona (ES)
(74) Representative: ABG Intellectual Property Law, S.L.

(57) **Abstract**

The invention relates to compounds based on a recombinant form of the CCP6 region of C4BP and an oligomerization domain for use in the prevention and/or treatment of immunological diseases. Moreover, the invention relates to methods for obtaining tolerogenic dendritic cells and tolerogenic macrophages using the compounds of the invention and to the cells obtained by these methods and their therapeutic uses.

## Description

### FIELD OF THE INVENTION

The invention relates to the field of immunology and, more in particular, to compounds based on a recombinant form of the CCP6 region of C4BP which are capable of inducing a tolerogenic state in mononuclear phagocytes and to the uses thereof for the prevention and/or treatment of diseases characterized by an undesired activation of the immune system.

### BACKGROUND OF THE INVENTION

The mononuclear phagocyte system (MPS) refers to a network of myeloid cells that share a number of functional features, including amongst others, the ability to secrete chemokine and cytokine mediators, resulting in the migration and activation of immune and nonimmune cells, as well as their own migration. The MPS comprises dendritic cells, macrophages and monocytes.

Dendritic cells (DC) are the professional APC of the immune system. At their immature stage, DC take up extracellular antigens by means of phagocytosis or pinocytosis and process the antigens to peptides in the endocytic compartment such as endosomes and phagosomes, where peptides are bound to MHC class II molecules. They also have the unique ability of loading the peptides from exogenous proteins to the MHC class I pathway of presentation, a process called "cross-presentation". Given the appropriate differentiation signals (such as microbial products), immature DC may develop into an immunogenic DC which is equipped with the ability to activate both naive and memory T cells. On the other side of the spectrum immature DC can also differentiate into a tolerogenic phenotype, which is thought to play a crucial role in the maintenance of peripheral tolerance (Steinman, Ann. Rev. Immunol. 2003, 21: 685-711; Morelli, Immunol Rev 2003: 125-146).

Numerous protocols for the generation of tolerogenic DC *in vitro* have been described (Xiao et al., J. lmmunother. 2006 (29) 465-471). The most well-characterized methods utilise pharmacological mediators (such as immunosuppressive drugs including vitamin D3 analogues, glucocorticoids, oestrogen), cytokines and growth factors (such as IL-10, TGF-beta, IL-4 and IFN-gamma) or genetic engineering, either to suppress the expression of T cell co-stimulatory molecules (such as CD86 and CD40) or to enhance the expression of T cell inhibitory molecules (such as CTLA-4 and indoleamine 2,3- dioxygenase).

Recently, it has been shown that the physiological isoform α₇β₀ of the complement regulator C4b-binding protein induces a semimature, anti-inflammatory state in dendritic cells (Olivar R. et al. 2013. J. Immunol., 190:2857-2872).

On the other hand, several protocols for the generation of tolerogenic or regulatory macrophages *in vitro* have been described including the use of glucocorticoids such as dexamethasone, IL-10, or TGF-β, or combinations of M-CSF+IFNγ or M-CSF+LPS, amongst other compounds (Mosser DM and Edwards JP. 2008. Nat. Rev. Immunol. 8:958-969; Zheng G. et al. 2013. Eur. J. Immunol. 43:219-227; Navarro-Barriuso J. et al. 2018. Front. Immunol. 9:2062; Foey AD. 2014. Immune response activation, Chapter 5: Macrophages: masters of immune activation, suppression and deviation, Publisher: InTech, Editors: Guy Huynh Thien Duc, pp.121-149).

To date, the majority of therapies approved by the US FDA for autoimmune disease have focused on the systemic inhibition of immune inflammatory activity. Although nonspecific immune suppression is partially effective in inhibiting auto-reactive immune cell function, the drugs used to suppress the immune response have numerous side effects and continuous therapy is not conductive to long-term host survival. Thus, it is desirable to develop auto-antigen-specific treatments that allow for the specific blockade of the deleterious effects of self-reactive immune cell function, while maintaining the ability of the immune system to clear infection. Hence, there is a strong need for methods that generate properly equipped DC that can efficiently induce antigen-specific immune tolerance.

In addition, *ex vivo* generated DC with appropriate tolerogenic function could also be implemented as therapeutic vaccine in treatment of allergy and for induction of transplant tolerance. As with immunotherapy for autoimmune diseases, efficient suppression of harmful immune responses involves the tolerance induction of both CD4+ and CD8+ T cells. Therefore, one can expect that *ex vivo* generated tolerogenic DC should have the same characteristics for treating autoimmune diseases, allergy and for prevention of graft rejection.

However, new and alternative methods for the production of tolerogenic dendritic cells and macrophages having a distinct tolerogenic phenotype and having expression of tolerogenic determinants is always a recurring object of research in this field.

### SUMMARY OF THE INVENTION

In a first aspect, the invention relates to a recombinant polypeptide comprising the CCP6 domain of the C4BP alpha chain or a functionally equivalent variant thereof and an oligomerization domain wherein said polypeptide does not comprise CCP1, CCP2, CCP3, CCP4, CCP5, CCP7 nor CCP8 domains of the C4BP alpha chain.

In a second aspect, the invention relates to an homooligomer of at least six recombinant polypeptides according to the first aspect of the invention.

In a third aspect, the invention relates to a polynucleotide encoding a recombinant polypeptide according to the first aspect of the invention.

In a fourth aspect, the invention relates to a vector comprising a polynucleotide according to the third aspect of the invention.

In a fifth aspect, the invention relates to a host cell comprising the vector of the fourth aspect of the invention.

In a sixth aspect, the invention relates to a pharmaceutical composition comprising a recombinant polypeptide according to the first aspect of the invention, an homooligomer according to the second aspect of the invention, a polynucleotide according to the third aspect of the invention, a vector according to the fourth aspect of the invention, or a host cell according to the fifth aspect of the invention and a pharmaceutically acceptable carrier.

In further aspects, the invention relates to a recombinant polypeptide, an homooligomer, a polynucleotide, a vector, a host cell or a pharmaceutical composition according to the invention for use in medicine, particularly for use in the prevention and/or treatment of an immunological disease caused by an undesired activation of the immune system.

In another aspect, the invention relates to an *in vitro* method for the generation of a population of tolerogenic dendritic cells comprising the steps of:
(i) incubating a population of dendritic precursor cells under conditions adequate for the formation of a population of immature dendritic cells, and
(ii) incubating the population of immature dendritic cells obtained in step
(i) under conditions adequate for the formation of mature dendritic cells
wherein steps (i) and/or (ii) are carried out in the presence of a composition of matter selected from the group consisting of:
(a) a polypeptide according to the invention,
(b) an homooligomer according to the invention,
(c) a polynucleotide according to the invention, and
(d) a vector according to the invention.

In another aspect, the invention relates to an *in vitro* method for the generation of a population of tolerogenic macrophages comprising the steps of:
(i) incubating a population of macrophage precursor cells under conditions adequate for the formation of a population of immature macrophages, and
(ii) incubating the population of immature macrophages obtained in step (i) under conditions adequate for the formation of mature macrophages
wherein steps (i) and/or (ii) are carried out in the presence of a composition of matter selected from the group consisting of:
(a) a polypeptide according to the invention,
(b) an homooligomer according to the invention,
(c) a polynucleotide according to the invention, and
(d) a vector according to the invention.

In yet another aspects, the invention relates to a tolerogenic dendritic cell or a tolerogenic macrophage obtained by the methods of the invention, to a cell population comprising at least 80% of said cells, to a pharmaceutical composition comprising a cell or population of the invention, and to the uses of the cells of the invention in medicine, particularly for the prevention and/or treatment of an immunological disease caused by an undesired activation of the immune system.

### DESCRIPTION OF THE FIGURES

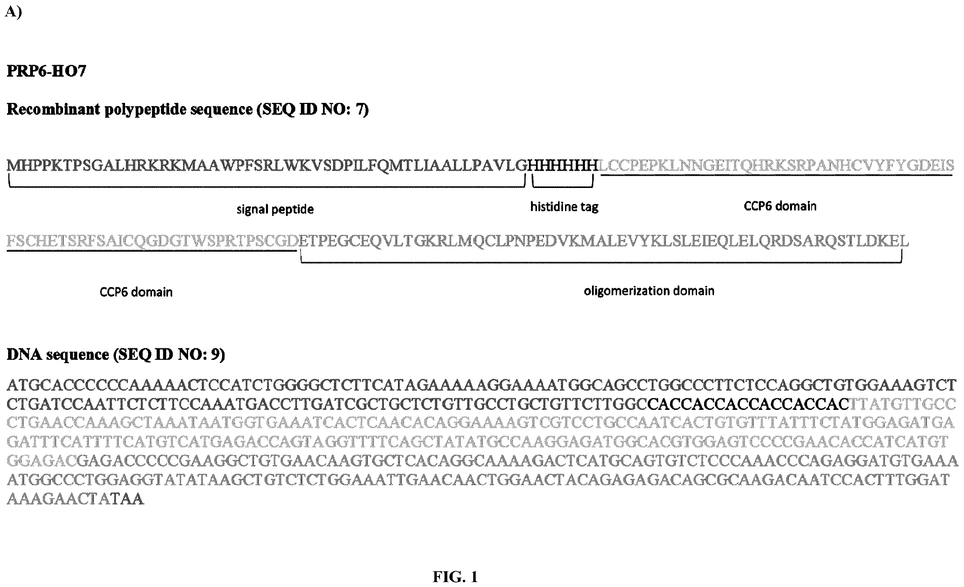

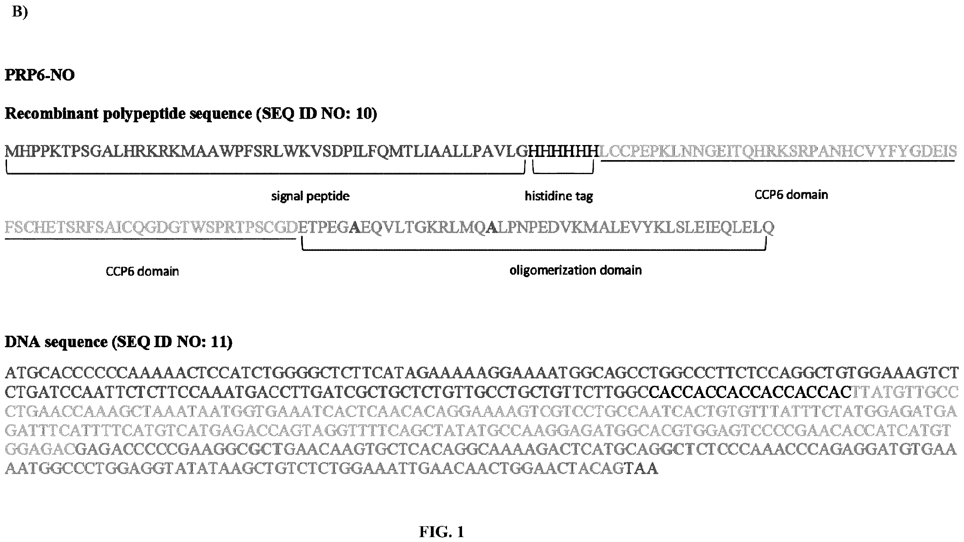

**Figure 1****. Immature sequences of PRP6-HO7 and PRP6-NO. (A)** Peptide and DNA immature sequences of PRP6-HO7. The figure shows the sequences of the signal peptide, histidine tag, CCP6 domain and oligomerization domain. The predicted molecular weight of PRP6-HO7 was 19.6 kDa and 14.3 kDa without the signal peptide. **(B)** Peptide and DNA immature sequences of PRP6-NO. The figure shows the sequences of the signal peptide, histidine tag, CCP6 domain and oligomerization domain. In PRP6-NO, the original oligomerization sequence was modified by substituting two cysteine residues by alanine (in bold) and by removing the last 13 C-terminal amino acids. The predicted molecular weight of PRP6-NO was 18 kDa and 12.7 kDa without the signal peptide.

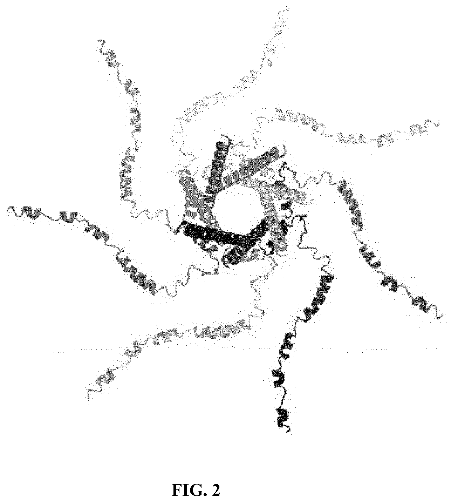

**Figure 2****. Molecular modeling of PRP6-HO7.** PRP6-HO7 homo-oligomer structure prediction from its monomer sequence/structure using the GalaxyHomomer server (http://galaxy.seoklab.org/homomer) (Baek et al. (2017) Nucleic Acids Res. 45: W320-W324).

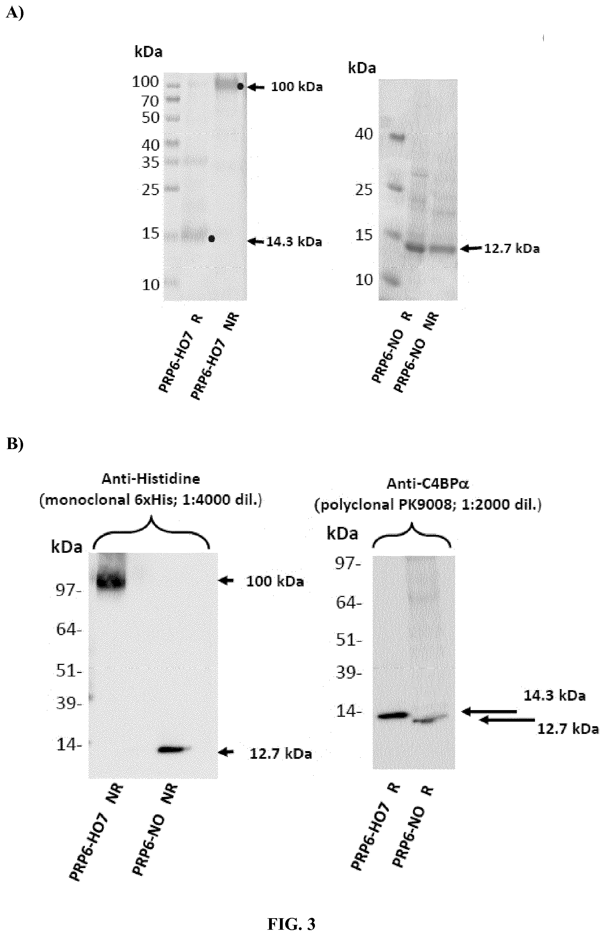

**Figure 3****. Visualization of PRP6-HO7 and PRP6-NO proteins by PAGE. (A)** 12% SDS-PAGE analysis and Coomassie Blue staining of purified PRP6-HO7 and PRP6-NO proteins under reducing (R) (monomers, 14.3 kDa and 12.7 kDa, respectively), and non-reducing (NR) (oligomer, 100 kDa) conditions. **(B)** Bis-Tris 4-12% SDS-PAGE and Western analysis of purified PRP6-HO7 and PRP6-NO proteins under non-reducing conditions (NR; left) and probed with an anti-His MoAb, and under reducing conditions (R; right) and probed with an anti-C4BPα chain polyclonal antibody.

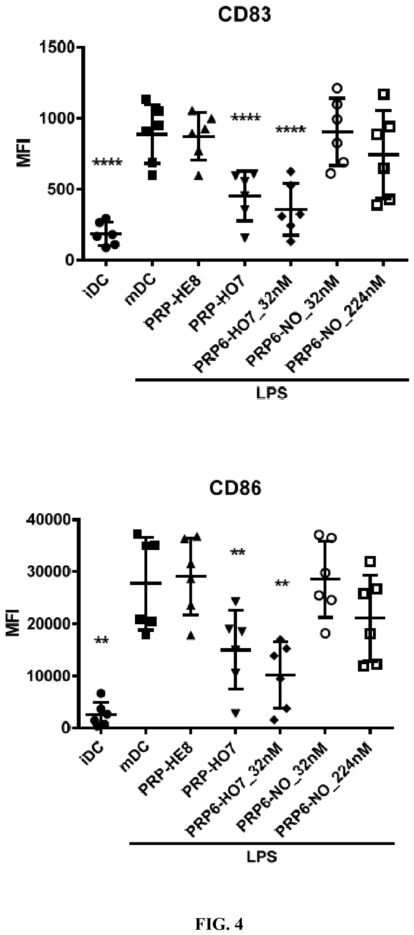

**Figure 4****. PRP6-HO7, but not PRP6-NO, downregulates CD83 and CD86 expression in LPS-activated DCs.** Human Mo-DCs were incubated throughout their differentiation process with PRP-HO7 and PRP-HE8 (corresponding to C4BP(β+), an inactive isoform of PRP (C4BP)) both at 12 nM, PRP6-HO7 (32 nM) and PRP6-NO (32 nM and 224 nM). DC maturation was achieved by LPS treatment (see Materials and Methods for details). Cells were then collected, washed, and analyzed by flow cytometry for cell surface expression of the activation marker CD83 and the co-stimulatory molecule CD86. MFI, median fluorescence intensity for the different surface markers. LPS, lipopolysaccharide. iDC, untreated, immature DCs; mDC, untreated, LPS-matured DCs. The results shown are the mean ± SD from 6 independent experiments (**, p < 0.01; ****, p < 0.0001 respect to mDC).

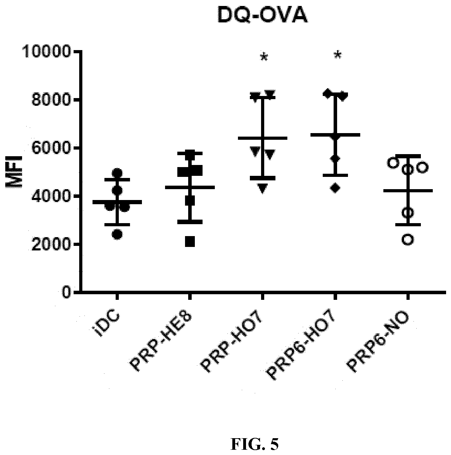

**Figure 5****. PRP6-HO7, but not PRP6-NO, treatment increases the endocytic activity of human iDCs.** The endocytic activity of iDCs was assessed by flow cytometry, measuring the fluorescent DQ-OVA internalization and processing (receptor-mediated endocytosis), at the differentiation stage. Monocytes were differentiated either untreated (iDC) or treated with PRP-HE8 and PRP-HO7 (both at 12 nM), and with PRP6-HO7 and PRP6-NO (both at 32 nM). MFI, median fluorescence intensity. iDC, untreated, immature DCs. Data shown are the mean MFI ± SD from 5 independent experiments (*, p < 0.05 compared to iDC).

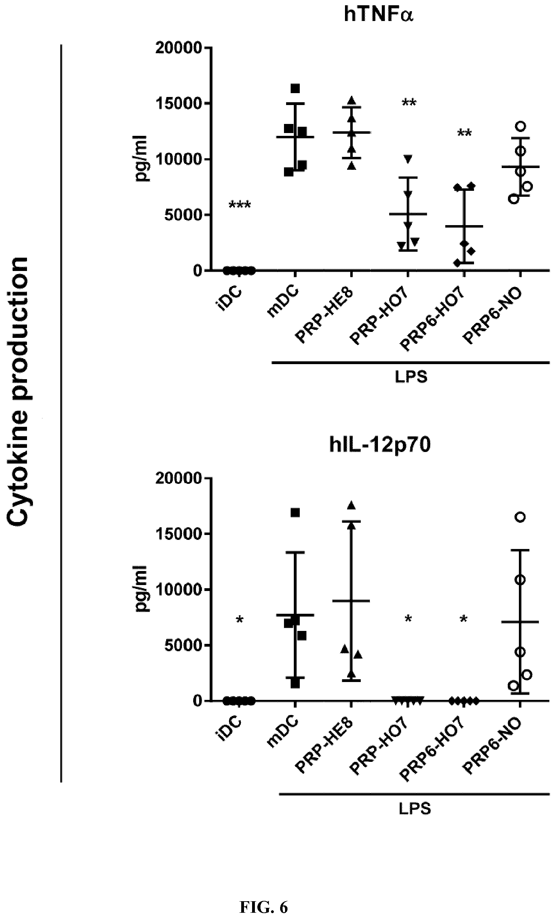

**Figure 6****. PRP6-HO7, but not PRP6-NO, modulates pro-inflammatory cytokine production in LPS-activated DCs.** Mo-DCs treated with the different PRP-based proteins: PRP-HE8, PRP-HO7 (both at 12 nM; 5 µg/ml), and PRP6-HO7, PRP6-NO (both at 32 nM; 3 µg/ml) were matured with LPS and the concentrations of IL-12p70 and TNF-α were analyzed in the respective supernatants by ELISA. Results shown are the mean ± SD from 5 independent experiments, performed in duplicate. LPS, lipopolysaccharide; iDC, untreated, immature DCs; mDC, untreated, LPS-matured DCs. (*, p < 0.05; **, p < 0.01; ***, p < 0.001 respect to mDC).

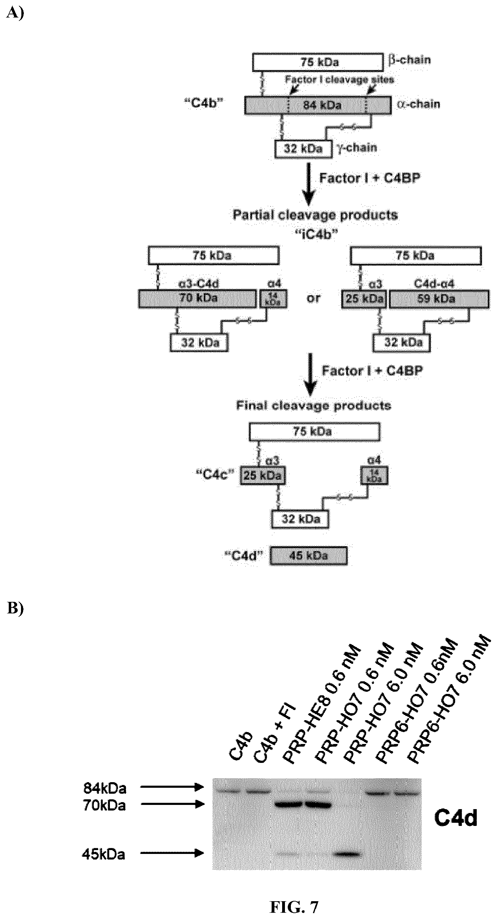

**Figure 7****. PRP6-HO7 is devoid of complement inhibitory activity. (A)** Schematic drawing of PRP-HE8 and PRP-HO7 cofactor activity for factor I-mediated cleavage of C4b. Cleavage of C4b by factor I requires the presence of a specific cofactor, in this case PRP-HE8/PRP-HO7. Factor I cleaves the α'-chain of C4b at two sites indicated by arrows. Cleavage at either one of the two sites generates fragments, α3-C4d (70 kDa) and α4 (14 kDa) or α3 (25 kDa) and C4d-α4 (59 kDa), which are seen after SDS-PAGE under reducing conditions. Further cleavage of the α'-chain of C4b yields the large soluble C4c (146 kDa) fragment and the small C4d (45 kDa) fragment which remains associated with targets. **(B)** Cofactor activity assay. PRP-HE8, PRP-HO7 and PRP6-HO7, at the indicated concentrations (lanes 3-7), were incubated with C4b (8.9 µg/ml) followed by addition of factor I (4.4 µg/ml). Reaction controls included C4b alone, and C4b + FI. All reactions were stopped after 30 min with SDS-reducing sample buffer. C4b cleavage fragments were separated by 4-12% SDS-PAGE under reducing conditions followed by Western blotting using an anti-C4d MoAb. C4b fragments are labeled at the left of the gel. Cofactor activity was confirmed by the appearance of α3-C4d (70 kDa) or C4d (45 kDa). FI, factor I. Results are representative of 3 independent experiments.

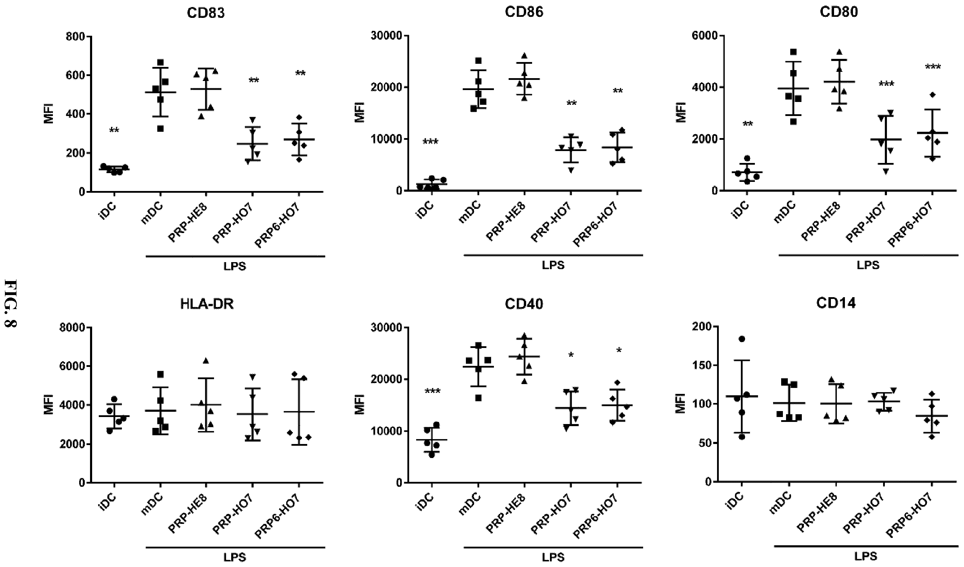

**Figure 8****. Surface markers expression from PRP6-HO7-treated and LPS-activated DCs.** Human Mo-DCs were incubated throughout their differentiation process with PRP-HO7 and PRP-HE8 both at 12 nM, and PRP6-HO7 (32 nM). DC maturation was achieved by LPS treatment. Cells were then collected, washed, and analyzed by flow cytometry for cell surface expression of CD83, CD86, HLA-DR, CD40, CD80, and CD14. MFI, median fluorescence intensity for the different surface markers. LPS, lipopolysaccharide. iDC, untreated, immature DCs; mDC, untreated, LPS-matured DCs. The results shown are the mean ± SD from 5 independent experiments (*, p < 0.05; **, p < 0.01; ***, p < 0.001 respect to mDC).

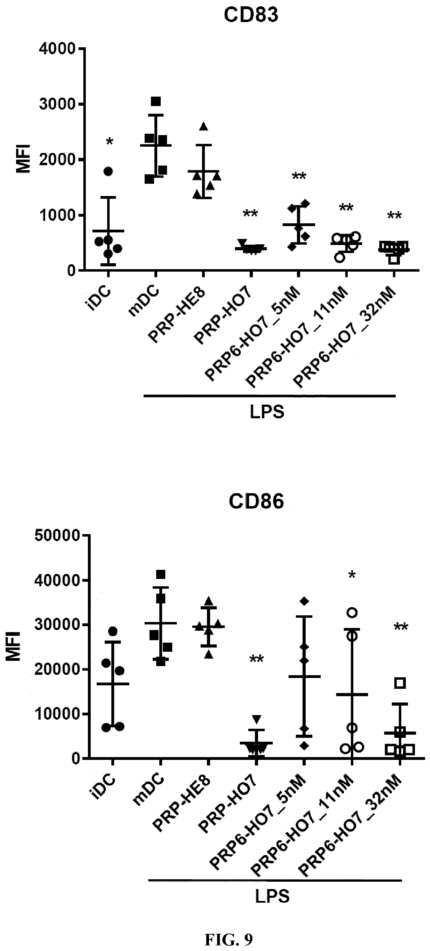

**Figure 9****. PRP6-HO7 downregulates CD83 and CD86 expression in LPS-activated DCs grown in 50% human serum.** Human Mo-DCs were in RPMI 1640 cell culture medium supplemented with 50% heat-inactivated human serum and incubated throughout their differentiation process with PRP-HO7 and PRP-HE8 both at 12 nM, and with the indicated concentrations of PRP6-HO7. DC maturation was achieved by LPS treatment. Cells were then collected, washed, and analyzed by flow cytometry for cell surface expression of CD83 and CD86. MFI, median fluorescence intensity for the different surface markers. LPS, lipopolysaccharide. iDC, untreated, immature DCs; mDC, untreated, LPS-matured DCs. The results shown are the mean ± SD from 5 independent experiments (*, p < 0.05; ** p < 0.01 respect to mDC).

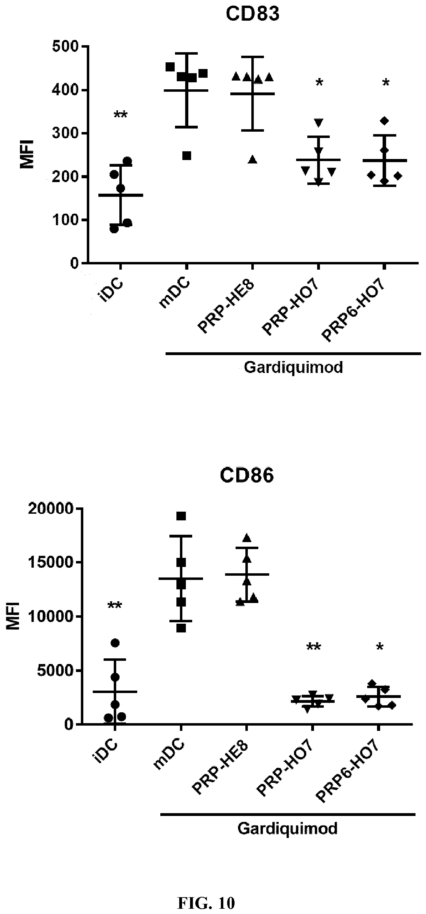

**Figure 10****. PRP6-HO7 downregulates CD83 and CD86 surface marker expression in gardiquimod-activated DCs.** Human Mo-DCs were incubated throughout their differentiation process with PRP-HE8 and PRP-HO7 both at 12 nM, and with PRP6-HO7 (32 nM). DC maturation was achieved by gardiquimod (TLR7 agonist) treatment (see Materials and Methods for details). Cells were then collected, washed, and analyzed by flow cytometry for cell surface expression of CD83 and CD86. MFI, median fluorescence intensity for the different surface markers. iDC, untreated, immature DCs; mDC, untreated, gardiquimod-matured DCs. The results shown are the mean ± SD from 5 independent experiments (*, p < 0.05; ** p < 0.01 respect to mDC).

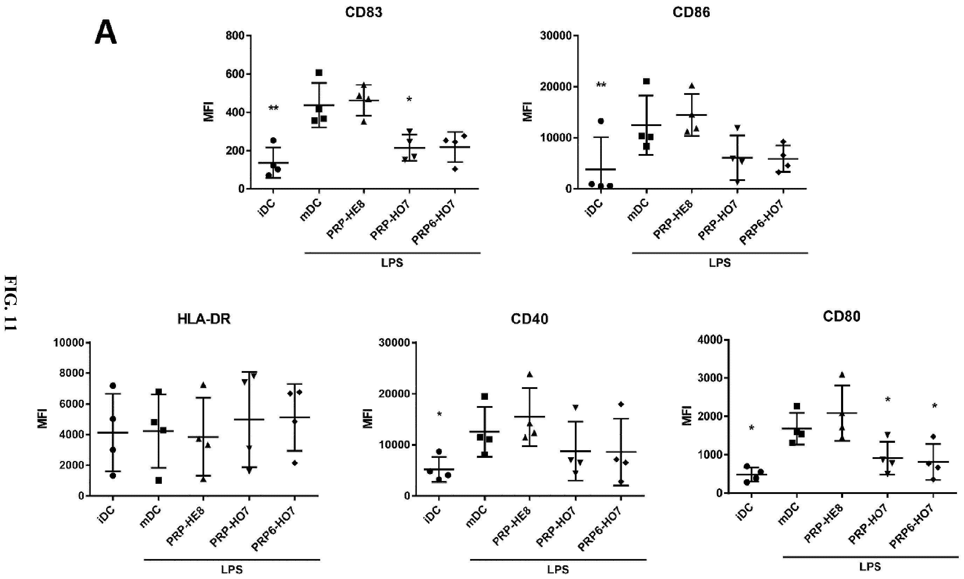

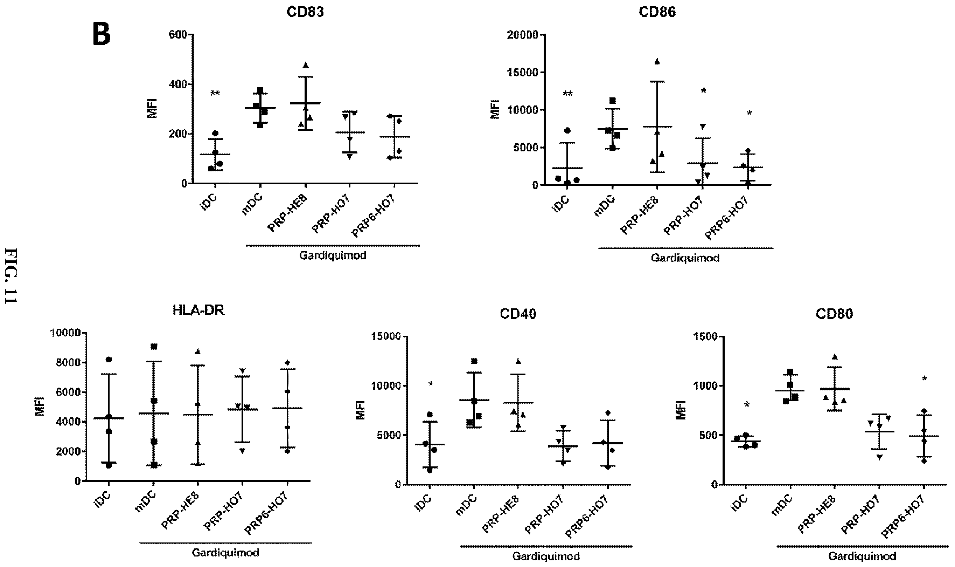

**Figure 11****. PRP6-HO7 down-regulates surface markers expression in both LPS- and gardiquimod-activated DCs isolated from lupus nephritis patients.** Human Mo-DCs from lupus nephritis patients were incubated throughout their differentiation process with PRP-HO7 and PRP-HE8 both at 12 nM, and PRP6-HO7 (32 nM). DC maturation was achieved either by LPS **(A)** or gardiquimod **(B)** treatment. Cells were then collected, washed, and analyzed by flow cytometry for cell surface expression of CD83, CD86, HLA-DR, CD40 and CD80. MFI, median fluorescence intensity for the different surface markers. LPS, lipopolysaccharide. iDC, untreated, immature DCs; mDC, untreated, LPS- or gardiquimod-matured DCs. The results shown are the mean ± SD from 4 independent experiments (*, p < 0.05; ** p < 0.01 respect to mDC).

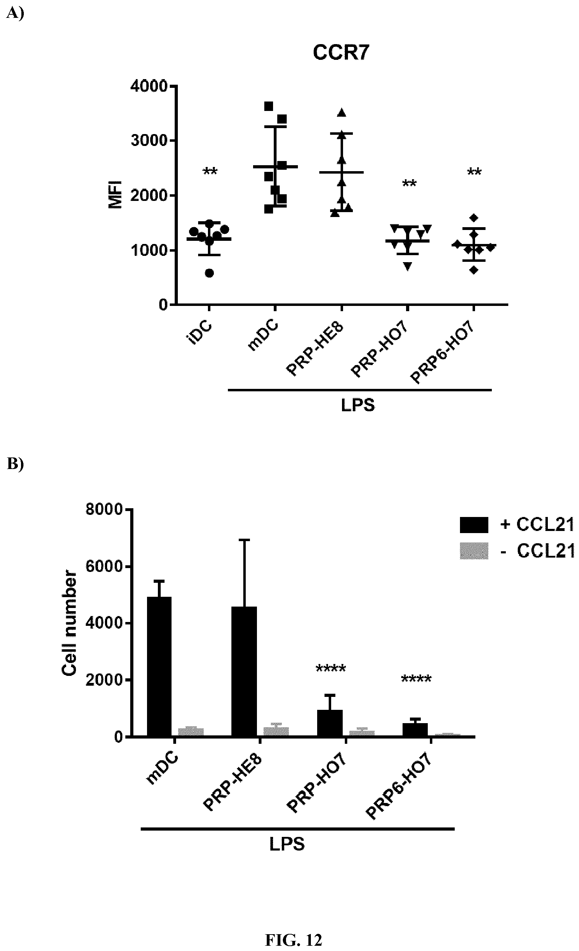

**Figure 12****. PRP6-HO7 down-regulates CCR7 expression and alters the chemotaxis of human DCs. (A)** CCR7 expression analysis of DCs at translational level. CCR7 surface expression on PRP based proteins-treated and LPS-matured DCs. The MFIs for CCR7 cell surface expression are indicated. Results shown are the mean ± SD from 7 independent experiments. (**, p < 0.01 compared to mDC). **(B)** Migration of untreated, PRP-HE8-treated (12 nM), PRP-HO7-treated (12 nM), and PRP6-HO7-treated (32 nM) DCs towards the chemokine CCL21 after LPS maturation was assessed in a transwell assay. Shown are the absolute numbers of LPS-matured DCs (mDC) migrated toward the lower CCL21-containing chamber after 2 h incubation (black columns). Spontaneous migration of DCs towards a lower chamber without CCL21 was also assessed (grey columns). Results are the mean ± SD from 7 independent experiments performed in duplicate. MFI, median fluorescence intensity for the CCR7 surface marker. LPS, lipopolysaccharide. iDC, untreated, immature DCs; mDC, untreated, LPS-matured DCs; PRP-HE8, PRP-HO7, and PRP6-HO7, PRP based proteins-treated, LPS-matured DCs. (****, p < 0.0001 compared to mDC).

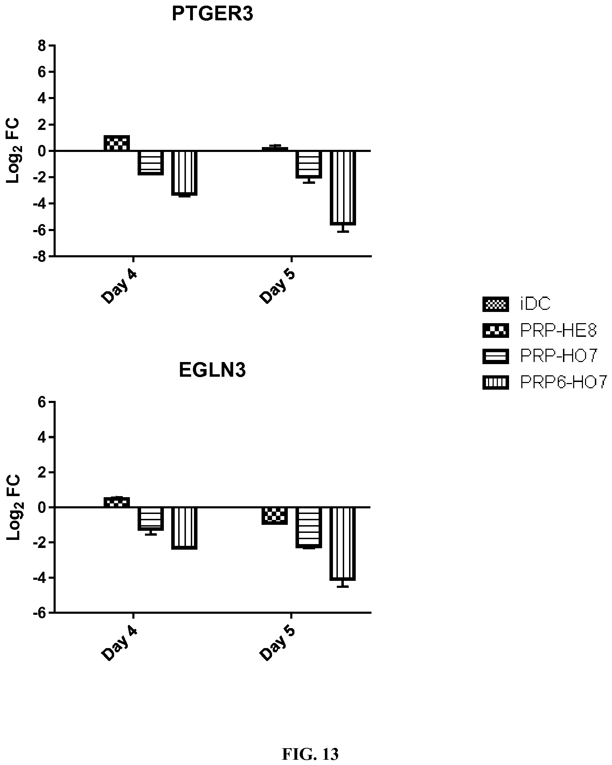

**Figure 13****. Human DCs exposed to PRP6-HO7 down-regulate *PTGER3* and *EGLN3* upon differentiation.** Expression of PTGER3 and EGLN3 transcripts from PRP-HE8-, PRP-HO7-, or PRP6-HO7-treated DCs relative to untreated, immature DCs (iDC) at days 4 and 5 of differentiation (n=1). Gene expression data are given as Log₂FC (FC, fold change). Relative expression data for the specified genes was obtained from total RNA extraction and *TaqMan* RT-qPCR validation. Log₂FC values are expressed as mean ± SD (2 technical replicates/sample) compared with untreated iDCs.

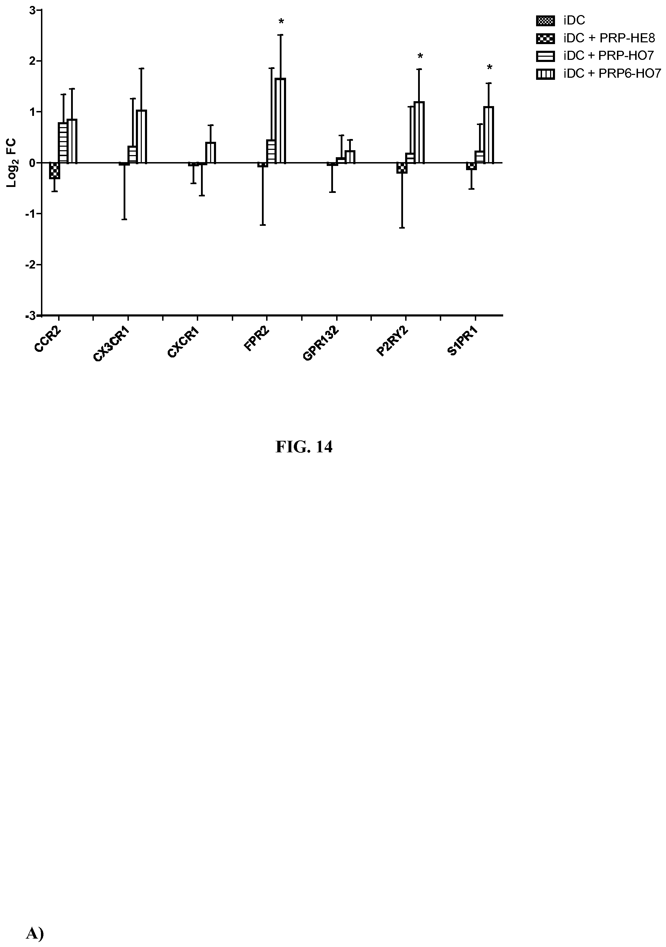

**Figure 14****. Human DCs exposed to PRP6-HO7 up-regulate *smell* receptors upon differentiation.** Expression of "find me" or *smell* receptor transcripts CCR2, CX3CR1, CXCR1, FPR2, GPR132, P2RY2, and S1PR1 from PRP-HE8-, PRP-HO7-, or PRP6-HO7-treated DCs relative to untreated, immature DCs (iDC) at day 5 of differentiation. Gene expression data are given as Log₂FC (FC, fold change). Relative expression data for the specified genes was obtained from total RNA extraction and *TaqMan* RT-qPCR validation. Log₂FC values are expressed as mean ± SD from 4 independent experiments (*, p < 0.05 compared to untreated iDC).

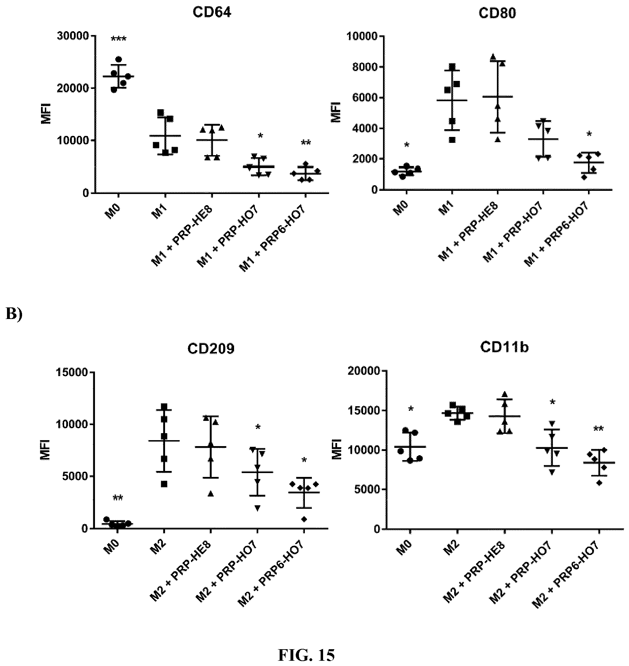

**Figure 15****. PRP6-HO7 prevents M1 and M2 polarization of human macrophages. (A)** Expression of M1 markers. **(B)** Expression of M2 markers. Human monocytes were incubated with PRP-HE8 and PRP-HO7 both at 12 nM, and with PRP6-HO7 (32 nM) throughout their differentiation to uncommited macrophages (M0) and further polarization to inflammatory (M1), or alternatively activated (M2) macrophages (see Materials and Methods for details). Cells were then collected, washed, and analyzed by flow cytometry for cell surface expression of CD64 and CD80 (M1 markers), or CD209 and CD1 1b (M2 markers). MFI, median fluorescence intensity for the different surface markers. M0, untreated, uncommited macrophages; M1/M2, untreated, M1- or M2-polarized macrophages. The results shown are the mean ± SD from 5 independent experiments (*, p < 0.05; ** p < 0.01; *** p < 0.001 respect to M1/M2).

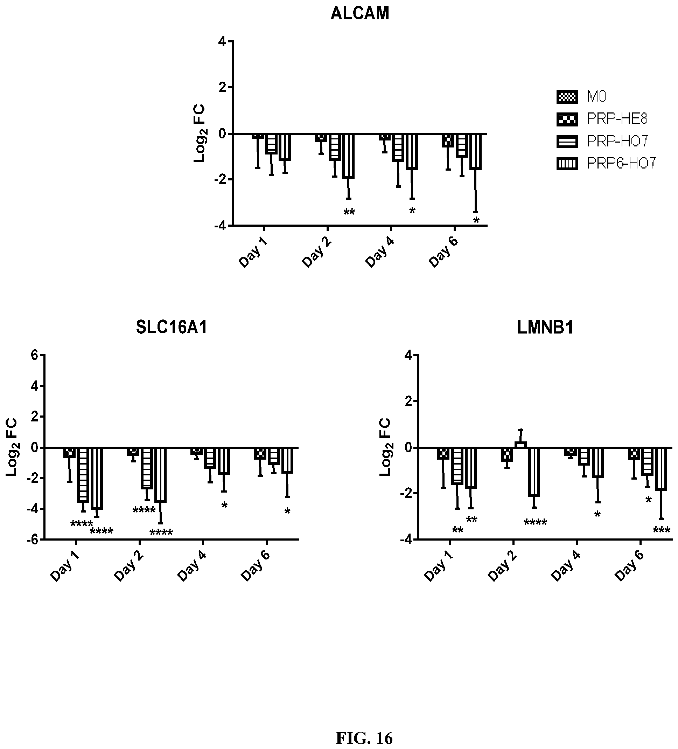

**Figure 16****. Human M0 macrophages exposed to PRP6-HO7 down-regulate *ALCAM, SLC16A1* and *LMNB1* upon differentiation.** Expression of ALCAM, LMNB1 and SLC16A1 transcripts from PRP-HE8-, PRP-HO7-, or PRP6-HO7-treated differentiating macrophages relative to untreated, uncommited (M0) macrophages at days 1, 2, 4 and 6 of differentiation. Gene expression data are given as Log₂FC (FC, fold change). Relative expression data for the specified genes was obtained from total RNA extraction and *TaqMan* RT-qPCR individual validation. Log₂FC values are expressed as mean ± SD from 5 independent experiments (*p < 0.05, **p < 0.01, ***p < 0.001, ****p < 0.0001 compared with untreated M0 macrophages).

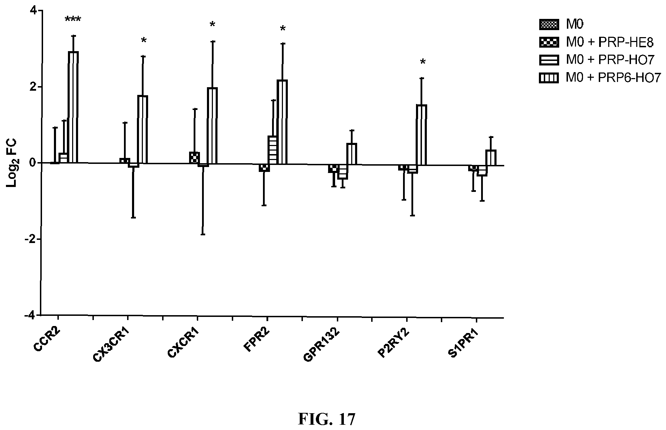

**Figure 17****. Differentiated human M0 macrophages exposed to PRP6-HO7 up-regulate *smell* receptors.** Expression of "find me" or *smell* receptor transcripts CCR2, CX3CR1, CXCR1, FPR2, GPR132, P2RY2, and S1PR1 from PRP-HE8-, PRP-HO7-, or PRP6-HO7-treated uncommited macrophages relative to untreated, uncommited macrophages (M0) at day 6 of differentiation. Gene expression data are given as Log₂FC (FC, fold change). Relative expression data for the specified genes was obtained from total RNA extraction and *TaqMan* RT-qPCR validation. Log₂FC values are expressed as mean ± SD from 4 independent experiments (*, p < 0.05; ***, p < 0.001 compared to untreated M0 macrophages).

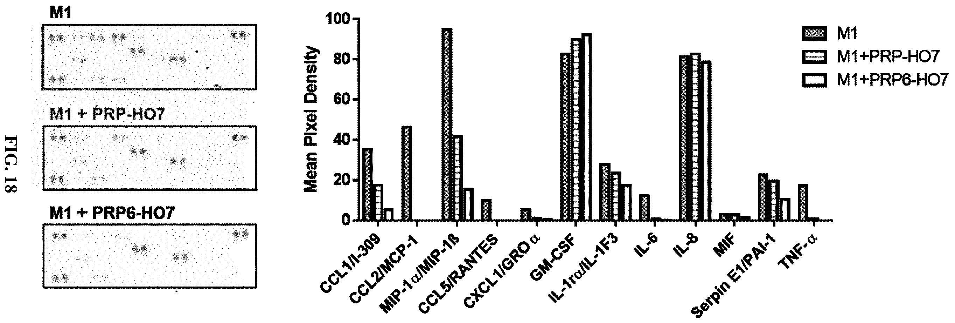

**Figure 18****. Comparative cytokine profile from PRP-HO7- and PRP6-HO7-treated M1 macrophages.** Cell culture supernatants from untreated (M1), PRP-HO7-treated and PRP6-HO7-treated M1 macrophages were incubated with the R&D Systems "Human Cytokine Array Kit", according to the manufacturer's guidelines. The density of each dot was quantified with Quantity One® software and displayed as normalized mean pixel density for each relevant cytokine and treatment.

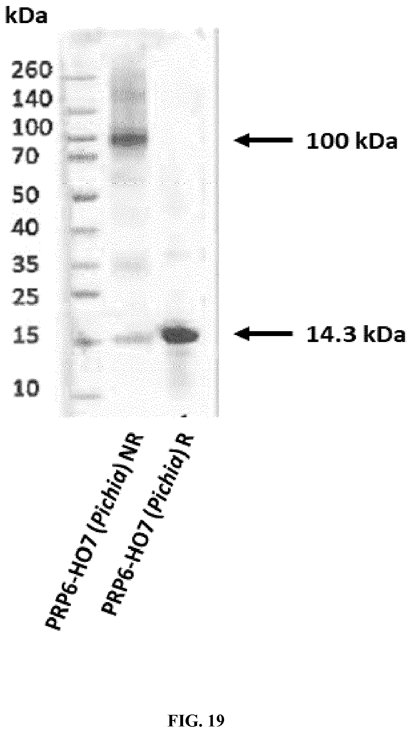

**Figure 19****. PRP6-HO7 protein production and purification in *Pichia Pastoris.*** 12% SDS-PAGE stained with Coomassie Blue visualizing PRP6-HO7 after purification through nickel affinity chromatography (His Tag). Elution fractions pool run under reducing (R) and non-reducing (NR) conditions.

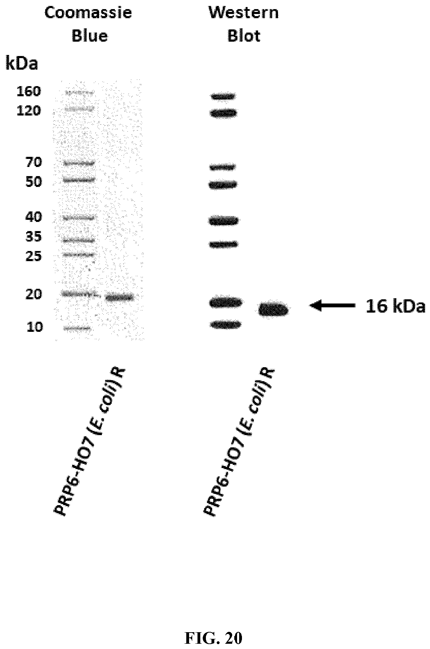

**Figure 20****. PRP6-HO7 protein production and purification in** ***E.coli.** 12%* SDS-PAGE stained with Coomassie Blue or Western analysis under reducing conditions (R) visualizing PRP6-HO7 after purification through nickel affinity chromatography (His Tag). The Western blot was probed with an anti-His MoAb.

### DETAILED DESCRIPTION OF THE INVENTION

The authors of the present invention have surprisingly found that the CCP6 domain of C4BP is sufficient to maintain the tolerogenic and immunomodulating activity of the C4BP alpha chain, and that none of the other CCP domains of the C4BP alpha chain is required. Furthermore, the inventors have surprisingly found that oligomerization of CCP6 is indispensable to preserve the immunomodulatory activity. As shown in examples 1 to 11 of the present invention, an homooligomer formed by seven recombinant polypeptides containing the CCP6 domain and the oligomerization domain of C4BP termed PRP6-HO7, has even more tolerogenic activity than the physiological isoform of C4BP lacking the beta chain C4BP(β-) termed PRP-HO7 as shown in gene expression experiments (Figures 13, 14, 16, 17 and 18) and in the conversion to regulatory or tolerogenic macrophages (Figure 15). The inventors have successfully expressed PRP6-HO7 in eukaryotic HEK (Expi293) cells, yeast *Pichia pastoris* (Example 12 and Figure 19) and bacterial cells (Example 13 and Figure 20), purified and tested *in vitro* to show that this compound is capable of inhibiting the maturation of dendritic cells in the presence of maturation stimuli and of promoting the generation of dendritic cells which show features of tolerogenic dendritic cells. Particularly, PRP6-HO7 is capable of down-regulate activation markers of human Mo-DCs (Examples 2, 4, 5 and 6; Figures 4, 8, 9, 10, 11 and 12), the release of inflammatory cytokines by LPS-matured human Mo-DCs (Example 2 and Figure 6) and key transcripts conforming the inflammatory molecular signature of Mo-DCs (Example 8 and Figure 13). Furthermore, PRP6-HO7 decreases the chemotaxis of human Mo-DCs (Example 7 and Figure 12), and up-regulates "find me" receptors at transcriptional level (Example 8 and Figure 14).

On the other hand, the inventors have demonstrated that PRP6-HO7 is also capable of inhibiting the maturation of macrophages and of promoting the generation of macrophages which show features of tolerogenic cells. Particularly, PRP6-HO7 is capable of down-regulate the expression of activation markers of human M1 and M2 (Example 9 and Figure 15), the release of inflammatory cytokines by M1 macrophages (Example 11 and Figure 18) and key transcripts conforming the molecular signature of differentiating monocyte-derived macrophages (Example 10 and Figure 16). Furthermore, PRP6-HO7 upregulates "find me" receptors in M0 macrophages at transcriptional level (Example 10 and Figure 17).

The homooligomer of the invention has additional advantages such as lacking complement inhibitory activity (Example 3 and Figure 7); having a reduced size (100 kDa) smaller than an antibody (Figure 2); superior stability (enhanced plasma half-life) than antibodies in body fluids; high avidity interaction with its potential surface receptors with a Kd in the low nanomolar range; and high thermodynamic stability because the C-terminal oligomerization scaffold is stabilized by intermolecular disulphide bonds and by layers of electrostatic interactions. Furthermore, PRP6-HO7, contrary to its precursor PRP-HO7, is not glycosylated, which allows its production and further purification in a variety of expression systems (eukaryotic cells, yeast, or bacterial cells). Finally, because of its simplified structure including only the CCP6 domain, PRP6-HO7 administration would not increase infection susceptibility compared to PRP-HO7.

### Recombinant polypeptide of the invention

The authors have demonstrated that a recombinant polypeptide containing the CCP6 domain of C4BP and the oligomerization domain is capable of forming homooligomers that have more tolerogenic activity than the physiological isoform of C4BP lacking the beta chain C4BP(β-).

Thus, in a first aspect, the invention relates to a recombinant polypeptide comprising the CCP6 domain of the C4BP alpha chain or a functionally equivalent variant thereof and an oligomerization domain wherein said polypeptide does not comprise CCP1, CCP2, CCP3, CCP4, CCP5, CCP7 nor CCP8 domains of the C4BP alpha chain.

The expressions "recombinant polypeptide" or "recombinant protein", are used herein interchangeably, and refer to a polypeptide or protein that fusions the CCP6 domain of C4BP and an oligomerization domain that is obtained by recombinant DNA methods upon expression of a recombinant polynucleotide. "Recombinant", as used herein, means that a particular nucleic acid (DNA or RNA) or vector is the product of various combinations of cloning, restriction, polymerase chain reaction (PCR) and ligation steps resulting in a construct having a structural coding sequence distinguishable from endogenous nucleic acids found in natural systems. DNA sequences encoding polypeptides can be assembled from cDNA fragments or from a series of synthetic oligonucleotides to provide a synthetic nucleic acid which is capable of being expressed from a recombinant transcriptional unit contained in a cell or in a cell-free transcription and translation system. Genomic DNA comprising the relevant sequences can also be used in the formation of a recombinant gene or transcriptional unit. Thus, the term "recombinant" nucleic acid refers to one which is not naturally occurring, e.g., is made by the artificial combination of two otherwise separated segments of sequence through human intervention. This artificial combination is often accomplished by either chemical synthesis means, or by the artificial manipulation of isolated segments of nucleic acids, e.g., by genetic engineering techniques, in order to join together nucleic acid segments of desired functions to generate a desired combination of functions. The recombinant polypeptide of the invention is not a deletion mutant obtained by deleting the complete CCP1, CCP2, CCP3, CCP4, CCP5, CCP7 and CCP8 domains of the C4BP.

The term "polypeptide" or "protein", as used herein, refers to a polymer of amino acid residues covalently linked by peptide bonds forming a linear chain. The terminal amino acid at one end of the chain (amino terminal) has a free amino group, while the terminal amino acid at the other end of the chain (carboxy terminal) has a free carboxyl group. As used herein, the term "amino terminus" (abbreviated N-terminus) refers to the free amino group on an amino acid at the amino terminal of a polypeptide or to the amino group when participating in a peptide bond of an amino acid at any other location within the peptide. Similarly, the term "carboxy terminus" refers to the free carboxyl group on the carboxy terminus of a polypeptide or the carboxyl group of an amino acid at any other location within the peptide. In an embodiment the polypeptide of the invention has less than 1000 amino acids, less than 900 amino acids, less than 800 amino acids, less than 700 amino acids, less than 600 amino acids, less than 500 amino acids, less than 475 amino acids, less than 450 amino acids, less than 425 amino acids, less than 400 amino acids, less than 375 amino acids, less than 350 amino acids, less than 325 amino acids, less than 300 amino acids, less than 275 amino acids, less than 250 amino acids, less than 225 amino acids, less than 200 amino acids or less than 175 amino acids. In a preferred embodiment, the polypeptide of the invention has between 100 and 200 amino acids, preferably between 115 and 175 amino acids, more preferably between 119 and 173 amino acids, even more preferably between 119 and 125 amino acids. In a preferred embodiment, the polypeptide of the invention has 119 amino acids.

As used herein, an "amino acid residue" refers to any naturally occurring amino acid, any amino acid derivative or any amino acid mimic known in the art. In certain embodiments, the residues of the polyeptide are sequential, without any non-amino acid interrupting the sequence of amino acid residues. In other embodiments, the sequence may comprise one or more non-amino acid moieties. In particular embodiments, the sequence of residues of the polypeptide may be interrupted by one or more non-amino acid moieties (see for example, Li et al. 2013. Molecules, 18(8):9797-9817).

The terms "C4BP" or "C4b-binding protein", as used herein, refer to a regulatory component of the classical pathway that is mainly synthesized by liver cells which acts as a cofactor for Factor I-dependent degradation of C3b and C4b and accelerates the decay of classical pathway C3/C5-convertases. C4BP circulates in the plasma as three isoforms, the proportion of which depends on the relative levels of C4BPα (70 kDa) and C4BPβ (45 kDa) chains. The major isoform of C4BP is composed of 7 identical α-chains and 1 β-chain (α₇β₁) and is termed C4BP(α₇β₁) or C4BP(β⁺). Upon inflammation a normally less abundant isoform is up-regulated that is exclusively composed of α-chains (α₇β₀), dubbed C4BP(α₇β₀) or C4BP(β⁻). Moreover, recombinant expression of the α-chains in eukaryotic cells might result in an oligomer comprising 6 α-chains (α₆β₀).

The term "C4BP α-chain", also known as PRP or proline-rich protein, as used herein, refers to the mature processed form of the human polypeptide defined under accession number P04003 in the NCBI database (release of July, 31, 2019) and which comprises amino acids 49 to 597. The term C4BP α-chain is also used to refer to orthologs of the human C4BP α-chain such as the mouse C4BP α-chain corresponding to the mature form of the polypeptide shown in the NCBI database under accession number P08607 (release of July, 31, 2019) (amino acids 57 to 469), the rat C4BP α-chain corresponding to the mature form of the polypeptide shown in the NCBI database under accession number Q63514 (release of May, 8, 2019) (amino acids 14 to 558), the bovine C4BP α-chain corresponding to the mature form of the polypeptide shown in the NCBI database under accession number Q28065 (release of May, 8, 2019) (amino acids 49 to 610).

The C4BP α-chain contains 8 complement control protein domains (CCP) which are 60 amino acid residues long with four cysteine residues disulfide bonded in a 1-3 2-4 arrangement, and a hydrophobic core built around an almost invariant tryptophan residue. Thus, the term "CCP domain", as used herein, refers to one of the complement control domains found in the C4BP alpha chain.

The expression "CCP6 domain", as used herein, corresponds to the region found between amino acids 363 and 424 (SEQ ID NO: 1) with respect to the human C4BP alpha chain defined in the sequence provided in the NCBI database under accession number P04003 (release of July 31, 2019) and which corresponds to the sequence:

LCCPEPKLNNGEITQHRKSRPANHCVYFYGDEISFSCHETSRFSAICQGDGTWSP
RTPSCGD (SEQ ID NO: 1)

Thus, in a preferred embodiment, the CCP6 domain is the CCP6 domain of the human C4BP alpha chain, more preferably is SEQ ID NO: 1. In an embodiment, the CCP6 domain of the C4BP alpha chain comprises SEQ ID NO: 1. In another embodiment, the CCP6 domain of the C4BP alpha chain consists of SEQ ID NO: 1.

The term "CCP6 domain" is also used herein to refer to the CCP6 domain of the C4BP alpha chain of any ortholog of the human C4BP alpha chain such as, for example, the CCP6 domain of the rabbit C4BP alpha chain (amino acids 401-460) (SEQ ID NO: 12) with respect to the sequence provided in the NCBI database under accession number G1T7A2 (release of October 16, 2019), the CCP6 domain of the rat C4BP alpha chain (amino acids 327-388) (SEQ ID NO: 13) with respect to the sequence provided in the NCBI database under accession number Q63514 (release of May, 8, 2019), or the CCP6 domain of the bovine C4BP alpha chain (amino acids 365-427) (SEQ ID NO: 14) with respect to the sequence provided in the NCBI database under accession number Q28065 (release of May, 8, 2019) as set forth below in Table 1.

**Table 1. CCP6 domain sequences of orthologs of human CCP6 domain of C4BP alpha chain.**

| **SEQUENCE ID** | **CCP6 domain sequences of orthologs of human CCP6 domain of C4BP alpha chain** |
|---|---|
| SEQ ID NO: 12 | ICCPPPQLDNGAITEHSRNPRANNCTYFFGDIVVYSCYQKQR PPVTCRADGTWSPHTPLC |
| SEQ ID NO: 13 | ICCPVPDPKSVRVIQHEKAHPDNDCTYFFGDEVSYTCQNDIM LTATCKSDGTWHPRTPSCHQ |
| SEQ ID NO: 14 | VCCPEPNLNNYGSITLHRRPSTSTHCTYISGDKISYECHSKYM FDALCTKHGTWSPRTPECRP |

The term "CCP6 domain" is also used herein to refer to any functionally equivalent variant of the naturally-occurring CCP6 domain defined above resulting from the substitution, insertion or deletion of one or more amino acids and which substantially preserves the ability to induce a tolerogenic phenotype of the original polypeptide when forming oligomers.

In a preferred embodiment the functionally equivalent variant of the CCP6 domain results from modification of any of the above sequences by substitutions (e.g., conservative amino acid substitutions) and/or insertions (e.g., small, single amino acid insertions, or insertions encompassing 2, 3, 4, 5, 10, 15, 20, or more contiguous amino acids) and/or deletions (e.g., small, single amino acid deletions, or deletions encompassing 2, 3, 4, 5, 10, 15, 20, or more contiguous amino acids). Thus, in certain embodiments, a variant of a native sequence is one that differs from a naturally-occurring sequence by (i) one or more (e.g., 2, 3, 4, 5, 6, or more) conservative amino acid substitutions, (ii) deletion of one or more (e.g., 2, 3, 4, 5, 6, or more) amino acids, (iii) insertion of one or more (e.g., 2, 3, 4, 5, 6, or more) amino acids, or (iv) a combination thereof. Deleted or inserted amino acids can be contiguous or non-contiguous.

In making such changes, the hydropathic index of amino acids is considered since it is known that certain amino acids may be substituted for other amino acids having a similar hydropathic index or score and result in a polypeptide with similar biological activity. For example, the relative hydropathic character of an amino acid residue affects the secondary and tertiary structure of the resultant polypeptide, which in turn defines the interaction of the polypeptide with other molecules, such as enzymes, substrates, receptors, antibodies, antigens, and the like. As outlined above, amino acid substitutions are generally based on the relative similarity of the amino acid side-chain substituents, for example, their hydrophobicity, hydrophilicity, charge, size, and the like. Exemplary substitutions which take various of the foregoing characteristics into consideration are well known to those of skill in the art and are set forth below in Table 2.

**Table 2. Amino acid substitutions**

| Amino acid substitutions | | | |
|---|---|---|---|
| Original residue | Exemplary Residue Substitution | Original residue | Exemplary Residue Substitution |
| Ala | Gly;Ser | Ile | Leu;Val |
| Arg | Lys | Leu | Ile;Val |
| Asn | Gln;His | Lys | Arg |
| Asp | Glu | Met | Leu;Tyr |
| Cys | Ser | Ser | Thr |
| Gln | Asn | Thr | Ser |
| Glu | Asp | Trp | Tyr |
| Gly | Ala | Tyr | Trp |
| His | Asn;Gln | Val | Ile;Leu |

In an embodiment, the functionally equivalent variant comprises additions of at least 1, at least 2, at least 3, at least 4, at least 5, at least 6, at least 7, at least 8, at least 9, at least 10, at least 15, at least 20 amino acids in the N-terminal end, in the C-terminal end or in both ends of the sequence of the CCP6 domain. In an embodiment, the functionally equivalent variant comprises additions of less than 20, less than 15, less than 10, less than 9, less than 8, less than 7, less than 6, less than 5, less than 4, less than 3, less than 2 or 1 amino acids. In another embodiment, the functionally equivalent variant comprises deletions of at least 1, at least 2, at least 3, at least 4, at least 5, at least 6, at least 7, at least 8, at least 9, at least 10, at least 15, at least 20 amino acids in the N-terminal end, in the C-terminal end or in both ends of the sequence of the CCP6 domain. In another embodiment, the functionally equivalent variant comprises deletions of less than 20, less than 15, less than 10, less than 9, less than 8, less than 7, less than 6, less than 5, less than 4, less than 3, less than 2 or 1 amino acids. In another embodiment, the functionally equivalent variant has a substitution of at least one, at least 2, at least 3, at least 4, at least 5, at least 6, at least 7, at least 8, at least 9, at least 10, at least 15, at least 20 amino acids in the sequence of the CCP6 domain. In another embodiment, the functionally equivalent variant has a substitution of less than 20, less than 15, less than 10, less than 9, less than 8, less than 7, less than 6, less than 5, less than 4, less than 3, less than 2 or 1 amino acids. In a preferred embodiment, the functionally equivalent variant is a variant having the addition of one amino acid at the N-terminal end of the CCP6 domain, preferably having the addition of a methionine.

In another embodiment, the functionally equivalent variant of the CCP6 domain of the C4BP alpha chain includes one or more of the residues equivalent to cysteine at position 2 of SEQ ID NO: 1, the cysteine at position 3 of SEQ ID NO: 1, the proline at position 4 of SEQ ID NO: 1, the proline at position 6 of SEQ ID NO: 1, the isoleucine at position 13 of SEQ ID NO: 1, the histidine at position 16 of SEQ ID NO: 1, the cysteine at position 25 of SEQ ID NO: 1, the tyrosine at position 27 of SEQ ID NO: 1, the glycine at position 30 of SEQ ID NO: 1, the aspartic acid at position 31 of SEQ ID NO: 1, the cysteine at position 37 of SEQ ID NO: 1, the cysteine at position 47 of SEQ ID NO: 1, the glycine at position 51 of SEQ ID NO: 1, the threonine at position 52 of SEQ ID NO: 1, the tryptophan at position 53 of SEQ ID NO: 1, the proline at position 55 of SEQ ID NO: 1, the threonine at position 57 of SEQ ID NO: 1, the proline at position 58 of SEQ ID NO: 1, and the cysteine at position 60 of SEQ ID NO: 1. Preferably, includes all of these residues. In a more preferred embodiment, the variant retains the relative spacing between these residues.

Functionally equivalent variants of the CCP6 domain of the C4BP alpha chain include, without limitation, naturally occurring polymorphic variants (i.e., allelic variants) as well as recombinantly manipulated or engineered variants. CCP6 domain variants suitable for use according to the present invention include, without limitation, polypeptides having at least 99%, at least 98%, at least 97%, at least 96%, at least 95%, at least 94%, at least 93%, at least 92%, at least 91%, at least 90%, at least 85%, at least 80%, at least 75%, at least 70%, at least 65%, at least 60%, at least 55%, at least 50%, at least 48%, at least 45%, at least 40%, at least 35% sequence identity with the naturally-occurring CCP6 domains as defined above and, in particular, with the naturally-occurring CCP6 domain of human origin.

The percent identity of the amino acid sequence of a CCP6 domain variant to the amino acid sequence set forth above can be readily determined by persons skilled in the art by sequence comparison. As used herein, two amino acid sequences have 100 percent amino acid sequence identity if the amino acid residues of the two amino acid sequences are the same when aligned for maximal correspondence. Sequence comparisons of polypeptides and polynucleotides (for example, the polynucleotides that encode the polypeptides described herein) can be performed using any method such as those that use computer algorithms well known to persons having ordinary skill in the art. Such algorithms include Align or the BLAST algorithm (see, e.g., Altschul, J. Mol. Biol. 219:555-565, 1991; Henikoff and Henikoff, Proc. Natl. Acad. Sci. USA 89:10915-10919, 1992), which are available at the NCBI website (see [online] Internet at ncbi.nlm.nih.gov/cgi-bin/BLAST). Default parameters may be used. In addition, standard software programs are available, such as those included in the LASERGENE bioinformatics computing suite (DNASTAR, Inc., Madison, Wis.); CLUSTALW program (Thompson et al., Nucleic Acids Res. 22:4673-80 (1991)); and "GeneDoc" (Nicholas et al., EMBNEW News 4:14 (1991)). Other methods for comparing two amino acid sequences by determining optimal alignment are practiced by persons having skill in the art (see, for example, Peruski and Peruski, The Internet and the New Biology: Tools for Genomic and Molecular Research (ASM Press, Inc. 1997); Wu et al. (eds.), "Information Superhighway and Computer Databases of Nucleic Acids and Proteins," in Methods in Gene Biotechnology, pages 123-151 (CRC Press, Inc. 1997); and Bishop (ed.), Guide to Human Genome Computing, 2nd Ed. (Academic Press, Inc. 1998)).

The expression "substantially preserves the ability to induce a tolerogenic phenotype of the original polypeptide when forming oligomers", as used herein, refers to polypeptides which are capable of inhibiting the maturation of dendritic cells and/or of macrophages as determined, e.g., in any of the examples 1 to 11 of the present invention when the CCP6 variant is fused to an oligomerization domain, preferably to the oligomerization domain of SEQ ID NO: 2. Particularly, the functionally equivalent variant shows an ability in generating tolerogenic dendritic cells when added to monocyte cells during the differentiation stage to immature dendritic cells and/or when added to immature dendritic cells during their maturation stage to mature dendritic cells. The ability of the variant to promote the generation of tolerogenic dendritic cells can be determined, e.g., by measuring the expression levels in the dendritic cells of maturation markers such as CD83, CD86 and/or CD80 of dendritic cells which have been matured in the presence of the variant (example 4 of the present invention). Thus, a polypeptide is considered as a functionally equivalent variant of the CCP6 domain of the C4BP alpha chain if it shows at least 100%, at least 95%, at least 90%, at least 85%, at least 80%, at least 75%, at least 70%, at least 65%, at least 60%, or at least 50% of the activity of the CCP6 domain of the human C4BP alpha chain when it is fused to an oligomerization domain, particularly to the oligomerization domain of SEQ ID NO: 2.

The term "oligomerization domain", as used herein, refers to a polypeptide domain having the property that polypeptides comprising said domain have a propensity to aggregate and form oligomers, i.e., is a region responsible for the oligomerization between monomers. The oligomerization domain contains amino acids in a region of one monomer that can interact with amino acids in a region of another monomer to enable oligomerization of the monomers. Suitable oligomerization domains are known in the art. In a preferred embodiment, the oligomerization domain is the oligomerization domain of C4BP alpha chain. In another embodiment, the oligomerization domain is a functionally equivalent variant of the oligomerization domain of the C4BP alpha chain. The expression "oligomerization domain of C4BP alpha chain", as used herein, corresponds to the region found between amino acids 541 and 597 (SEQ ID NO: 2) with respect to the human C4BP alpha chain defined in the sequence provided in the NCBI database under accession number P04003 (release of July, 31, 2019) and which corresponds to the sequence:

ETPEGCEQVLTGKRLMQCLPNPEDVKMALEVYKLSLEIEQLELQRDSA
RQSTLDKEL (SEQ ID NO: 2)

Therefore, in a preferred embodiment, the oligomerization domain is the oligomerization domain of human C4BP alpha chain or a functionally equivalent variant thereof; more preferably is SEQ ID NO: 2 or a functionally equivalent variant thereof; even more preferably is SEQ ID NO: 2. In an embodiment the oligomerization domain comprises SEQ ID NO: 2 or a functionally equivalent variant thereof, preferably SEQ ID NO: 2. In another embodiment, the oligomerization domain consists of SEQ ID NO: 2 or a functionally equivalent variant thereof, preferably SEQ ID NO: 2.

The term "oligomerization domain of C4BP alpha chain" is also used herein to refer to the oligomerization domain of any ortholog of the human C4BP alpha chain such as, for example, the oligomerization domain of the mouse C4BP alpha chain (amino acids 416-469) (SEQ ID NO: 15) with respect to the sequence provided in the NCBI database under accession number P08607 (release of July, 31, 2019), the oligomerization domain of the rat C4BP alpha chain (amino acids 504-558) (SEQ ID NO: 16) with respect to the sequence provided in the NCBI database under accession number Q63514 (release of May, 8, 2019), the oligomerization domain of the bovine C4BP alpha chain (amino acids (544-610) (SEQ ID NO: 17) with respect to the sequence provided in the NCBI database under accession number Q28065 (release of May, 8, 2019) as set forth below in Table 3. The oligomerization domain of C4BP alpha chain of other mammalian orthologs useful in the present invention are SEQ ID NO: 18 to SEQ ID NO: 33 set forth in Table 3. Functionally equivalent variants of the oligomerization domain of human C4BP alpha chain useful in the present invention are SEQ ID NO: 34 to SEQ ID NO: 41. The oligomerization domain of avian orthologs described in PCT patent application WO 2007/062819 are also included such as the oligomerization domain of chicken C4BP alpha chain (SEQ ID NO: 42) or its avian homologue in zebra finch (SEQ ID NO: 43). Functionally equivalent variants of the oligomerization domain of C4BP disclosed in table 1 of WO 2007/062819 are also included (SEQ ID NO: 44 to SEQ ID NO: 55).

**Table 3. Oligomerization domain sequences of orthologs and functionally equivalent variants of human oligomerization domain of C4BP alpha chain.**

| **SEQUENCE ID** | **oligomerization domain sequences of orthologs and functionally equivalent variants of human oligomerization domain of C4BP alpha chain** |
|---|---|
| SEQ ID NO: 15 | EASEDLKPALTGNKTMQYVPNSHDVKMALEIYKLTLEVELL QLQIQKEKHTEAH |
| SEQ ID NO: 16 | EVPKDCEHVFAGKKLMQCLPNSNDVKMALEVYKLTLEIKQ LQLQIDKAKHVDREL |
| SEQ ID NO: 17 | EYPEGCEQVVTGRKLLQCLSRPEEVKLALEVYKLSLEIEILQT NKLKKEAFLLREREKNVTCDFNPE |
| SEQ ID NO: 18 | ETPEGCEQVLTGKRLMQCLPNPEDVKMALEVYKLSLEIEQL ELQRDSARQYTLDKEL |
| SEQ ID NO: 19 | ETPEGCEQVLAGKRLMQCLPNPEDVKMALEVYKLSLEIEQL ELQRDRARQSTLDKEL |
| SEQ ID NO: 20 | ETPEGCEQVLAGKRLMQCLPNPEDVKMALEVYKLSLEIEQL ELQRDRARQSTWDKEL |
| SEQ ID NO: 21 | EVPEGCEQVQAGRRLMQCLADPYEVKMALEVYKLSLEIELL ELQRDKARKSSVLRQL |
| SEQ ID NO: 22 | VVPEGCEHILKGRKTMQCLPNPEDVKMALEIYKLSLDIELLE LQRDRAKESTVQSPV |
| SEQ ID NO: 23 | EYPEDCEQVHEGKKLMQCLPNLEEIKLALELYKLSLETKLLE LQIDKEKKAKAKYSI |
| SEQ ID NO: 24 | EYPEDCEQVHEGKKLMECLPTLEEIKLALALYKLSLETNLLE LQIDKEKKAKAKYST |
| SEQ ID NO: 25 | EIAEGCEQVLAGRKIMQCLPKPEDVRTALELYKLSLEIKQLE KKLEKEEKCTPEVQE |
| SEQ ID NO: 26 | EYPEGCEQVVTGRKLLKCLSRPEEVKLALEVYKLSLEIALLE LQIDKPKDAS |
| SEQ ID NO: 27 | EVPENCEQVIVGKKLMKCLSNPDEAQMALQLYKLSLEAELL RLQIVKARQGS |
| SEQ ID NO: 28 | VSAEVCEAVFKGQKLLKCLPNAMEVKMALEVYKLSLEIEKL EQEKRKLEIA |
| SEQ ID NO: 29 | EVPEECKQVAAGRKLLECLPNPSDVKMALEVYKLSLEIEQLE KEKYVKIQEKFSKKEMKQLTSALH |
| SEQ ID NO: 30 | EVLEDCRIVSRGAQLLHCLSSPEDVHRALKVYKLFLEIERLE HQKEKWIQLHRKPQSMK |
| SEQ ID NO: 31 | EGPEDCEIVNKGRQLLQCLSSPEDVQRALEVYKLSLEIERLE QQREKRTSVHRKAHYTKVDGP |
| SEQ ID NO: 32 | EAPEGCEQVLTGRKLMQCLPSPEDVKVALEVYKLSLEIKQLE KERDKLMNTHQKFSEKEEMKDLFFP |
| SEQ ID NO: 33 | EVPEGCEQVLTGKKLMQCLPNPEDVKMALEVYKLSLEIELL ELQIDKARQGS |
| SEQ ID NO: 34 | GCEQVLTGKRLMQCLPNPEDVKMALEVYKLSLEIEQLELQR DSARQS |
| SEQ ID NO: 35 | ETPEGCEQVLTGKRLMQeLPNPEDVKMALEVYKLSLEIEQLE LQRDSARQS |
| SEQ ID NO: 36 | GSEQVLTGKRLMQSLPNPEDVKMALEVYKLSLEIEQLELQR DSARQSTLDKEL |
| SEQ ID NO: 37 | ETPEGCEQVLTGKRLMQeLPNPEDVKMALEIYKLTLEIEQLE LQRDSARQSTLDKEL |
| SEQ ID NO: 38 | ETPEGCEQVLTGKRLMQeLPNPEDVKMALEIYKLSLEIKQLE LQRDSARQSTLDKEL |
| SEQ ID NO: 39 | EGCEQILTGKRLMQeLPDPEDVKMALEIYKLSLEIKQLELQR DRARQSTL |
| SEQ ID NO: 40 | ETPEGCEQVLTGKRLMQeLPNPEDVKMALEVYKLSLEIKQLE LQRDRARQSTLDKEL |
| SEQ ID NO: 41 | EGCEQILTGKRLMQeLPNPEDVKMALEIYKLSLEIEQLELQR DRARQSTLDK |
| SEQ ID NO: 42 | KKQGDADVCGEVAYIQSVVSDCHVPTEDVKTLLEIRKLFLEI QKLKVELQGLSKEFLEHILH |
| SEQ ID NO: 43 | MKEGDGDVCQEVHYIKSTFECGVPVEEVKILLEIQKLLLEIN KLEMELEN |
| SEQ ID NO: 44 | KKQGDADVCGEVAYIQSVVSDCHVPTEDVKTLLEVRKLFLE IQKLKVELQGLSKEFLEHILH |
| SEQ ID NO: 45 | KKQGDADVCGEVAYIQSVVSDCHVPTEDVKMLLEVRKLFL EIQKLKVELQGLSKEFLEHILH |
| SEQ ID NO: 46 | KKQGDADVCGEVIYIQSVVSDCHVPTEDVRTLLEIRKLFLEI QKLKVELQGLSKEFLEHILH |
| SEQ ID NO: 47 | KKQGDADVCGEVIYIQSVVSDCHVPTEDVKTLLEIRKLFLEI QKLKVELNGLSKEFLEHILH |
| SEQ ID NO: 48 | KKQGDADVCGEVIYIQSVVSDCHVPTEDVKMLLEIRKLFLEI QKLKVELNGLSKEFLEHILH |
| SEQ ID NO: 49 | KKQGDADVCGEVIYIQSVVSDCLPNTEDVKTLLEVRKLFLEI QKLKVELQGLSKEFLEHILH |
| SEQ ID NO: 50 | DADVCGEVAYIQSVVSDCHVPTEDVKTLLEIRKLFLEIQKLK VELQGLSKEFLE |
| SEQ ID NO: 51 | ADVCGEVAYIQSVVSDCHVPTEDVKTLLEIRKLFLEIQKLKV ELQGLSKEF |
| SEQ ID NO: 52 | ADVCGEVIYIQSVVSDCHVPTEDVKTLLEVRKLFLEIQKLKV ELQGLSKEF |
| SEQ ID NO: 53 | ADVCGEVAYIQSVVSDCHVPTEDVKTLLEIRKLFLEIQKLKV ELNGLS |
| SEQ ID NO: 54 | KKQGDADVCGEVAYIQSVVSDCHVPTEDVKTLLEIRKLFLEI QKLKVELQGLSKE |
| SEQ ID NO: 55 | KKQGDADVCGEVAYIQSVVSDCHVPTAELRTLLEIRKLFLEI QKLKVELQGLSKE |

In a preferred embodiment, the oligomerization domain is the oligomerization domain of a mammalian C4BP alpha chain, more preferably of a human, mouse or rat C4BP alpha chain, most preferably of a human C4BP alpha chain.

In a preferred embodiment, the oligomerization domain is an oligomerization domain selected from the group consisting of SEQ ID NO: 2 and any one of SEQ ID NO: 15 to 55; preferably is selected from the group consisting of SEQ ID NO: 2 and any one of SEQ ID NO: 15 to SEQ ID NO: 41; more preferably is selected from the group consisting of SEQ ID NO: 2 and any one of SEQ ID NO: 15 to SEQ ID NO: 33; even more preferably is selected from the group consisting of SEQ ID NO: 2 and any one of SEQ ID NO: 15 to SEQ ID NO: 32; even more preferably is selected from the group consisting of SEQ ID NO: 2 and any one of SEQ ID NO: 15 to 17; even more preferably is SEQ ID NO: 2.

The oligomerization domain of C4BP alpha chain include also other mammalian and non-mammalian homologues of these sequences. The means to obtain such homologues are routine techniques available to those of skill in the art. In essence, such techniques include using nucleic acid encoding any of the sequences of oligomerization domains of the present invention, or fragments thereof, as a probe to recover and to determine the sequence of C4BP homologues in other species. A wide variety of techniques are available for this, for example PCR amplification and cloning of the homologue using a suitable source of mRNA (e.g. from an embryo or an actively dividing differentiated or tumor cell), or by methods comprising obtaining a cDNA library from the animal, e.g., a cDNA library from one of the above-mentioned sources, probing said library with a nucleic acid encoding any of the oligomerization domains of the present invention under stringent conditions, and recovering a cDNA encoding all or part of the sequences homologues of that animal. Where a partial cDNA is obtained, the full-length coding sequence may be determined by primer extension techniques. Alternatively, where all or part of the genome sequence of the animal is available, homology searches with the sequences of the invention may be used to determine suitable homologues.

The term "oligomerization domain of C4BP alpha chain" is also used herein to refer to any functionally equivalent variant of the naturally occurring oligomerization domain of C4BP alpha chain defined above resulting from the substitution, insertion or deletion of one or more amino acids and which substantially preserves the ability to form oligomers of the original polypeptide when forming part of a recombinant polypeptide of the invention.

Therefore, the functionally equivalent variant can be a fragment of the oligomerization domain wherein amino acids not relevant for the oligomerization ability have been deleted but amino acids relevant for the oligomerization are preserved, or a mutant wherein amino acids not involved in the oligomerization are mutated.

In a preferred embodiment, the functionally equivalent variant of the oligomerization domain of C4BP alpha chain results from modification of any of the above sequences by substitutions (e.g., conservative amino acid substitutions) and/or insertions (e.g., small, single amino acid insertions, or insertions encompassing 2, 3, 4, 5, 10, 15, 20, or more contiguous amino acids) and/or deletions (e.g., small, single amino acid deletions, or deletions encompassing 2, 3, 4, 5, 10, 15, 20, or more contiguous amino acids). Thus, in certain embodiments, a variant of a native sequence is one that differs from a naturally-occurring sequence by (i) one or more (e.g., 2, 3, 4, 5, 6, or more) conservative amino acid substitutions, (ii) deletion of one or more (e.g., 2, 3, 4, 5, 6, or more) amino acids, (iii) insertion of one or more (e.g., 2, 3, 4, 5, 6, or more) amino acids, or (iv) a combination thereof. Deleted or inserted amino acids can be contiguous or non-contiguous.

In making such changes, the hydropathic index of amino acids is considered as was explained in relation to the functionally equivalent variant of CCP6 domain. Table 2 which discloses exemplary substitutions is also applicable to the functionally equivalent variants of the oligomerization domain of C4BP alpha chain.

In an embodiment, the functionally equivalent variant comprises additions of at least 1, at least 2, at least 3, at least 4, at least 5, at least 6, at least 7, at least 8, at least 9, at least 10, at least 15, at least 20 amino acids in the N-terminal end, in the C-terminal end or in both ends of the sequence of the oligomerization domain. In an embodiment, the functionally equivalent variant comprises additions of less than 20, less than 15, less than 10, less than 9, less than 8, less than 7, less than 6, less than 5, less than 4, less than 3, less than 2 or 1 amino acids. In another embodiment, the functionally equivalent variant comprises deletions of at least 1, at least 2, at least 3, at least 4, at least 5, at least 6, at least 7, at least 8, at least 9, at least 10, at least 15, at least 20 amino acids in the N-terminal end, in the C-terminal end or in both ends of the sequence of the oligomerization domain. In another embodiment, the functionally equivalent variant comprises deletions of less than 20, less than 15, less than 10, less than 9, less than 8, less than 7, less than 6, less than 5, less than 4, less than 3, less than 2 or 1 amino acids. In another embodiment, the functionally equivalent variant has a substitution of at least one, at least 2, at least 3, at least 4, at least 5, at least 6, at least 7, at least 8, at least 9, at least 10, at least 15, at least 20 amino acids in the sequence of the oligomerization domain. In another embodiment, the functionally equivalent variant has a substitution of less than 20, less than 15, less than 10, less than 9, less than 8, less than 7, less than 6, less than 5, less than 4, less than 3, less than 2 or 1 amino acids. In a preferred embodiment, the functionally equivalent variant is a variant having the addition of one amino acid at the N-terminal end of the oligomerization domain, preferably having the addition of a methionine.

In an embodiment, a fragment of the oligomerization domain of the C4BP alpha chain capable of forming oligomers may comprise at least 47 amino acids, preferably at least 50 amino acids.

In another embodiment, the variant of the oligomerization domain of the C4BP alpha chain includes one or more residues equivalent to glutamate at position 1 of SEQ ID NO: 2, glycine at position 12 of SEQ ID NO: 2, glutamine at position 17 of SEQ ID NO: 2, valine at position 25 of SEQ ID NO: 2, lysine at position 26 of SEQ ID NO: 2, alanine at position 28 of SEQ ID NO: 2, leucine at position 29 of SEQ ID NO: 2, glutamate at position 30 of SEQ ID NO: 2, tyrosine at position 32 of SEQ ID NO: 2, lysine at position 33 of SEQ ID NO: 2, leucine at position 34 of SEQ ID NO: 2, leucine at position 36 of SEQ ID NO: 2, glutamate at position 37 of SEQ ID NO: 2, and leucine at position 41 of SEQ ID NO: 2. Preferably, the variant includes all of these residues. More preferably, the variant retains the relative spacing between these residues.

In a preferred embodiment, the variant includes the residues equivalent to glycine at position 12 of SEQ ID NO: 2, the alanine at position 28 of SEQ ID NO: 2, the leucines at positions 29, 34, 36 and 41 of SEQ ID NO: 2, the tyrosine at position 32 of SEQ ID NO: 2, and the lysine at position 33 of SEQ ID NO: 2. Preferably, the variant retains the relative spacing between these residues.

In another preferred embodiment, the variant of the oligomerization domain of the C4BP alpha chain includes the residues equivalent to glycine at position 12 of SEQ ID NO: 2, the alanine at position 28 of SEQ ID NO: 2, the leucines at positions 29, 34, 36 and 41 of SEQ ID NO: 2, the tyrosine at position 32 of SEQ ID NO: 2, the lysine at position 33 of SEQ ID NO: 2, and the two cysteine residues at positions 6 and 18 of SEQ ID NO: 2. Preferably, the variant retains the relative spacing between these residues.

Functionally equivalent variants of the oligomerization domain of the C4BP alpha chain for use according to the present invention include, without limitation, naturally occurring polymorphic variants (i.e., allelic variants) as well as recombinantly manipulated or engineered variants. Variants of the oligomerization domain suitable for use according to the present invention include, without limitation, polypeptides having at least 99%, at least 98%, at least 97%, at least 96%, at least 95%, at least 94%, at least 93%, at least 92%, at least 91%, at least 90%, at least 85%, at least 80%, at least 75%, at least 70%, at least 65%, at least 60%, at least 55%, at least 50%, at least 45%, at least 40%, at least 35% sequence identity with the naturally-occurring oligomerization domains as defined above and, in particular, with the naturally-occurring oligomerization domain of C4BP alpha chain of human origin.

Methods for determining the percent identity of the amino acid sequence of a variant of the oligomerization domain were previously described in the context of the CCP6 domain.

The expression "substantially preserves the ability to form oligomers of the original polypeptide when forming part of a recombinant polypeptide of the invention", as used herein, refers to polypeptides which are capable of forming oligomers as determined, e.g., as shown in Example 1 and Figure 3 of the present invention when the oligomerization domain variant is fused to a CCP6 domain, preferably to the CCP6 domain of SEQ ID NO: 1. Methods for determining whether a polypeptide is capable of forming oligomers are available to the skilled person and include, for instance, a method as described by Blom et al. (J.Biol.Chem. 2001, 276: 27136-27144) based on the analysis by polyacrylamide gel electrophoresis under native conditions of a purified C4BP obtained by recombinant expression of a variant α-chain in eukaryotic cell (e.g. 293 cells) followed by affinity purification using an antibody specific for CCP6 region. Alternatively, the ability of a variant of the oligomerization domain to form oligomers may be tested by expressing the variant in prokaryotic or eukaryotic host cells according to the invention and analyzing the variants recovered by polyacrylamide gel electrophoresis and Coomassie Blue staining under reducing and non-reducing conditions (i.e., by comparing the behaviour of the variant on an SDS-PAGE gel in the presence and absence of the reducing agent beta-mercaptoethanol) or by carrying out a Western blot probed with an anti-tag antibody or an anti-C4BP alpha chain antibody in reducing and non-reducing conditions as explained in Example 1 and Figure 3. Alternatively, the ability of a variant of the oligomerization domain to form oligomers may be tested by expressing the variant in a prokaryotic host cell according to the invention, recovering the variant under conditions which result in oligomerization of the full 57 amino acid oligomerization domain of human C4BP alpha chain, and determining, e.g., by gel filtration, whether the variant forms oligomers.

Thus, a polypeptide is considered as a functionally equivalent variant of the oligomerization domain of C4BP alpha chain if it shows at least 100%, at least 95%, at least 90%, at least 85%, at least 80%, at least 75%, at least 70%, at least 65%, at least 60%, or at least 50% of the capability of forming oligomers of the oligomerization domain of the human C4BP alpha chain when it is fused to a CCP6 domain, particularly to the CCP6 domain of SEQ ID NO: 1.

The functionally equivalent variant of the oligomerization domain of human C4BP alpha chain preferably maintains: (a) the cysteine residue equivalent to the cysteine residue 498 of the mature form of human C4BP alpha chain (i.e., the form of human C4BP alpha chain lacking the signal peptide), corresponding to amino acid 546 of the immature form defined under accession number P04003 in the NCBI database (release of July, 31, 2019); (b) the cysteine residue equivalent to the cysteine residue 510 of the mature form of human C4BP alpha chain corresponding to amino acid 558 of the immature form defined under accession number P04003 in the NCBI database (release of July, 31, 2019); and/or (c) the amphipatic α-helix region of the C-terminal end, more preferably the last 13 residues equivalent to residues 537-549 of the mature form of human C4BP alpha chain, corresponding to amino acids 585-597 of the immature form defined under accession number P04003 in the NCBI database (release of July, 31, 2019). Preferably the functionally equivalent variant needs to maintain (c); more preferably the functionally equivalent variant needs to maintain (a), (b) and (c) for intracellular oligomerization of the molecule.

The recombinant polypeptide of the invention comprising the CCP6 domain of the C4BP alpha chain does not contain any of the other CCP domains found in the C4BP alpha chain. Therefore, the polypeptide of the invention does not comprise CCP1, CCP2, CCP3, CCP4, CCP5, CCP7 and CCP8 domains of the C4BP alpha chain. When referring to human C4BP alpha chain, the CCP1 domain corresponds to amino acids 49 to 110, the CCP2 domain corresponds to amino acids 111 to 172, the CCP3 domain corresponds to amino acids 173 to 236, the CCP4 domain corresponds to amino acids 237 to 296, the CCP5 domain corresponds to amino acids 297 to 362, the CCP7 domain corresponds to amino acids 425 to 482 and the CCP8 domain corresponds to amino acids 483 to 540 of the polypeptide shown in the NCBI database under accession number P04003 (release of July, 31, 2019). The person skilled in the art knows how to determine the CCPs equivalent to the human C4BP alpha chain in other C4BP alpha chains.

In an embodiment, the recombinant polypeptide consists of the CCP6 domain of the C4BP alpha chain or a functionally equivalent variant thereof and an oligomerization domain; preferably consists of the CCP6 domain of the C4BP alpha chain and the oligomerization domain of the C4BP alpha chain; more preferably consists of the CCP6 domain of the human C4BP alpha chain and the oligomerization domain of the human C4BP alpha chain. Both domains can be in any order.

In a preferred embodiment of the polypeptide of the invention, the CCP6 domain of the C4BP alpha chain and the oligomerization domain, preferably the oligomerization domain of the C4BP alpha chain, pertain to the same species.

In another embodiment, the recombinant polypeptide consists of (a) a methionine, (b) the CCP6 domain of the C4BP alpha chain or a functionally equivalent variant thereof, and (c) an oligomerization domain; preferably consists of (a) a methionine, (b) the CCP6 domain of the C4BP alpha chain, and (c) the oligomerization domain of the C4BP alpha chain; more preferably consists of (a) a methionine, (b) the CCP6 domain of the human C4BP alpha chain, and (c) the oligomerization domain of the human C4BP alpha chain. The methionine is at the N-terminal end of the recombinant polypeptide and the domains can be in any order.

In a preferred embodiment, the recombinant polypeptide of the invention comprises SEQ ID NO: 3 or a functionally equivalent variant thereof, preferably comprises SEQ ID NO: 3. In a more preferred embodiment, the recombinant polypeptide of the invention consists of SEQ ID NO: 3 or a functionally equivalent variant thereof, preferably consists of SEQ ID NO: 3.

LCCPEPKLNNGEITQHRKSRPANHCVYFYGDEISFSCHETSRFSAICQGDGTWSP
RTPSCGDETPEGCEQVLTGKRLMQCLPNPEDVKMALEVYKLSLEIEQLELQRDS
ARQSTLDKEL (SEQ ID NO: 3)

In another embodiment, the recombinant polypeptide of the invention consists of the sequence SEQ ID NO: 3 and an additional methionine at the N-terminal end of the polypeptide.

In an embodiment, the polypeptide of the invention does not comprise a region of a protein different from C4BP. For example, the polypeptide of the invention cannot be a fusion protein comprising a region which forms part of a different protein from C4BP.

In another embodiment, the polypeptide of the invention comprises a polypeptide which does not form part of C4BP. The polypeptide which does not form part of C4BP is considered as a heterologous polypeptide, i.e., a polypeptide that is derived from a different gene as that coding for C4BP.

The recombinant polypeptide of the invention may comprise the CCP6 domain and the oligomerization domain in any order. The recombinant polypeptide of the present invention may comprise in an amino terminal to carboxy terminal direction, (a) the region which comprises the CCP6 domain of C4BP alpha chain, and (b) the region which comprises the oligomerization domain. Alternatively, the recombinant polypeptide of the invention may comprise in an amino terminal to carboxy terminal direction, (a) the region which comprises the oligomerization domain and (b) the region which comprises the CCP6 domain of the C4BP alpha chain. The person skilled in the art will understand that the recombinant polypeptide of the invention may contain additional amino acids between the CCP6 domain and the oligomerization domain. In a preferred embodiment, the C-terminus of the CCP6 domain of the C4BP alpha chain is directly fused to the N-terminus of the oligomerization domain, i.e., there is no additional amino acids or linkers between both domains. In another embodiment, the C-terminus of the oligomerization domain is directly fused to the N-terminus of the CCP6 domain of the C4BP alpha-chain. In another embodiment, the C-terminus of the CCP6 domain of the C4BP alpha chain is separated from the N-terminus of the oligomerization domain by additional amino acids, i.e., amino acids not present in the natural-occurring domains for which they derive. In another embodiment, the C-terminus of the oligomerization domain is separated from the N-terminus of the CCP6 domain of the C4BP alpha chain by additional amino acids. In a preferred embodiment, the polypeptide of the invention comprises, in an amino terminal to carboxy terminal direction, (a) a methionine, (b) the region which comprises the CCP6 domain of C4BP alpha chain, and (c) the region which comprises the oligomerization domain. In another embodiment, the polypeptide of the invention comprises, in an amino terminal to carboxy terminal direction, (a) a methionine, (b) the region which comprises the oligomerization domain and (c) the region which comprises the CCP6 domain of the C4BP alpha chain. In another embodiment, the recombinant polypeptide of the invention consists of in an amino terminal to carboxy terminal direction, of (a) the CCP6 domain of C4BP alpha chain, and (b) the oligomerization domain of C4BP alpha chain. In another embodiment, the recombinant polypeptide of the invention consists of in an amino terminal to carboxy terminal direction, of (a) the oligomerization domain of C4BP alpha chain, and (b) the CCP6 domain of C4BP alpha chain. All these embodiments apply equally when the polypeptide of the invention is formed by functionally equivalent variants of the CCP6 domain of the C4BP alpha chain and/or functionally equivalent variants of the oligomerization domain.

The recombinant polypeptide of the invention can be in the form of a precursor. The term "precursor" refers to a polypeptide which, once processed, can give rise to the mature form of the polypeptide. The precursor is a polypeptide comprising a signal peptide to export the polypeptide to the extracellular media when the host cell is an eukaryotic cell.

In another embodiment, the recombinant polypeptide of the invention further comprises a signal peptide. The expression "signal peptide", as used herein, refers to a signal sequence which is located in the N-terminus of the polypeptide. As it is used herein, the term "signal peptide" or "signal sequence" or "secretory signal peptide" or "secretory signal sequence" refers to a peptide of a relatively short length, generally between 5 and 30 amino acid residues, directing proteins synthesized in the cell towards the secretory pathway. The signal peptide usually contains a series of hydrophobic amino acids adopting a secondary alpha helix structure. Additionally, many peptides include a series of positively-charged amino acids that can contribute to the protein adopting the suitable topology for its translocation. The signal peptide tends to have at its carboxyl end a motif for recognition by a peptidase, which is capable of hydrolizing the signal peptide giving rise to a free signal peptide and a mature protein. The signal peptide can be cleaved once the protein of interest has reached the appropriate location. Any signal peptide may be used in the present invention. The signal sequence may be from the same species of the organism that is transformed or may be from a different species. As a way of illustrative, non-limitative examples, signal peptides from *Chlamydomonas reinhardtii* carbonic anhydrase (CAH1), signal peptide from *Chlamydomonas reinhardtii* periplasmic arylsulfatase (ARS1) or the signal peptide from *Chlamydomonas reinhardtii* Gametolysin M11 may be used. Signal peptides useful for expression in bacteria can be Sec- or Tat- signal peptides. Signal peptides particularly for expression in yeasts can be the signal peptide from *S.cerevisiae* alpha-mating factor prepro peptide, the signal peptides from the *P.pastoris* acid phosphatase gene (PHO1) and the extracellular protein X (EPX1). In a preferred embodiment, the signal peptide is the signal peptide from alpha-factor mating peptide, preferably the alpha-factor mating peptide from *Saccharomyces cerevisiae.* Signal peptides useful for expression in mammalian eukaryotic cells are, without limitation, signal peptides from human OSM, VSV-G, mouse Ig Kappa, human IgG2 H, BM40, secrecon, human IgKVIII, CD33, tPA, human chymotrypsinogen, human trypsinogen-2, human IL-2, gaussia luc, albumin (HSA), influenza haemagglutinin, human insulin or silkworm fibroin LC.

In a preferred embodiment, the signal peptide is a human signal peptide. In a preferred embodiment, the signal peptide is the signal peptide of C4BP alpha chain, preferably of human C4BP alpha chain, more preferably is SEQ ID NO: 4 or a functionally equivalent variant thereof; more preferably is SEQ ID NO: 4.
MHPPKTPSGALHRKRKMAAWPFSRLWKVSDPILFQMTLIAALLPAVLG (SEQ ID NO: 4)

Therefore, in an embodiment, the signal peptide comprises SEQ ID NO: 4. In another embodiment the signal peptide consists of SEQ ID NO: 4.

Fusion of signal peptide to the polypeptide results in secretion of the fusion protein to the media, which is the preferred strategy since it permits easy and efficient purification from the extracellular medium.

In an embodiment, the polypeptide of the invention is a fusion protein which comprises in an amino terminal to carboxy terminal direction, (a) a signal peptide, (b) the region which comprises the CCP6 domain of C4BP alpha chain and (c) the region which comprises the oligomerization domain. Alternatively, the polypeptide of the invention is a fusion protein which comprises in an amino terminal to carboxy terminal direction, (a) a signal peptide, (b) the region which comprises the oligomerization domain and (c) the region which comprises the CCP6 domain of C4BP alpha chain. All these embodiments are equally applicable when the polypeptide of the invention consists of a signal peptide, a region consisting of the CCP6 domain of C4BP alpha chain, and a region consisting of the oligomerization domain. All these embodiments apply equally when the polypeptide of the invention is formed by functionally equivalent variants of the CCP6 domain of the C4BP alpha chain and/or functionally equivalent variants of the oligomerization domain.

In an embodiment, the recombinant polypeptide of the invention comprises SEQ ID NO: 5 or a functionally equivalent variant thereof, preferably comprises SEQ ID NO: 5. In another embodiment the recombinant polypeptide of the invention consists of SEQ ID NO: 5 or a functionally equivalent variant thereof, preferably consists of SEQ ID NO: 5.

MHPPKTPSGALHRKRKMAAWPFSRLWKVSDPILFQMTLIAALLPAVLGLCCPE
PKLNNGEITQHRKSRPANHCVYFYGDEISFSCHETSRFSAICQGDGTWSPRTPSC
GDETPEGCEQVLTGKRLMQCLPNPEDVKMALEVYKLSLEIEQLELQRDSARQS
TLDKEL (SEQ ID NO: 5)

In another embodiment, the polypeptide of the invention does not comprise a signal peptide.

In another embodiment, the recombinant polypeptide further comprises a peptide that is not part of the C4BP alpha chain.

In an embodiment, the polypeptide of the invention is a fusion protein which comprises a region which comprises the CCP6 domain of C4BP alpha chain, a region which comprises an oligomerization domain and one or more regions that comprise sequences which does not form part of C4BP. Any of these regions can be in any order with respect to each other.

Examples of recombinant polypeptides that comprise a peptide that is not part of the C4BP alpha chain can be, for example, fusion proteins that improve pharmacokinetic properties.

The recombinant polypeptide of the invention may be modified in order to modulate affinity for the receptor, modulate circulating half-life, modulate therapeutic half-life, modulate stability of the polypeptide, modulate cleavage by proteases, modulate dose, modulate release or bio-availability, facilitate purification, or improve or alter a particular route of administration. Similarly, the polypeptide may comprise protease cleavage sequences, reactive groups, or other molecules that improve detection, purification or other traits of the polypeptide. Preferably, the polypeptide comprises MBP sequences or AFV, slyD, tsf, SUMO, Bla, or GST sequences that are cleaved by proteases.

In a preferred embodiment, the peptide that is not part of the C4BP alpha chain is a tag peptide.

The expression "tag peptide", as used herein, refers to a peptide suitable for detection, isolation and/or purification of the recombinant polypeptide. Non-limiting examples of tags include an affinity purification tag such as polyhistidine [poly(His)] sequences; peptide sequences capable of being recognized by antibodies that may be used to purify the resultant fusion protein by immunoaffinity chromatography, for example epitopes derived from the hemagglutinin of the fever virus, c-myc-tag (recognized by an anti-c-myc antibody); streptavidin-binding peptide tag or SBP-tag; Stag; calmodulin-binding peptide (CBP); cellulose-binding domain; chitin-binding domain (CBD); glutathione S-transferase-tag; maltose-binding protein (MBP); 3xHA tag or hemagglutinin tag; NusA; TrxA; DsbA; Avi-tag; Strep-tag; an arginine tag (Arg-tag); FLAG-tag, etc. (Zhao et al. 2013. J. Anal. Methods Chemistry, 2013:581093; Terpe K. 2003. Appl. Microbiol. Biotechnol. 60(5):523-533). Said affinity purification tag can be fused directly in-line or, alternatively, fused to the monomer polypeptide via a cleavable linker, i.e., a peptide segment containing an amino acid sequence that is specifically cleavable by enzymatic or chemical means (i.e., a recognition/cleavage site). In a particular embodiment, said cleavable linker comprises an amino acid sequence which is cleavable by a protease (or protease recognition site) once the protein has been translated. As illustrative, the cleavable linker can be an amino acid sequence cleavable by a protease such as enterokinase, Arg C endoprotease, Glu C endoprotease, Lys C endoprotease, factor Xa, thrombin, TEV (tobacco etch virus) protease, human rhinovirus 3C protease, sortase A, PreScission protease (Zhao et al. 2013. J. Anal. Methods Chem. 2013:581093), SUMO proteases (Butt TR. et al. 2005. Protein Expr. Purif. 43(1):1-9), etc.; alternatively, in another particular embodiment, said cleavable linker comprises an amino acid sequence which is cleavable by a chemical reagent, such as, for example, cyanogen bromide which cleaves methionine residues, or any other suitable chemical reagent. In another embodiment, the cleavable linker is an intein. In a preferred embodiment, the cleavable linker is an amino acid sequence cleavable by enterokinase, preferably is the sequence DDDDK (SEQ ID NO: 59). The cleavable linker is useful if subsequent removal of the affinity purification tag is desirable. The tag may be located at any position of the monomer, particularly C-terminally or N-terminally to the CCP6 domain of C4BP alpha chain and to the oligomerization domain. In a more preferred embodiment, the tag peptide is linked to the N-terminal end of the CCP6 domain of C4BP alpha chain. In a preferred embodiment the tag is linked to the polypeptide via a linker cleavable by a TEV protease. In another embodiment the tag is linked to the polypeptide via a linker cleavable by enterokinase. In another embodiment, the tag is fused directly in-line with the polypeptide. In a more preferred embodiment, the tag is a poly(His) tag, preferably a poly(His) tag having 2, 3, 4, 5, 6, 7, 8, 9, 10, 11, 12, 13, 14, 15, 16, 17, 18, 19, 20 or more than 20 histidines. In a preferred embodiment, the tag is a hexahistidine tag or His6-tag. In another embodiment, the poly(His) tag has at least 2 histidines, preferably at least 5 histidines, more preferably at least 6 histidines.

Preferably, the tag is a poly(His)tag, more preferably a His6-tag, and the tag is linked to the polypeptide via a linker cleavable by a TEV protease.

In another embodiment, the tag is a Strep-tag II peptide of SEQ ID NO: 60. WSHPQFEK (SEQ ID NO: 60)

The person skilled in the art will understand that the polypeptide of the invention can comprise more than one tag. Therefore, the recombinant polypeptide of the invention can have 1, 2, 3, 4, 5, 6 or more tags, preferably has 2 tags, more preferably has one tag. In a preferred embodiment, the recombinant polypeptide of the invention has a poly(His)tag and a Strep-tag II.

In an embodiment, the polypeptide of the invention is a fusion protein which comprises a region which comprises the CCP6 domain of C4BP alpha chain, a region which comprises an oligomerization domain and one or more tag peptides. Any of these regions can be in any order with respect to each other. Thus, in an embodiment, the polypeptide of the invention is a fusion protein which comprises in an amino terminal to carboxy terminal direction, (a) at least a tag peptide, (b) the region which comprises the CCP6 domain of C4BP alpha chain, and (c) the region which comprises the oligomerization domain. In another embodiment, the polypeptide of the invention is a fusion protein which comprises in an amino terminal to carboxy terminal direction, (a) the region which comprises the CCP6 domain of C4BP alpha chain, (b) the region which comprises the oligomerization domain, and (c) at least a tag peptide. In another embodiment, the polypeptide of the invention is a fusion protein which comprises in an amino terminal to carboxy terminal direction, (a) the region which comprises the CCP6 domain of C4BP alpha chain, (b) at least a tag peptide, and (c) the region which comprises the oligomerization domain. In another embodiment, the polypeptide of the invention is a fusion protein which comprises in an amino terminal to carboxy terminal direction (a) at least a tag peptide, (b) the region which comprises the oligomerization domain, and (c) the region which comprises the CCP6 domain of C4BP alpha chain. In another embodiment, the polypeptide of the invention is a fusion protein which comprises in an amino terminal to carboxy terminal direction (a) the region which comprises the oligomerization domain, and (b) the region which comprises the CCP6 domain of C4BP alpha chain, and (c) at least a tag peptide. In another embodiment, the polypeptide of the invention is a fusion protein which comprises in an amino terminal to carboxy terminal direction, (a) the region which comprises the oligomerization domain, (b) at least a tag peptide, and (c) the region which comprises the CCP6 domain of C4BP alpha chain. The person skilled in the art will understand that, as used herein, the reference to "a tag peptide" refers both to a tag containing a cleavable linker or directly fused to the recombinant polypeptide. The person skilled in the art will understand that the expression "at least a tag peptide" means that the tag peptide of the recombinant polypeptide can be 1, 2, 3, 4, 5, 6 or more tag peptides, preferably 2 tag peptides, more preferably 1 tag peptides. The person skilled in the art will understand that all these embodiments may comprise an additional methionine at the N-terminal end of the polypeptide. All these embodiments are equally applicable when the polypeptide of the invention consists of at least a tag peptide, a region consisting of the CCP6 domain of C4BP alpha chain, and a region consisting of the oligomerization domain. All these embodiments apply equally when the polypeptide of the invention is formed by functionally equivalent variants of the CCP6 domain of the C4BP alpha chain and/or functionally equivalent variants of the oligomerization domain.

In an embodiment, the polypeptide of the invention is a fusion protein which comprises a region which comprises the CCP6 domain of C4BP alpha chain, a region which comprises an oligomerization domain, at least a tag peptide and a signal peptide. Any of these regions can be in any order with respect to each other. In an embodiment, the polypeptide of the invention is a fusion protein which comprises in an amino terminal to carboxy terminal direction, (a) a signal peptide, (b) at least a tag peptide, (c) a region which comprises the CCP6 domain of C4BP alpha chain, and (d) a region which comprises an oligomerization domain. In an embodiment, the polypeptide of the invention is a fusion protein which comprises in an amino terminal to carboxy terminal direction, (a) a signal peptide, (b) at least a tag peptide, (c) a region which comprises an oligomerization domain, and (d) a region which comprises the CCP6 domain of C4BP alpha chain. In an embodiment, the polypeptide of the invention is a fusion protein which comprises in an amino terminal to carboxy terminal direction, (a) a signal peptide, (b) a region which comprises an oligomerization domain, (c) at least a tag peptide, and (d) a region which comprises the CCP6 domain of C4BP alpha chain. In an embodiment, the polypeptide of the invention is a fusion protein which comprises in an amino terminal to carboxy terminal direction, (a) a signal peptide, (b) a region which comprises the CCP6 domain of C4BP alpha chain, (c) at least a tag peptide, and (d) a region which comprises an oligomerization domain. In an embodiment, the polypeptide of the invention is a fusion protein which comprises in an amino terminal to carboxy terminal direction, (a) a signal peptide, (b) a region which comprises the CCP6 domain of C4BP alpha chain, (c) a region which comprises an oligomerization domain, and (d) at least a tag peptide. In an embodiment, the polypeptide of the invention is a fusion protein which comprises in an amino terminal to carboxy terminal direction, (a) a signal peptide, (b) a region which comprises an oligomerization domain, (c) a region which comprises the CCP6 domain of C4BP alpha chain, and (d) at least a tag peptide. The person skilled in the art will understand that, as used herein, the reference to "a tag peptide" refers both to a tag peptide containing a cleavable linker or directly fused to the recombinant polypeptide. The person skilled in the art will understand that the expression "at least a tag peptide" means that the tag peptide of the recombinant polypeptide can be 1, 2, 3, 4, 5, 6 or more tag peptides, preferably 2 tag peptides, more preferably 1 tag peptides. All these embodiments are equally applicable when the polypeptide of the invention consists of a signal peptide, at least a tag peptide, a region consisting of the CCP6 domain of C4BP alpha chain, and a region consisting of the oligomerization domain. All these embodiments apply equally when the polypeptide of the invention is formed by functionally equivalent variants of the CCP6 domain of the C4BP alpha chain and/or functionally equivalent variants of the oligomerization domain.

In a preferred embodiment, the recombinant polypeptide of the invention comprises SEQ ID NO: 6 or a functionally equivalent variant thereof, preferably comprises SEQ ID NO: 6. In a preferred embodiment the recombinant polypeptide of the invention consists of SEQ ID NO: 6 or a functionally equivalent variant thereof; more preferably consists of SEQ ID NO: 6.

HHHHHHLCCPEPKLNNGEITQHRKSRPANHCVYFYGDEISFSCHETSRFSAICQG
DGTWSPRTPSCGDETPEGCEQVLTGKRLMQCLPNPEDVKMALEVYKLSLEIEQ
LELQRDSARQSTLDKEL (SEQ ID NO: 6)

In another embodiment, the recombinant polypeptide of the invention consists of the sequence SEQ ID NO: 6 and an additional methionine at the N-terminal end of the polypeptide.

In another embodiment, the polypeptide of the invention comprises SEQ ID NO: 7 or a functionally equivalent variant thereof, preferably comprises SEQ ID NO: 7. In a preferred embodiment, the polypeptide of the invention consists of SEQ ID NO: 7 or a functionally equivalent variant thereof; more preferably consists of SEQ ID NO: 7.

MHPPKTPSGALHRKRKMAAWPFSRLWKVSDPILFQMTLIAALLPAVLGHHHH
HHLCCPEPKLNNGEITQHRKSRPANHCVYFYGDEISFSCHETSRFSAICQGDGT
WSPRTPSCGDETPEGCEQVLTGKRLMQCLPNPEDVKMALEVYKLSLEIEQLEL
QRDSARQSTLDKEL (SEQ ID NO: 7)

In another embodiment, the recombinant polypeptide of the invention does not comprise a tag peptide; preferably does not comprise a tag peptide and a signal peptide.

In a preferred embodiment, the polypeptide of the invention comprises or consists of, preferably consists of, in an amino-terminal to carboxy-terminal direction of (a) a signal peptide, (b) at least a tag peptide, (c) a cleavable linker, (d) the CCP6 domain of the C4BP alpha chain, and (e) the oligomerization domain. In a preferred embodiment, the polypeptide of the invention comprises or consists of, preferably consists of, in an amino-terminal to carboxy-terminal direction of (a) a signal peptide, (b) one tag peptide, (c) a cleavable linker, (d) the CCP6 domain of the C4BP alpha chain, and (e) the oligomerization domain. In a preferred embodiment, the polypeptide of the invention comprises or consists of, preferably consists of, in an amino terminal to carboxy-terminal direction of (a) the signal peptide of CB4P alpha chain, (b) a poly(His)tag, (c) a linker cleavable by TEV, (d) the CCP6 domain of the C4BP alpha chain, and (e) the oligomerization domain of C4BP alpha chain. In a preferred embodiment, the polypeptide of the invention comprises or consists of, preferably consists of, SEQ ID NO: 56.

MHPPKTPSGALHRKRKMAAWPFSRLWKVSDPILFQMTLIAALLPAVLGHHHH
HHENLYFQGLCCPEPKLNNGEITQHRKSRPANHCVYFYGDEISFSCHETSRFSAI
CQGDGTWSPRTPSCGDETPEGCEQVLTGKRLMQCLPNPEDVKMALEVYKLSLE
IEQLELQRDSARQSTLDKEL (SEQ ID NO: 56)

In another preferred embodiment, the recombinant polypeptide of the invention comprises or consists of, preferably consists of, in an amino-terminal to carboxy-terminal direction of (a) at least a tag peptide, (b) a cleavable linker, (c) the CCP6 domain of the C4BP alpha chain, and (d) the oligomerization domain. In another preferred embodiment, the recombinant polypeptide of the invention comprises or consists of, preferably consists of, in an amino-terminal to carboxy-terminal direction of (a) one tag peptide, (b) a cleavable linker, (c) the CCP6 domain of the C4BP alpha chain, and (d) the oligomerization domain. In a preferred embodiment, the recombinant polypeptide of the invention comprises or consists of, preferably consists of, in an amino-terminal to carboxy-terminal direction, of (a) a poly(His)tag, (b) a linker cleavable by TEV, (c) the CCP6 domain of the C4BP alpha chain, and (d) the oligomerization domain of C4BP alpha chain. The person skilled in the art will understand that all these embodiments are applicable to recombinant polypeptides having a methionine at the N-terminal end. In a preferred embodiment, the polypeptide of the invention comprises or consists of, preferably consists of, SEQ ID NO: 57.

HHHHHHENLYFQGLCCPEPKLNNGEITQHRKSRPANHCVYFYGDEISFSCHETS
RFSAICQGDGTWSPRTPSCGDETPEGCEQVLTGKRLMQCLPNPEDVKMALEVY
KLSLEIEQLELQRDSARQSTLDKEL (SEQ ID NO: 57)

In another embodiment, the recombinant polypeptide of the invention consists of the sequence SEQ ID NO: 57 and an additional methionine at the N-terminal end of the polypeptide.

In another preferred embodiment, the recombinant polypeptide of the invention comprises or consists of, preferably consists of, in an amino-terminal to carboxy-terminal direction of (a) a glycine residue, (b) the CCP6 domain of the C4BP alpha chain, and (c) the oligomerization domain, preferably the oligomerization domain of C4BP alpha chain. In a preferred embodiment, the polypeptide of the invention comprises or consists of, preferably consists of, SEQ ID NO: 58.

GLCCPEPKLNNGEITQHRKSRPANHCVYFYGDEISFSCHETSRFSAICQGDGTWS
PRTPSCGDETPEGCEQVLTGKRLMQCLPNPEDVKMALEVYKLSLEIEQLELQRD
SARQSTLDKEL (SEQ ID NO: 58)

In a preferred embodiment, the recombinant polypeptide of the invention comprises or consists of, preferably consists of, in an amino-terminal to carboxy-terminal direction of (a) a methionine or a signal peptide, (b) two tag peptides, (c) a cleavable linker, (d) the CCP6 domain of the C4BP alpha chain, and (e) the oligomerization domain. In a more preferred embodiment, the recombinant polypeptide of the invention comprises or consists of, preferably consists of, in an amino-terminal to carboxy-terminal direction of (a) a methionine or a signal peptide, (b) a His6-tag, (c) a Strep-tag II, (d) a linker cleavable by enterokinase, (e) the CCP6 domain of the human C4BP alpha chain, and (f) the oligomerization domain of human C4BP alpha chain. In a preferred embodiment, the recombinant polypeptide of the invention consists of, in an amino-terminal to carboxy-terminal direction, of (a) a methionine, (b) a His6-tag, (c) a Strep-tag II of SEQ ID NO: 60, (d) a linker cleavable by enterokinase of SEQ ID NO: 59, (e) the CCP6 domain of the human C4BP alpha chain, and (f) the oligomerization domain of human C4BP alpha chain. In another preferred embodiment, the recombinant polypeptide of the invention consists of, in an amino-terminal to carboxy-terminal direction, of (a) a signal peptide, (b) a His6-tag, (c) a Strep-tag II of SEQ ID NO: 60, (d) a linker cleavable by enterokinase of SEQ ID NO: 59, (e) the CCP6 domain of the human C4BP alpha chain, and (f) the oligomerization domain of human C4BP alpha chain. In a preferred embodiment, the recombinant polypeptide of the invention comprises SEQ ID NO: 61. In a more preferred embodiment, the recombinant polypeptide of the invention consists of SEQ ID NO: 61.

MHHHHHHWSH PQFEKDDDDK LCCPEPKLNN GEITQHRKSR PANHCVYFYG
DEISFSCHET SRFSAICQGD GTWSPRTPSC GDETPEGCEQ VLTGKRLMQC
LPNPEDVKMA LEVYKLSLEI EQLELQRDSA RQSTLDKEL (SEQ ID NO: 61)

Preferably, the recombinant polypeptide of the invention has a size between 75 and 1000 amino acids, more preferably between 100 and 800 amino acids, more preferably between 100 and 600 amino acids, more preferably between 110 and 550 amino acids, even more preferably between 115 and 550 amino acids, even more preferably between 119 and 550 amino acids. In an embodiment, the recombinant polypeptide of the invention preferably has at least 90 amino acids, at least 100 amino acids, at least 110 amino acids, at least 115 amino acids, at least 119 amino acids, at least 120 amino acids, at least 130 amino acids, at least 150 amino acids. In a preferred embodiment, the recombinant polypeptide of the invention has a size between 115 and 650 amino acids, preferably between 119 and 550 amino acids.

In a preferred embodiment, when insertions are made, preferably the size of the polypeptide does not exceed the length of the wild type sequence of C4BP alpha chain by more than 20, preferably by no more than 15, more preferably by no more than 10, amino acids. Thus, for example, when modified by insertion the protein containing the CCP6 domain of human C4BP alpha chain will desirably be no more than 617 amino acids.

The person skilled in the art will understand that the different domains of the recombinant polypeptide of the invention may be joined by spacers. As disclosed herein a spacer is an insert connecting or linking peptide of suitable length and character. In general, said spacer acts as a hinge region between said domains, allowing them to move independently from one another while maintaining the three-dimensional form of the individual domains. In this sense, a preferred spacer would be a hinge region characterized by a structural ductility or flexibility allowing this movement. The length of the spacer can vary. Typically, the number of amino acids in the spacer is 100 or less amino acids, preferably 50 or less amino acids, more preferably 40 or less amino acids, still more preferably 30 or less amino acids, or even more preferably 20 or less amino acids.

Alternatively, a suitable spacer can be based on the sequence of 10 amino acid residues of the upper hinge region of murine IgG3; which has been used for the production of dimerized antibodies by means of a coiled coil (Pack P. and Pluckthum, A., 1992, Biochemistry 31:1579-1584) and can be useful as a spacer peptide according to the present invention. It can also be a corresponding sequence of the upper hinge region of human IgG3 or other human Ig subclasses (IgG1, IgG2, IgG4, IgM and IgA). The sequences of human Igs are not expected to be immunogenic in human beings. Additional spacers that can be used in the instant invention include the peptides of the amino acid sequences GAP, AAA.

In a particular embodiment, said spacer is a peptide having structural flexibility (i.e., a flexible linking peptide or "flexible linker"). Generally, such linkers are a few amino acids in length, such as from 1 to 20, e.g., from 2 to 10 amino acids in length. These amino acids are selected from the group consisting of glycine, serine, alanine and threonine. In another particular embodiment, the flexible linker is a peptide containing repeats of amino acid residues, particularly Gly and Ser, or any other suitable repeats of amino acid residues. Virtually any flexible linker can be used as spacer according to the invention. One such linker is a (Glyₘ-Ser)ₙ linker, where m and n are each independently from 1 to 4. These are used in the art to attach protein domains to each other. Thus, the first component may be linked to the second by such a linker.

In a preferred embodiment, the CCP6 domain of C4BP alpha chain and the oligomerization domain are directly linked. In another embodiment, they are linked through a flexible linker. In a more preferred embodiment, the flexible linker is at least 1, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20 residues.

It will be understood that sometimes it will be necessary to incorporate a sequence of amino acids suitable for expression of the protein. This will include at least an N-terminal methionine. The N-terminal sequence may include a cleavage site for chemical or enzymatic removal of all or part of the sequence.

The recombinant polypeptide of the invention can be modified to include any of a variety of known chemical groups or molecules. Such modifications include, but are not limited to, glycosylation, acetylation, acylation, ADP-ribosylation, amidation, covalent attachment to polyethylene glycol (e.g., PEGylation), covalent attachment of flavin, covalent attachment of a heme moiety, covalent attachment of a nucleotide or nucleotide derivative, covalent attachment of a lipid or lipid derivative, covalent attachment of phosphatidylinositol, cross-linking, cyclization, disulfide bond formation, demethylation, formation of covalent crosslinks, formation of cystine, formation of pyroglutamate, formylation, gamma carboxylation, glycosylation, GPI anchor formation, hydroxylation, iodination, methylation, esterification, myristoylation, oxidation, proteolytic processing, phosphorylation, prenylation, racemization, selenoylation, sulfation, ubiquitination, modifications with fatty acids, transfer-RNA mediated addition of amino acids to proteins such as arginylation, etc. Analogues of an amino acid (including unnatural amino acids) and peptides with substituted linkages are also included.

The recombinant polypeptide of the invention can be obtained by recombinant DNA techniques known to the person skilled in the art. Briefly, the recombinant nucleic acid encoding the CCP6 domain of C4BP alpha chain and the oligomerization domain is expressed in eukaryotic or prokaryotic cells and the product is recovered.

It should be understood that expression of polypeptides often involves post-translational modifications, such as cleavage of the signal sequence and/or cleavage of the tag peptide.

The person skilled in the art will understand that the recombinant polypeptide of the invention as a whole needs to substantially preserve the ability to induce a tolerogenic phenotype of the original CCP6 domain of C4BP alpha chain and the ability to form oligomers of the original oligomerization domain, particularly, the ability to induce a tolerogenic phenotype of the CCP6 domain of human C4BP alpha chain and the ability to form oligomers of the oligomerization domain of C4BP alpha chain, more particularly the ability to induce a tolerogenic phenotype and the ability to form oligomers of the recombinant polypeptide of SEQ ID NO: 6.

All the embodiments referring to the CCP6 domain of the C4BP alpha chain or the oligomerization domain apply equally to functionally equivalent variants of the CCP6 domain of the C4BP alpha chain and to functionally equivalent variants of the oligomerization domain.

### Homooligomer of the invention

The authors of the present invention have found that the recombinant polypeptide of the invention oligomerizes forming an homooligomer of seven identical monomers, i.e., seven recombinant polypeptides of the invention. The inventors have found that the homooligomer of the invention is potentially very attractive as pharmaceutical active principle since it is able to modulate the maturation of mononuclear phagocytes, mainly dendritic cells (DCs) and macrophages, with high specificity and superior efficiency compared with the physiological isoform of C4BP (C4BP(β-)) as shown in gene expression experiments (Figures 13, 14, 16, 17 and 18) and in the conversion to regulatory or tolerogenic macrophages (Figure 15).

Therefore, in an aspect, the present invention relates to an homooligomer of at least six recombinant polypeptides comprising the CCP6 domain of the C4BP alpha chain or a functionally equivalent variant thereof and an oligomerization domain, wherein said polypeptides does not comprise CCP1, CCP2, CCP3, CCP4, CCP5, CCP7 nor CCP8 domains of C4BP alpha chain.

The expression "homooligomer", as used herein, refers to an oligomer formed by identical monomers. An oligomer, as used herein, is a macromolecular complex formed by non-covalent bonding of proteins or polypeptides that consist of a few repeating units. Each of the repeating units is a monomer. Particularly, the homooligomer of the present invention is an hexamer or an heptamer, composed of six or seven monomers, respectively. Each of the monomers of the homooligomers of the invention is a recombinant polypeptide according to the invention.

Thus, the homooligomers which are useful in the present invention include any homooligomer resulting from the association of a plurality of recombinant polypeptides of the invention and which are devoid of β-chain. For instance, the homooligomers of the present invention may contain at least six, at least seven or at least eight monomers, i.e., recombinant polypeptides of the invention. In an embodiment, the homooligomer is formed by seven recombinant polypeptides of the invention.

In an embodiment, the homooligomers of the invention comprise at least six recombinant polypeptides of the invention; particularly comprise six, seven or eight polypeptides of the invention; particularly comprise six or seven polypeptides of the invention, more particularly comprise seven polypeptides of the invention.

In a preferred embodiment, the homooligomers of the invention consist of six,seven or eight polypeptides of the invention. In a more preferred embodiment, the homooligomers of the invention consist of six or seven polypeptides of the invention.

In an embodiment, the homooligomer of the invention consists of six monomer chains, i.e., consists of six recombinant polypeptides of the invention.

In a preferred embodiment, the homooligomer of the invention consists of seven monomer chains, i.e., consists of seven polypeptides of the invention.

In an embodiment, the homooligomer consists of seven recombinant polypeptides of the invention wherein each recombinant polypeptide comprises, in an amino-terminal to carboxy-terminal direction, the CCP6 domain of C4BP alpha chain or a functionally equivalent variant thereof and the oligomerization domain. In a preferred embodiment, the homooligomer consists of seven recombinant polypeptides of the invention, wherein each recombinant polypeptide consists of, in an amino-terminal to carboxy-terminal direction, the CCP6 domain of C4BP alpha chain and the oligomerization domain of C4BP alpha chain. In a more preferred embodiment, the homooligomer consists of seven recombinant polypeptides of the invention, wherein each recombinant polypeptide consists of, in an amino-terminal to carboxy-terminal direction, the CCP6 domain of human C4BP alpha chain and the oligomerization domain of human C4BP alpha chain. In this last embodiment the CCP6 domain and the oligomerization domain are directly linked.

In a preferred embodiment, the polypeptide that forms the homooligomer is the polypeptide consisting of SEQ ID NO: 6 or a functionally equivalent variant thereof, particularly the polypeptide consisting of SEQ ID NO: 6.

All the terms and embodiments previously described in the context of the recombinant polypeptide of the invention are equally applicable to the homooligomer of the invention.

The obtention of homooligomers involves, for example, creating a recombinant sequence encoding the CCP6 domain of C4BP alpha chain and the oligomerization domain in which the 3' end of a DNA sequence encoding the CCP6 domain is ligated to the 5' end of a DNA sequence encoding the oligomerization domain. Upon expression in an appropriate host, this hybrid DNA sequence will produce the recombinant polypeptide of the invention that will assemble into an oligomer, particularly into an homooligomer, more particularly into an heptamer formed by seven identical recombinant polypeptides.

In a preferred embodiment, the recombinant polypeptides forming the homooligomers of the invention do not comprise a region of a protein different from C4BP. For example, the polypeptide of the invention cannot be a fusion protein comprising a region which forms part of a different protein from C4BP.

The person skilled in the art will understand that the homooligomers of the invention are usually formed by the mature form of the recombinant polypeptides of the invention, i.e., that the signal peptide of the recombinant polypeptide of the invention is usually cleaved before it is secreted. Therefore, in a preferred embodiment, the homooligomers of the invention are formed by recombinant polypeptides not containing the signal polypeptide.

On the other hand, the homooligomers of the invention can be formed by recombinant polypeptides maintaining the tag peptide. This is the case for small-size tags such as His(6) tag, FLAG, Strep II and CBP, which usually do not need to be removed. Therefore, in an embodiment, the homooligomers of the invention are formed by recombinant polypeptides containing at least a tag peptide.

However, in some instances, it can be preferred to remove the tag peptide. Preferably, the tag peptide is cleaved in the mature form of the recombinant polypeptides forming homooligomers. Therefore, in another embodiment, the homooligomers of the invention are formed by recombinant polypeptides not containing tag peptides. For example, if the poly(His) tag is fused to the CCP6 domain of C4BP alpha chain by a linker cleavable by TEV protease, wherein the sequence recognized by the protease is ENLYFQ/G (SEQ ID NO: 59), the mature form of the recombinant polypeptide of the invention that forms the homooligomers of the invention can have a glycine residue at the N-terminal end, since the TEV protease cleaves between Q and G residues. This residue of glycine does not affect the activity of the recombinant polypeptide of the invention. Thus, the recombinant polypeptides of the invention forming the homooligomers of the invention can have additional amino acids at its N-terminal end as a result of the cleavage of a cleavable linker that does not affect its activity or that have been added to allow its expression in bacteria (for example, an additional methionine).

Therefore, in an embodiment, the recombinant polypeptides of the homooligomers of the invention have an additional amino acid at its N-terminal end.

In an embodiment, the recombinant polypeptides of the homooligomers of the invention does not comprise a signal peptide and a tag peptide.

The homooligomers of the invention associate spontaneously in the recombinant expression system used. Methods to assess if a complex is an homooligomer of the invention have been disclosed previously in the context of the variants of the oligomerization domains.

### Polynucleotides, vectors and host cells of the invention

The invention provides, as well, polynucleotides encoding the polypeptides of the invention. Thus, in another aspect, the invention relates to a polynucleotide encoding a recombinant polypeptide comprising the CCP6 domain of the C4BP alpha chain or a functionally equivalent variant thereof and an oligomerization domain, wherein said polypeptide does not comprise CCP1, CCP2, CCP3, CCP4, CCP5, CCP7 nor CCP8 domains of the C4BP alpha chain.

The terms "polynucleotide", "nucleic acid" and "nucleic acid molecule" are used interchangeably to refer to polymeric forms of nucleotides of any length. The polynucleotides may contain deoxyribonucleotides, ribonucleotides, and/or their analogues. Nucleotides may have any three-dimensional structure, and may perform any function, known or unknown. The term "polynucleotide" includes, for example, single-stranded, double-stranded and triple helical molecules, a gene or gene fragment, exons, introns, mRNA, tRNA, rRNA, ribozymes, cDNA, recombinant polynucleotides, branched polynucleotides, plasmids, vectors, isolated DNA of any sequence, isolated RNA of any sequence, nucleic acid probes, and primers. In addition to a native nucleic acid molecule, a nucleic acid molecule of the present invention may also comprise modified nucleic acid molecules. As used herein, mRNA refers to an RNA that can be translated in a cell.

The polynucleotides of the invention may further comprise a single promoter region regulating the transcription of the region encoding the polypeptides of the invention provided that said promoters are compatible with the cells in which the polypeptides are to be expressed. The polynucleotides encoding for the polypeptides of the invention can be found isolated as such or forming part of vectors allowing the propagation of said polynucleotides in suitable host cells.

Therefore, in another aspect, the invention relates to a vector comprising a polynucleotide according to the invention

The choice of expression vector will depend upon the choice of host. Vectors suitable for the insertion of said polynucleotides are vectors derived from expression vectors in prokaryotes such as pUC18, pUC19, Bluescript and the derivatives thereof, pET and the derivatives thereof, mp18, mp19, pBR322, pMB9, ColE1, pCR1, RP4, phages and "shuttle" vectors such as pSA3 and pAT28, expression vectors in yeasts such as vectors of the type of 2 micron plasmids, integration plasmids, YEP vectors, pBGZα vector, centromere plasmids and the like, expression vectors in insect cells such as vectors of the pAC series and of the pVL, expression vectors in plants such as pIBI, pEarleyGate, pAVA, pCAMBIA, pGSA, pGWB, pMDC, pMY, pORE series and the like, and expression vectors in eukaryotic cells, including baculovirus suitable for transfecting insect cells using any commercially available baculovirus system. The vectors for eukaryotic cells include preferably viral vectors (adenoviruses, viruses associated to adenoviruses (AAV), retroviruses and, particularly, lentiviruses) as well as non-viral vectors such as pSilencer 4.1-CMV (Ambion), pcDNA3, pcDNA3.1/hyg, pHMCV/Zeo, pCR3.1, pEFI/His, pIND/GS, pRc/HCMV2, pSV40/Zeo2, pTRACER-HCMV, pUB6/V5-His, pVAX1, pZeoSV2, pCI, pSVL and PKSV-10, pBPV-1, pML2d and pTDT1. In a preferred embodiment, the vector is pcDNA3.1.

The vectors may also comprise a reporter or marker gene which allows identifying those cells that have been incorporated the vector after having been put in contact with it. Useful reporter genes in the context of the present invention include lacZ, luciferase, thymidine kinase, GFP and the like. Useful marker genes in the context of this invention include, for example, the neomycin resistance gene, conferring resistance to the aminoglycoside G418; the hygromycin phosphotransferase gene, conferring resistance to hygromycin; the ODC gene, conferring resistance to the inhibitor of the ornithine decarboxylase (2-(difluoromethyl)-DL-ornithine (DFMO); the dihydrofolate reductase gene, conferring resistance to methotrexate; the puromycin-N-acetyl transferase gene, conferring resistance to puromycin; the ble gene, conferring resistance to zeocin; the adenosine deaminase gene, conferring resistance to 9-beta-D-xylofuranose adenine; the cytosine deaminase gene, allowing the cells to grow in the presence of N-(phosphonacetyl)-L-aspartate; thymidine kinase, allowing the cells to grow in the presence of aminopterin; the xanthine-guanine phosphoribosyltransferase gene, allowing the cells to grow in the presence of xanthine and the absence of guanine; the trpB gene of *E. coli,* allowing the cells to grow in the presence of indol instead of tryptophan; the hisD gene of *E. coli,* allowing the cells to use histidinol instead of histidine. The selection gene is incorporated into a plasmid that can additionally include a promoter suitable for the expression of said gene in eukaryotic cells (for example, the CMV or SV40 promoters), an optimized translation initiation site (for example, a site following the so-called Kozak's rules or an IRES), a polyadenylation site such as, for example, the SV40 polyadenylation or phosphoglycerate kinase site, introns such as, for example, the beta-globulin gene intron. Alternatively, it is possible to use a combination of both the reporter gene and the marker gene simultaneously in the same vector.

Vectors that contain both a promoter and a cloning site into which a polynucleotide according to the invention can be operatively linked are also provided in the present invention. Such vectors are capable of transcribing RNA *in vitro* or *in vivo.* In order to optimize expression and/or *in vitro* transcription, it may be necessary to remove, add or alter 5' and/or 3' untranslated portions of the clones to eliminate extra, potential inappropriate alternative translation initiation codons or other sequences that may interfere with or reduce expression, either at the level of transcription or translation. Alternatively, consensus ribosome binding sites can be inserted immediately 5' of the start codon to enhance expression. Gene delivery vehicles also include several non-viral vectors, including DNA/liposome complexes, and targeted viral protein-DNA complexes. Liposomes that also comprise a targeting antibody or fragment thereof can be used in the methods of this invention. To enhance delivery to a cell, nucleic acids or proteins of this invention can be conjugated to antibodies or binding fragments thereof which bind cell surface antigens.

As is well known in the art, for expression of the DNA sequences of this invention, the DNA sequence should be operatively linked to an expression control sequence in an appropriate expression vector and employed in that expression vector to transform an appropriate unicellular host. Such operative linking of a DNA sequence of this invention to an expression control sequence, of course, includes the provision of a translation start signal in the correct reading frame upstream of the DNA sequence. If a particular DNA sequence being expressed does not begin with a methionine, the start signal will result in an additional amino acid (methionine) being located at the N-terminus of the product. While such a methionyl-containing product may be employed directly in the methods of the invention, it is usually more desirable to remove the methionine before use. Methods are known to those skilled in the art to remove such N-terminal methionine from polypeptides expressed with them. For example, certain hosts and fermentation conditions permit removal of substantially all of the N-terminal methionine *in vivo.* Other hosts require *in vitro* removal of the N-terminal methionine. However, such *in vitro* and *in vivo* methods are well known in the art.

In another aspect the invention relates to a host cell comprising a vector as described previously. The term "host cell" is used such that it refers not only to the particular subject cell, but to the progeny or potential progeny of such a cell. Because certain modifications may occur in succeeding generations due to either mutation or environmental influences, such progeny may not, in fact, be identical to the parent cell, but are still included within the scope of the term as used herein.

The recombinant polypeptide of the invention can be expressed in any of the recombinant polypeptide production systems known.

A wide variety of unicellular host cells are also useful in expressing the DNA sequences of this invention. In a preferred embodiment, the host cells are selected from bacteria, yeasts and mammalian eukaryotic cells. These hosts include well known eukaryotic and prokaryotic hosts, such as strains of *E.coli, Pseudomonas, Bacillus, Lactobacillus, Thermophilus, Salmonella, Enterobacteriaceae or Streptomyces,* fungi, such as yeasts, and animal cells, such as CHO and mouse cells, African green monkey cells, such as cos-1, COS-7, BSC 1, BSC 40, and BMT 10, insect cells, and human cells and plant cells in tissue culture. For animal cell expression, CHO, COS-7 cells and HEK293 cells are preferred, more preferably HEK293 cells.

For example, if *E.coli* from the genera *Escherichia* is used in the method of the invention, preferred strains of this bacterium to use would include BL21(DE3) and their derivatives including C41(DE3); C43(DE3)or C0214(DE3), C3030H, or other strains resistant to the toxicity of recombinant protein expression. Even more preferably, derivatives of these strains lacking the prophage DE3 may be used when the promoter is not the T7 promoter.

In a preferred embodiment, the expression cassette comprises from 5' to 3' in the same open reading frame a nucleotide sequence encoding the CCP6 domain of C4BP alpha chain and a nucleotide sequence encoding an oligomerization domain. As it is used herein, the term "open reading frame" or "ORF" means a length of nucleic acid, either DNA, cDNA or RNA, that comprises a translation start signal or initiation codon, such as an ATG or AUG, and a termination codon and can be potentially translated into a polypeptide sequence. Said DNA sequence does not contain any internal end codon and can generally be translated into a peptide.

In a preferred embodiment, the expression cassette comprises from 5' to 3' a nucleotide sequence encoding a signal peptide, a nucleotide sequence encoding a first tag, a nucleotide sequence encoding a selectable marker, a nucleotide sequence encoding a second tag, a nucleotide sequence encoding a CCP6 domain of C4BP alpha chain and a nucleotide sequence encoding an oligomerization domain. In another embodiment, the expression cassette comprises from 5' to 3' a nucleotide sequence encoding a signal peptide, a nucleotide sequence encoding a first tag, a nucleotide sequence encoding a second tag, a nucleotide sequence encoding a cleavable linker, a nucleotide sequence encoding a CCP6 domain of C4BP alpha chain and a nucleotide sequence encoding an oligomerization domain. In another embodiment, the expression cassette comprises from 5' to 3' a nucleotide sequence encoding a signal peptide, a nucleotide sequence encoding a tag, a nucleotide sequence encoding a cleavable linker, a nucleotide sequence encoding a CCP6 domain of C4BP alpha chain and a nucleotide sequence encoding an oligomerization domain. In another embodiment, the expression cassette comprises from 5' to 3' a nucleotide sequence encoding a signal peptide, a nucleotide sequence encoding a tag, a nucleotide sequence encoding a CCP6 domain of C4BP alpha chain and a nucleotide sequence encoding an oligomerization domain. In a more preferred embodiment, all elements are in the same open reading frame. In a still more preferred embodiment, all elements are under the operation control of a regulatory nucleotide sequence. In a preferred embodiment, the first tag is different from the second tag.

In a preferred embodiment, the polynucleotide encoding the polypeptide of SEQ ID NO: 7 is the polynucleotide of SEQ ID NO: 9.

Methods for cloning the nucleotide sequences into a vector and for expressing and purifying the recombinant polypeptides of the invention are well known in the art. Exemplary conditions for expression and purification are shown in the Materials and Methods section of the examples of the present invention.

All the terms and embodiments previously described in the context of the recombinant polypeptide of the invention and the homooligomer of the invention are equally applicable to the nucleotides, vectors and host cells of the invention.

### Pharmaceutical compositions of the invention

An additional aspect of this invention is a pharmaceutical composition comprising a recombinant polypeptide of the invention, an homooligomer of the invention, a polynucleotide of the invention, a vector according to the invention, a host cell according to the invention, a tolerogenic dendritic cell of the invention, a tolerogenic macrophage of the invention or a cell population of the invention and a pharmaceutically acceptable carrier.

Therapeutic methods of this invention comprise the step of treating patients in a pharmaceutically acceptable manner with those compositions. These compositions may be used to treat any mammal, including humans.

The pharmaceutical compositions of this invention may be in a variety of forms. These include, for example, solid, semi-solid and liquid dosage forms, such as tablets, pills, powders, liquid solutions or suspensions, liposomes, suppositories, injectable and infusable solutions and sustained release forms. Generally, the pharmaceutical compositions of the present invention may be formulated and administered using methods and compositions similar to those used for pharmaceutically important polypeptide such as, for example, alpha interferon. Thus, the recombinant proteins of this invention may be stored in lyophilized form, reconstituted with sterile water just prior to administration, and administered by conventional routes of administration such as parenteral, subcutaneous, intravenous, intramuscular or intralesional routes. In a preferred embodiment, the compounds of the invention are administered subcutaneously.

A composition may be a pharmaceutical composition that is a sterile aqueous or non-aqueous solution, suspension or emulsion, which additionally comprises a physiologically acceptable or suitable carrier. A pharmaceutically acceptable or suitable carrier may include (or refer to) an excipient (i.e., a non-toxic material that does not interfere with the activity of the active ingredient) and/or a diluent. Such compositions may be in the form of a solid, liquid, or gas (aerosol). Alternatively, compositions described herein may be formulated as a lyophilizate, or compounds may be encapsulated within liposomes using technology known in the art. Pharmaceutical compositions may also contain other components, which may be biologically active or inactive. Such components include, but are not limited to, buffers (e.g., neutral buffered saline or phosphate buffered saline), carbohydrates (e.g., glucose, mannose, sucrose or dextrans), mannitol, proteins, polypeptides or amino acids such as glycine, antioxidants, chelating agents such as EDTA or glutathione, stabilizers, dyes, flavoring agents, and suspending agents and/or preservatives.

Any suitable excipient or carrier known to those of ordinary skill in the art for use in pharmaceutical compositions may be employed in the compositions described herein. Excipients for therapeutic use are well known, and are described, for example, in Remingtons Pharmaceutical Sciences, Mack Publishing Co. (A. R. Gennaro ed. 1985). In general, the type of excipient is selected based on the mode of administration. Pharmaceutical compositions may be formulated for any appropriate manner of administration, including, for example, topical, oral, nasal, intrathecal, rectal, vaginal, intraocular, subconjunctival, sublingual or parenteral administration, including subcutaneous, intravenous, intramuscular, intrasternal, intracavernous, intrameatal or intraurethral injection or infusion. For parenteral administration, the carrier preferably comprises water, saline, alcohol, a fat, a wax or a buffer. For oral administration, any of the above excipients or a solid excipient or carrier, such as mannitol, lactose, starch, magnesium stearate, sodium saccharine, talcum, cellulose, kaolin, glycerine, starch dextrins, sodium alginate, carboxymethylcellulose, ethyl cellulose, glucose, sucrose and/or magnesium carbonate, may be employed.

A pharmaceutical composition (e.g., for oral administration or delivery by injection) may be in the form of a liquid. A liquid pharmaceutical composition may include, for example, one or more of the following: a sterile diluent such as water for injection, saline solution, preferably physiological saline, Ringer's solution, isotonic sodium chloride, fixed oils that may serve as the solvent or suspending medium, polyethylene glycols, glycerin, propylene glycol or other solvents; antibacterial agents; antioxidants; chelating agents; buffers and agents for the adjustment of tonicity such as sodium chloride or dextrose. A parenteral preparation can be enclosed in ampoules, disposable syringes or multiple dose vials made of glass or plastic. The use of physiological saline is preferred, and an injectable pharmaceutical composition is preferably sterile.

The agents described herein, including polypeptides, homooligomers, polynucleotides, vectors, host cells, tolerogenic dendritic cells or tolerogenic macrophages, may be formulated for sustained or slow release. Such compositions may generally be prepared using well known technology and administered by, for example, oral, rectal or subcutaneous implantation, or by implantation at the desired target site. Sustained-release formulations may contain an agent dispersed in a carrier matrix and/or contained within a reservoir surrounded by a rate controlling membrane. Excipients for use within such formulations are biocompatible, and may also be biodegradable; preferably the formulation provides a relatively constant level of active component release. The amount of active compound contained within a sustained release formulation depends upon the site of implantation, the rate and expected duration of release, and the nature of the condition to be treated or prevented.

Pharmaceutical compositions may be administered in a manner appropriate to the disease to be treated (or prevented) as determined by persons skilled in the medical art. An appropriate dose and a suitable duration and frequency of administration will be determined by such factors as the condition of the patient, the type and severity of the patient's disease, the particular form of the active ingredient, and the method of administration. In general, an appropriate dose and treatment regimen provides the composition(s) in an amount sufficient to provide therapeutic and/or prophylactic benefit (e.g., an improved clinical outcome, such as more frequent complete or partial remissions, or longer disease-free and/or overall survival, or a lessening of symptom severity). For prophylactic use, a dose should be sufficient to prevent, delay the onset of, or diminish the severity of a disease associated with an immunological disease or disorder.

Optimal doses may generally be determined using experimental models and/or clinical trials. The optimal dose may depend upon the body mass, weight, or blood volume of the patient. In general, the amount of polypeptide present in a dose, or produced *in situ* by DNA present in a dose, ranges from about 0.01 µg to about 1000 µg per kg of host. The use of the minimum dosage that is sufficient to provide effective therapy is usually preferred. Patients may generally be monitored for therapeutic or prophylactic effectiveness using assays suitable for the condition being treated or prevented, which assays will be familiar to those having ordinary skill in the art. When administered in a liquid form, suitable dose sizes will vary with the size of the patient, but will typically range from about 1 ml to about 500 ml (comprising from about 0.01 µg to about 1000 µg per kg) for a 10-60 kg subject.

In a preferred embodiment, the pharmaceutical composition comprises from 0.45 mg to 18.90 mg of the homooligomer of the invention, more preferably from 0.45 mg to 9.45 mg, more preferably from 0.56 mg to 8.51 mg, more preferably from 0.75 mg to 8.13 mg, even more preferably from 0.79 mg to 8.05 mg. In a preferred embodiment, the dose is 0.79 mg. In another preferred embodiment, the dose is 0.8 mg. In another preferred embodiment, the dose is 8 mg. In another preferred embodiment, the dose is 8.05 mg.

In an embodiment, the pharmaceutical composition comprises from 7.56 mg to 18.90 mg of a compound of the invention, preferably from 9.45 mg to 15.13 mg, preferably from 11.35 mg to 15.13 mg, preferably from 13.24 mg to 15.13 mg.

In an embodiment, the pharmaceutical composition comprises from 1.89 mg to 17.02 mg of a compound of the invention, preferably from 3.78 mg to 15.13 mg, preferably from 5.67 mg to 13.24 mg, more preferably from 7.56 mg to 11.35 mg.

In an embodiment, the pharmaceutical composition comprises from 0.5 mg to 9 mg of a compound of the invention, more preferably from 0.75 mg to 8.5 mg. In another embodiment, the composition comprises from 7 mg to 18.90 mg of a compound of the invention, more preferably from 7.5 mg to 18 mg, more preferably from 9 mg to 15 mg, more preferably from 11 mg to 15 mg, even more preferably from 13 mg to 15 mg. In another embodiment, the pharmaceutical composition comprises from 1 mg to 17 mg of a compound of the invention, preferably from 3 mg to 15 mg, preferably from 5 mg to 13 mg, more preferably from 7 mg to 11 mg.

For pharmaceutical compositions comprising an agent that is a nucleic acid molecule including an aptamer, the nucleic acid molecule may be present within any of a variety of delivery systems known to those of ordinary skill in the art, including nucleic acid, and bacterial, viral and mammalian expression systems such as, for example, recombinant expression constructs as provided herein. Techniques for incorporating DNA into such expression systems are well known to those of ordinary skill in the art. The DNA may also be "naked," as described, for example, in Ulmer et al., Science 259:1745-49, 1993 and reviewed by Cohen, Science 259:1691-1692, 1993. The uptake of naked DNA may be increased by coating the DNA onto biodegradable beads, which are efficiently transported into the cells.

Nucleic acid molecules may be delivered into a cell according to any one of several methods described in the art (see, e.g., Akhtar et al., Trends Cell Bio. 2:139 (1992); Delivery Strategies for Antisense Oligonucleotide Therapeutics, ed. Akhtar, 1995, Maurer et al., Mol. Membr. Biol. 16:129-40 (1999); Hofland and Huang, Handb. Exp. Pharmacol. 137:165-92 (1999); Lee et al., ACS Symp. Ser. 752:184-92 (2000); U.S. Pat. No. 6,395,713; International Patent Application Publication No. WO 94/02595); Selbo et al., Int. J. Cancer 87:853-59 (2000); Selbo et al., Tumour Biol. 23:103-12 (2002); U.S. Patent Application Publication Nos. 2001/0007666, and 2003/077829). Such delivery methods known to persons having skill in the art, include, but are not restricted to, encapsulation in liposomes, by iontophoresis, or by incorporation into other vehicles, such as biodegradable polymers; hydrogels; cyclodextrins (see, e.g., Gonzalez et al., Bioconjug. Chem. 10: 1068-74 (1999); Wang et al., International Application Publication Nos. WO 03/47518 and WO 03/46185); poly(lactic-co-glycolic)acid (PLGA) and PLCA microspheres (also useful for delivery of peptides and polypeptides and other substances) (see, e.g., U.S. Pat. No. 6,447,796; U.S. Patent Application Publication No. 2002/130430); biodegradable nanocapsules; and bioadhesive microspheres, or by proteinaceous vectors (International Application Publication No. WO 00/53722). In another embodiment, the nucleic acid molecules for use in altering (suppressing or enhancing) an immune response in an immune cell and for treating an immunological disease or disorder can also be formulated or complexed with polyethyleneimine and derivatives thereof, such as polyethyleneimine-polyethyleneglycol-N-acetylgalactosamine (PEI-PEG-GAL) or polyethyleneimine-polyethyleneglycol-tri-N-acetylgalactosamine (PEI-PEG-triGAL) derivatives (see also, e.g., U.S. Patent Application Publication No. 2003/0077829).

The pharmaceutical compositions/medicaments of the invention may comprise further, e.g. active ingredients, e.g. other immunomodulatory antibodies such as anti-ICOS, anti-CD 154, anti-CD 134L or recombinant proteins such as, but not limited to rCTLA-4 (CD 152), rOX40 (CD134), or anti-inflammatory agents or immunomodulatory compounds such as, but not limited to cyclosporin A, FTY720, RAD, rapamycin, FK506, 15- deoxyspergualin, steroids; as described above.

In addition, one may use DNA sequences encoding recombinant polypeptides of the invention in somatic gene therapy. This involves, for example, inserting DNA sequences into retroviral-based vectors suitable for infection of human somatic cells (A. Kasid et al. 1990. "Human Gene Transfer: Characterization of human tumor-infiltrating lymphocytes as vehicles for retroviral-mediated gene transfer", Proc. Natl. Acad. Sci., USA, 87:473-477). For example, patients with an immunoinflammatory disease could be treated as follows. First, one would prepare a retrovirus characterized by a DNA sequence encoding a recombinant polypeptide of the invention. Then, one would isolate cells from the patient and infect them *in vitro,* with the retrovirus. One would then reintroduce these cells into the patient, where the vector will express and the cell will secrete the recombinant polypeptide of the invention which will assemble to form the homooligomer of the invention.

All the terms and embodiments described in the context of the remaining aspects of the invention are equally applicable to the pharmaceutical compositions of the invention.

### Therapeutic uses of the polypeptides, homooligomers, polynucleotides, vectors, host cells and pharmaceutical compositions of the invention

Thus, in another aspect, the invention relates to a recombinant polypeptide of the invention, an homooligomer of the invention, a polynucleotide of the invention, a vector of the invention, a host cell of the invention, or a pharmaceutical composition of the invention for use in medicine.

In another aspect, the invention relates to the use of a recombinant polypeptide of the invention, an homooligomer of the invention, a polynucleotide of the invention, a vector of the invention, a host cell of the invention or a pharmaceutical composition of the invention for the manufacture of a medicament.

In another aspect, the invention relates to a recombinant polypeptide of the invention, an homooligomer of the invention, a polynucleotide of the invention, a vector of the invention, a host cell of the invention, or a pharmaceutical composition of the invention for use in the prevention and/or treatment of an immunological disease caused by an undesired activation of the immune system.

In another aspect, the invention relates to the use of a recombinant polypeptide of the invention, an homooligomer of the invention, a polynucleotide of the invention, a vector of the invention, a host cell of the invention, or a pharmaceutical composition of the invention for the manufacture of a medicament for the prevention and/or treatment of an immunological disease caused by an undesired activation of the immune system.

In another aspect, the invention relates to a method for the prevention and/or treatment of an immunological disease caused by an undesired activation of the immune system, in a subject in need thereof comprising the administration to said subject of a recombinant polypeptide of the invention, an homooligomer of the invention, a polynucleotide of the invention, a vector of the invention, a host cell of the invention or a pharmaceutical composition of the invention.

In another aspect, the invention relates to a method for increasing tolerogenic dendritic cell populations and/or tolerogenic macrophage populations in a subject in need thereof which comprises the administration to said subject of a polypeptide according to the invention, an homooligomer according to the invention, a polynucleotide according to the invention, a vector according to the invention, a host cell according to the invention or a pharmaceutical composition according to the invention.

The term "prevention", as used herein, refers to the administration of a compound of the invention in an initial or early stage of the disease, or to also prevent its onset.

The term "treatment" is used to designate the administration of a compound of the invention to control the progression of the disease before or after the clinical signs have appeared. Control of the progression of the disease is understood as the beneficial or desired clinical results which include but are not limited to reduction of the symptoms, reduction of the duration of the disease, stabilization of pathological conditions (specifically avoiding additional impairment), delaying the progression of the disease, improving the pathological condition and remission (both partial and complete). The control of the progression of the disease also involves a prolongation of survival in comparison to the expected survival if the treatment was not applied.

The expression "immunological disease caused by an undesired activation of the immune system", as used herein, refers to any disease which is caused by an undesired activation of the immune system, including the innate or adaptative immune system as well as the humoral or cell branch of the immune system. Preferably, the immunological disease of the invention is a disease in which the immune system is activated in response to an alloantigen or an autoantigen. Therefore, immunological diseases in which the immune system is depressed are not encompassed by the present invention.

In a preferred embodiment, the immunological disease is selected from the group consisting of an immunoinflammatory disease, sepsis, an autoimmune disease, transplant rejection, graft-versus-host disease and a hypersensitivity disease.

The term "immunoinflammatory disease", as used herein, refers to inflammatory diseases and disorders in which immune cells and/or cytokines are involved in the pathophysiology of the disease or disorder. Examples of immunoinflammatory diseases include conditions such as rheumatoid arthritis, juvenile rheumatoid arthritis, osteoarthritis, acute respiratory distress syndrome and asthma. The term immunoinflammatory disease includes both acute and chronic inflammatory disorders. The term "acute inflammatory disorder" is intended to include disorders and episodes of disorders, characterized by rapid onset of symptoms associated with an inflammatory response and relatively short duration of symptoms, whereas a "chronic inflammatory disorder" is intended to include disorders characterized by the continued presence of symptoms associated with an inflammatory response and ongoing duration of symptoms. Immunoinflammatory diseases which can be treated with the methods according to the present invention include, without limitation, cardiovascular diseases such as infarct or stroke, atherosclerosis, pulmonary fibrosis, rheumatoid arthritis, juvenile rheumatoid arthritis, osteoarthritis, acute respiratory distress syndrome, asthma, and cancer. Also comprised within the immunoinflammatory diseases that can be treated according to the present invention are diseases which appear during pregnancy such as pre-eclampsia and eclampsia. Pre-eclampsia is a pregnancy-related disease characterised by hypertension, proteinuria and oedema. Pre-eclampsia is understood and shall be defined herein to encompass and reside within a spectrum of pre-eclampsia disorders, including placental insufficiency, intrauterine growth retardation, early miscarriage, preterm birth, intrauterine death and eclampsia.

The term "sepsis", as used herein, refers to a systemic host response to microorganisms in previously sterile tissues characterized by end-organ dysfunction away from the primary site of infection. To qualify as sepsis, there must be an infection suspected or proven (by culture, stain, or polymerase chain reaction (PCR)), or a clinical syndrome pathognomonic for infection. Specific evidence for infection includes WBCs in normally sterile fluid (such as urine or cerebrospinal fluid (CSF), evidence of a perforated viscus (free air on abdominal x-ray or CT scan, signs of acute peritonitis), abnormal chest x-ray (CXR) consistent with pneumonia (with focal opacification), or petechiae, purpura, or purpura fulminans. The more critical subsets of sepsis are severe sepsis (sepsis with acute organ dysfunction) and septic shock (sepsis with refractory arterial hypotension). As an alternative, when two or more of the systemic inflammatory response syndrome criteria are met without evidence of infection, patients may be diagnosed simply with "SIRS." Patients with SIRS and acute organ dysfunction may be termed "severe SIRS." Patients are defined as having "severe sepsis" if they have sepsis plus signs of systemic hypoperfusion: either end-organ dysfunction or serum lactate greater than 4 mmol/dL. Other signs include oliguria and altered mental status. Patients are defined as having septic shock if they have sepsis plus hypotension after aggressive fluid resuscitation (typically upwards of 6 liters or 40 ml/kg of crystalloid). Examples of end-organ dysfunction include acute lung injury or acute respiratory distress syndrome, encephalopathy, or dysfunction affecting liver (disruption of protein synthetic function and metabolic functions), kidney (oliguria and anuria, electrolyte abnormalities, volume overload), and heart (systolic and diastolic heart failure).

Suitable sepsis conditions that can be treated with the compounds according to the present invention include, without limitation, severe sepsis and septic shock. In one embodiment, the condition associated with sepsis syndrome is selected from the group consisting of an organ dysfunction, preferably a kidney dysfunction or a liver dysfunction, a multiple organ dysfunction syndrome (MODS), an acute respiratory distress syndrome (ARDS), and disseminated intravascular coagulation (DIC).

Sepsis may be induced by a bacterium or more than one bacterium selected from the group consisting of Gram-negative bacteria and Gram-positive bacteria. Preferably, the Gram-negative bacterium is selected from the group consisting of *Escherichia coli, Klebsiella* species, *Serratia* species, *Enterobacter* species, *Proteus* species, *Pseudomonas aeruginosa, Haemophilus influenzae, Neisseria* species, and *Listeria* species. Alternatively, the Gram-positive bacterium is selected from the group consisting of *Staphylococcus aureus, Streptococcus pneumoniae,* coagulase-negative Staphylococci, *Enterococcus* species, *Streptococcus pyogenes,* and *Streptococcus viridans.* In one embodiment, the sepsis syndrome is induced by LPS. In yet another embodiment, the sepsis is induced by a microorganism or more than one microorganism selected from the group consisting of anaerobic bacteria, fungi, rickettsiae, chlamydiae, mycoplasma, spirochetes, and viruses.

In a preferred embodiment, the immunological disease is an autoimmune disease.

The term "autoimmune disease", "disease associated with immune dysfunction/dysregulation" or "immune inflammatory disease" is used throughout the specification to refer to a pathogenic condition in which the patients' immune system results in disease from a self antigen (autoimmunity) or a foreign antigen (immune dysfunction/dysregulation or immune inflammatory disease). Autoimmunity is present in everyone to some extent. It is usually harmless and probably a universal phenomenon of vertebrate life. However, autoimmunity can be the cause of a broad spectrum of human illnesses, known as autoimmune diseases. This concept of autoimmunity as the cause of human illness is relatively new, and it was not accepted into the mainstream of medical thinking until the 1950s and 1960s. Autoimmune diseases are, thus, defined when the progression from benign autoimmunity to pathogenic autoimmunity occurs. This progression is determined by both genetic influences and environmental triggers. The concept of autoimmunity as the actual cause of human illness (rather than a consequence or harmless accompaniment) can be used to establish criteria that define a disease as an autoimmune disease. Autoimmune diseases or diseases which are characterized as involving immune dysfunction or dysregulation (immune inflammatory disease), which may be treated by the present invention include systemic lupus erythematosus (SLE), lupus nephritis, central nervous system (CNS) lupus, diabetes mellitus (type I), asthma, ulcerative colitis, Crohn's disease, Grave's disease, arthritis, including rheumatoid arthritis and osteoarthritis, pernicious anemia, inflammatory bowel disease and multiple sclerosis, among numerous others. Numerous autoimmune diseases may be treated using the method of the present invention including autoimmune blood diseases, including pernicious anemia, autoimmune hemolytic anemia, aplastic anemia, idiopathic thrombocytopenic purpura, ankylosing spondilitis; autoimmune diseases of the musculature including polymyositis and dermatomyositis; autoimmune diseases of the ear including autoimmune hearing loss and Meniere's syndrome; autoimmune eye diseases, including Mooren's disease, Reiter's syndrome and Vogt-Koyanagi-Harada disease; autoimmune diseases of the kidney including glomerulonephritis, IgA nephropathy, and lupus nephritis; diabetes mellitus (type I); autoimmune skin diseases including pemphigus (autoimmune bullous diseases), such as pemphigus vulgaris, pemphigus foliaceus, pemphigus erythematosus, bullous pemphigoid, vitiligo, epidermolysis bullosa acquisita, psoriasis and alopecia areata; cardiovascular autoimmune diseases, including autoimmune myocarditis, vasculitis including Churg-Strauss syndrome, giant cells arteritis, Kawasaki's disease, polyarteritis nodosa, Takayasu's arteritis and Wegener's granulomatosis; endocrine autoimmune diseases, including Addison's disease, autoimmune hypoparathyroidism, autoimmune hypophysitis, autoimmune oophoritis, autoimmune orchitis, Grave's Disease, Hashimoto's thyroiditis, polyglandular autoimmune syndrome type 1 (PAS-1), polyglandular autoimmune syndrome type 2 (PAS-2), and polyglandular autoimmune syndrome type 3 (PAS-3); autoimmune gastroenteric diseases including autoimmune hepatitis, primary biliary cirrhosis, inflammatory bowel disease, celiac disease, Crohn's disease; autoimmune nervous diseases, including multiple sclerosis, myasthenia gravis, Guillan-Barre syndrome and chronic inflammatory demyelinating neuropathy; and systemic autoimmune diseases including systemic lupus erythematosus, antiphospholid syndrome, autoimmune lymphoproliferative disease, autoimmune polyendocrinopathy, Bechet's disease, Goodpasture's disease, arthritis, including rheumatoid arthritis, osteoarthritis and septic arthritis, sarcoidosis, scleroderma and Sjogren's syndrome and psoriasis among others.

In an embodiment, the autoimmune disease is lupus erythematosus. The expression "lupus erythematosus", as used herein, refers to a name given to a collection of autoimmune diseases that have common symptoms that affect joints, skin, kidneys, blood cells, heart and lungs. Lupus erythematosus may manifest as systemic disease or in a purely cutaneous form also known as incomplete lupus erythematosus. Lupus has four main types: systemic, discoid, drug-induced and neonatal. The term "lupus erythematosus" in the context of the present invention encompasses, without limitation, acute cutaneous lupus erythematosus, subacute cutaneous lupus erythematosus, discoid lupus erythematosus (chronic cutaneous), childhood discoid lupus erythematosus, generalized discoid lupus erythematosus, localized discoid lupus erythematosus, chilblain lupus erythematosus (Hutchinson), lupus erythematosus-lichen planus overlap syndrome, lupus erythematosus panniculitis (lupus erythematosus profundus), tumid lupus erythematosus, verrucous lupus erythematosus (hypertrophic lupus erythematosus), cutaneous lupus mucinosis, complement deficiency syndromes, drug-induced lupus erythematosus, neonatal lupus erythematosus and systemic lupus erythematosus. The most common severe form is systemic lupus erythematosus.

In a preferred embodiment, the autoimmune disease is selected from the group consisting of systemic lupus erythematosus, lupus nephritis, rheumatoid arthritis, inflammatory bowel disease and ulcerative colitis; more preferably is selected from the group consisting of systemic lupus erythematosus, lupus nephritis, inflammatory bowel disease and rheumatoid arthritis; more preferably from systemic lupus erythematosus, lupus nephritis and rheumatoid arthritis. In a preferred embodiment, the autoimmune disease is selected from the group consisting of systemic lupus erythematosus and lupus nephritis.

In a preferred embodiment the autoimmune disease is systemic lupus erythematosus.

The expression "systemic lupus erythematosus" or "SLE", as used herein, refers to a systemic autoimmune disease in which the body's immune system mistakenly attacks healthy tissue in many parts of the body. Symptoms vary from person to person and may be mild to severe. Common symptoms include painful and swollen joints, fever, chest pain, hair loss, mouth ulcers, swollen lymph nodes, feeling tired, and a red rash which is most commonly on the face. Often there are periods of illness, called flares, and periods of remission when there are few symptoms. Almost everyone with SLE has joint pain and swelling. Some develop arthritis. SLE often affects the joints of the fingers, hands, wrists, and knees. Renal disease in SLE carries significant morbidity and mortality. Acute or chronic renal impairment may develop with lupus nephritis, leading to acute or end-stage kidney failure.

In a preferred embodiment the autoimmune disease is lupus nephritis.

The expression "lupus nephritis" or "LN", also known as SLE nephritis, is an inflammation of the kidneys caused by systemic lupus erythematosus. It is a type of glomerulonephritis in which the glomeruli become inflamed. As the result of SLE, the cause of glomerulonephritis is said to be secondary and has a different pattern and outcome from conditions with a primary cause originating in the kidney. General symptoms of lupus nephritis include fever, oedema, high blood pressure, joint pain, muscle pain, malar rash and foamy urine.

In another preferred embodiment, the autoimmune disease is inflammatory bowel disease.

The expression "inflammatory bowel disease", as used herein, refers to a group of inflammatory conditions of the colon and small intestine. Crohn's disease and ulcerative colitis are the principal types of inflammatory bowel disease. Crohn's disease affects the small intestine and large intestine, as well as the mouth, esophagus, stomach and the anus; whereas ulcerative colitis primarily affects the colon and the rectum. The symptoms can be: abdominal pain, diarrhea, rectal bleeding, severe internal cramps/muscle spasms in the region of the pelvis and weight loss.

In another preferred embodiment, the autoimmune disease is rheumatoid arthritis.

The expression "rheumatoid arthritis" or "RA", as used herein, refers to a long-term systemic autoimmune disorder characterized by chronic inflammation of the joints and the subsequent destruction of cartilage and bone. It typically results in warm, swollen, and painful joints. Pain and stiffness often worsen following rest. Most commonly, the wrist and hands are involved, with the same joints typically involved on both sides of the body. The disease may also affect other parts of the body. This may result in a low red blood cell count, inflammation around the lungs, and inflammation around the heart. Fever and low energy may also be present. Often, symptoms come on gradually over weeks to months. RA primarily starts as a state of persistent cellular activation leading to autoimmunity and immune complexes in both joints and other organs where it manifests. The initial site of disease is the synovial membrane, where swelling and congestion leads to infiltration by immune cells. The various phases of progression of RA are:
- Initiation phase, due to non-specific inflammation.
- Amplification phase, due to T cell activation
- Chronic inflammatory phase with tissue injury, due to cytokines IL-1, TNF-alpha and IL-6.

The expression "transplant rejection", as used herein, refers to an immune condition in which a transplanted cell, tissue, or organ is not accepted by the body of the transplant recipient. The expression transplant rejection encompasses both acute and chronic transplant rejection.

"Acute rejection or AR" is the rejection by the immune system of a tissue transplant recipient when the transplanted tissue is immunologically foreign. Acute rejection is characterized by infiltration of the transplanted tissue by immune cells of the recipient, which carry out their effector function and destroy the transplanted tissue. The onset of acute rejection is rapid and generally occurs in humans within a few weeks after transplant surgery.

"Chronic transplant rejection or CR" generally occurs in humans within several months to years after engraftment, even in the presence of successful immunosuppression of acute rejection. Fibrosis is a common factor in chronic rejection of all types of organ transplants. Chronic rejection can typically be described by a range of specific disorders that are characteristic of the particular organ. For example, in lung transplants, such disorders include fibroproliferative destruction of the airway (bronchiolitis obliterans); in heart transplants or transplants of cardiac tissue, such as valve replacements, such disorders include fibrotic atherosclerosis; in kidney transplants, such disorders include, obstructive nephropathy, nephrosclerosis, tubulointerstitial nephropathy; and in liver transplants, such disorders include disappearing bile duct syndrome. Chronic rejection can also be characterized by ischemic insult, denervation of the transplanted tissue, hyperlipidemia and hypertension associated with immunosuppressive drugs.

As in known in the transplantation field, the transplant organ, tissue or cell(s) may be allogeneic or xenogeneic, such that the grafts may be allografts or xenografts. A feature of the graft tolerant phenotype detected or identified by the subject methods is that it is a phenotype which occurs without immunosuppressive therapy, i.e., it is present in a host that is not undergoing immunosuppressive therapy such that immunosuppressive agents are not being administered to the host. The transplant graft maybe any solid organ and skin transplant. Examples of organ transplants that can be treated by the methods described herein include but are not limited to kidney transplant, pancreas transplant, liver transplant, heart transplant, lung transplant, intestine transplant, pancreas after kidney transplant, and simultaneous pancreas-kidney transplant.

The methods according to the present invention are also suitable for the prevention and/or treatment of delayed Graft Function (DGF) due to ischemia-reperfusion injury. The term "delayed graft function", as used herein, refers to a form of acute renal failure resulting in post-transplantation oliguria, increased allograft immunogenicity and risk of acute rejection episodes, and decreased long-term survival. DGF may be caused by different factors related to the donor and prerenal, renal, or postrenal transplant factors related to the recipient. However, a major cause of delayed graft function is ischaemia and reinstitution of blood flow in ischaemically damaged kidneys after hypothermic preservation.

The term "graft-versus-host disease" or GVHD, as used herein, refers to a condition that occurs when T cells present in donor tissue attack the host, or recipient, of the grafted cells or tissue. Any type of GVHD can be treated by the therapeutic agents of the present invention, including acute GVHD and chronic GVHD.

The term "hypersensitivity disease" refers to a condition in which the subject has an abnormal sensitivity to an innocuous agent, known as allergen. Hypersensivity disease can be categorized into four types, Type I, Type II, Type III and Type IV. Type I is described as atopic or anaphylactic which results from a release of mediators from IgE-sensitized basophils and mast cells. Type II is described as cytotoxic which involves complement-fixing antibody with cell lysis or antibody-dependent cellular cytotoxocity. Type III is described as immune-complex-mediated which is associated with soluble antigen-antibody complexes. Type IV is described as cell-mediated or delayed hypersensitivity which results from a release of lymphokines by sensitized T lymphocytes after contact with an antigen.

In a preferred embodiment, the immunological disease is inflammatory bowel disease, more preferably ulcerative colitis. In another embodiment, the immunological disease is Crohn's disease.

The prevention and/or treatment of an immunological disease is achieved through the increase of tolerogenic dendritic cell and/or tolerogenic macrophage populations.

The expression "increasing tolerogenic dendritic cell population and/or tolerogenic macrophage population" is understood to mean that the administration of a polypeptide according to the invention, an homooligomer according to the invention, a polynucleotide encoding a polypeptide or an homooligomer of the invention, a vector comprising the polynucleotide, a host cell comprising the vector or a pharmaceutical composition comprising thereof produces an increase in the number of tolerogenic dendritic cells and/or of tolerogenic macrophages with respect to an untreated subject. The tolerogenic dendritic cell population and/or tolerogenic macrophage population is increased by 10%, 20%, 30%, 40%, 50%, 60%, 70%, 80%, 90%, 95%, 97%, 98%, 99%, or 100% in comparison with an untreated subject. The capacity of the compounds of the invention of increasing a tolerogenic dendritic cell population and/or a tolerogenic macrophage population can be determined, for instance, as described in examples 1 to 11.

In a preferred embodiment, the compound of the invention is administered subcutaneously. In another preferred embodiment, the compound of the invention is administered in a regimen comprising a plurality of administrations and wherein the compound is administered no more than once a week. In a more preferred embodiment, the compound of the invention is administered subcutaneously in a regimen comprising a plurality of administrations and wherein the compound is administered no more than once a week.

A plurality of administrations means at least two administrations. Therefore, in a preferred embodiment, the regimen comprises at least 2 administrations, at least 3, at least 4, at least 5, at least 6, at least 7, at least 8, at least 9, at least 10, at least 11, at least 12, at least 13, at least 14, at least 15, at least 30, at least 50, at least 100 or more. Preferably, the compound is administered chronically. Preferably, the compound is administered at least during 1 year, at least during 2 years, at least during 5 years or more.

The expression "the compound is administered no more than once a week" means that during a week the maximum number of administrations is one. This means that if the compound is administered on day 1, the subsequent administration cannot be on days 2, 3, 4, 5, 6 or 7. However, the expression "the compound is administered no more than once a week" encompasses the possibility that no administration is given during a week, for example, because the compound is administered once every two weeks. Therefore, according to the invention one administration is separated at least 7 days from another administration. Therefore, in a preferred embodiment, each administration is separated at least by 7 days from another, at least by 8 days, at least by 9 days, at least by 10 days, at least by 11 days, at least by 12 days, at least by 13 days, at least by 14 days, at least by 15 days, at least by 16 days, at least by 17 days, at least by 18 days, at least by 19 days, at least by 20 days, at least by 21 days, at least by 22 days, at least by 23 days, at least by 24 days, at least by 25 days, at least by 26 days, at least by 27 days, at least by 28 days, at least by 29 days, at least by 30 days, at least by 31 days, at least by 32 days, at least by 33 days, at least by 34 days, at least by 35 days, at least by 36 days, at least by 37 days, at least by 38 days, at least by 39 days, at least by 40 days, at least by 41 days, at least by 42 days, at least by 43 days, at least by 44 days, at least by 45 days, at least by 46 days, at least by 47 days, at least by 48 days, at least by 49 days, at least by 50 days, at least by 51 days, at least by 52 days, at least by 53 days, at least by 54 days, at least by 55 days, at least by 56 days, at least by 57 days, at least by 58 days, at least by 59 days, at least by 60 days or more.

In a preferred embodiment, the compound is administered once a week. In another preferred embodiment, the compound is administered once every two weeks. In another embodiment, the compound is administered once every three weeks. In another embodiment, the compound is administered once every four weeks. In another embodiment, the compound is administered once every five weeks. In another embodiment, the compound is administered once every six weeks. In another embodiment, the compound is administered once every seven weeks. In another embodiment, the compound is administered once every eight weeks. In another embodiment, the compound is administered once every nine weeks. In another embodiment, the compound is administered once every ten weeks. In another embodiment, the compound is administered once every eleven weeks. In another embodiment, the compound is administered once every twelve weeks. In an embodiment, the compound is administered monthly. In another embodiment, the compound is administered once every two months.

The compound of the invention has efficacy at low doses. Therefore, in a preferred embodiment, the dose of each administration ranges from 0.24 mg/m² to 9.99 mg/m². In a more preferred embodiment, the dose of each administration ranges from 0.24 mg/m² to 5 mg/m², preferably from 0.3 mg/m² to 4.5 mg/m², preferably from 0.4 mg/m² to 4.3 mg/m², even more preferably from 0.42 mg/m² to 4.26 mg/m². In a preferred embodiment, the dose is 0.42 mg/m². In another preferred embodiment, the dose is 4.26 mg/m².

In an embodiment the dose of each administration ranges from 4 mg/m² to 9.99 mg/m², preferably from 5 mg/m² to 8 mg/m², preferably from 6 mg/m² to 8 mg/m², preferably from 7 mg/m² to 8 mg/m².

In an embodiment the dose of each administration ranges from 1 mg/m² to 9 mg/m², preferably from 2 mg/m² to 8 mg/m², preferably from 3 mg/m² to 7 mg/m², more preferably from 4 mg/m² to 6 mg/m².

In another embodiment, the compound is administered at a dose of from 0.24 µg/m² to 0.24 mg/m², preferably from 1 µg/m² to 0.24 mg/m², preferably from 10 µg/m² to 0.24 mg/m², preferably from 50 µg/m² to 0.24 mg/m², more preferably from 100 µg/m² to 0.24 mg/m², more preferably from 150 µg/m² to 0.24 mg/m², more preferably from 200 µg/m² to 0.24 mg/m².

However, the present invention covers also the administration of the compound at higher doses.

A subject in need of such treatment may be a human or may be a non-human primate or other animal (i.e., veterinary use) who has developed symptoms of an immunological disease or who is at risk for developing an immunological disease. Examples of non-human primates and other animals include but are not limited to farm animals, pets, and zoo animals (e.g., horses, cows, buffalo, llamas, goats, rabbits, cats, dogs, chimpanzees, orangutans, gorillas, monkeys, elephants, bears, large cats, etc.). In a preferred embodiment, the compound is administered to a mammal, preferably a human.

The treatment of the invention may also comprise a previous step of administration that does not need to be separated by seven days from the subsequent administration. In a particular case this could be considered an induction step. Therefore, in an embodiment the administration further comprises a previous step of subcutaneous administration of the compound separated by less than seven days from the subsequent administration. Preferably, the previous step is separated by less than 6 days from the subsequent administration, more preferably by less than 5 days, more preferably by less than 4 days, more preferably by less than 3 days, more preferably by less than 2 days, preferably by less than 1 day.

In a more preferred embodiment the previous step of subcutaneous administration is separated by two days from the subsequent step. Preferably, the previous step of a treatment for rheumatoid arthritis is separated by less than three days from the subsequent step, preferably by two days, preferably by less than two days, preferably by one day, preferably by less than one day.

In a preferred embodiment, the dose administered at the previous step and the dose administered in each subsequent administration is the same.

In another preferred embodiment, the dose administered at the previous step is higher than the dose administered in each subsequent administration. In a preferred embodiment, said dose is comprised between 40-45 mg/m², preferably is of 42.84 mg/m². Preferably, said dose is administered in the treatment of rheumatoid arthritis. In another preferred embodiment, said dose is administered in the treatment of systemic lupus erythematosus or lupus nephritis.

In another embodiment, the compound is administered in combination with one or more therapeutic agents useful in the treatment of an immunological disease caused by an undesired activation of the immune system, preferably said therapeutic agent is selected from the group consisting of cyclosporine A, tacrolimus, methotrexate, thiopurines, anti-TNF agents, infliximab, adalimumab, certolizumab, golimumab, etanercept, rituximab, epratuzumab, belimumab, rapamycin, anti-interferon antibodies, tocilizumab, laquinimod, tabalumab, ofatumumab, ixekizumab, brodalumab, briakinumab, sarilumab, rilonacept, anifrolumab, cyclophosphamide, mycophenolate mofetil, azathioprine, anticalcineurinics, prednisolone, methylprednisolone, vitamin D, vasoactive intestinal peptide, hydroxychloroquine, chloroquine, ocrelizumab, atacicept, abatacept, alemtuzumab, sirukumab, eculizumab and T cell vaccine.

All the embodiments disclosed in the context of the previous aspects of the invention are also applicable to this aspect.

### Methods for the generation of a population of tolerogenic dendritic cells obtained using the polypeptides, homooligomers, polynucleotides or vectors of the invention

The authors of the present invention have observed that dendritic cells contacted with either a polypeptide according to the invention, an homooligomer according to the invention, a polynucleotide according to the invention or a vector according to the invention show a tolerogenic phenotype.

Thus, in another aspect, the invention relates to an *in vitro* method for the generation of a population of tolerogenic dendritic cells comprising the steps of:
(i) incubating a population of dendritic precursor cells under conditions adequate for the formation of a population of immature dendritic cells, and
(ii) incubating the population of immature dendritic cells obtained in step
(i) under conditions adequate for the formation of mature dendrite cells
wherein steps (i) and/or (ii) are carried out in the presence of a composition of matter selected from the group consisting of:
(a) a polypeptide of the invention,
(b) an homooligomer of the invention,
(c) a polynucleotide of the invention, and
(d) a vector of the invention.

Therefore, the polypeptide of the invention, the homooligomer of the invention, the polynucleotide of the invention or the vector of the invention can be contacted with the cells during the differentiation stage (i.e., during the time wherein the dendritic precursor cells differentiate into immature dendritic cells), during the maturation step (i.e., during the time wherein the immature dendritic cells mature into dendritic cells) or during both stages.

The term "dendritic cell", as used herein, refers to any member of a diverse population of morphologically similar cell types found in lymphoid or non-lymphoid tissues. Dendritic cells are a class of "professional" antigen presenting cells, and have a high capacity for sensitizing MHC-restricted T cells. Dendritic cells may be recognized by function, or by phenotype, particularly by cell surface phenotype. These cells are characterized by their distinctive morphology, intermediate to high levels of surface MHC-class II expression and ability to present antigen to T cells, particularly to naive T cells (Steinman et al. (1991) Ann. Rev. Immunol. 9:271; incorporated herein by reference for its description of such cells). The dendritic cells affected by the methods of the invention may be selected to be immature or mature dendritic cells.

Dendritic cells include groups of bone marrow-derived cells with dendritic morphology distributed in various tissues and organs in the body, groups of cells with dendritic morphology distributed in various organs and tissues in the body that result from *in vitro* differentiation using cytokines or such from bone marrow- or blood-derived stem cells and equivalent cells. Specifically, the dendritic cells include, for example, lymphocytic dendritic cells (including cells which induce Th2 or immune tolerance), bone marrow dendritic cells (generally used dendritic cells, including immature and mature dendritic cells), Langerhans cells (dendritic cells important as antigen-presenting cells in the skin), interdigitating cells (distributed in the lymph nodes and spleen T cell region, and believed to function in antigen presentation to T cells), and follicular dendritic cells (important as antigen-presenting cells for B cells). The cell surface of dendritic cells is unusual, with characteristic veil-like projections, and is characterized by expression of the cell surface markers CD1a⁺, CD4⁺, CD86⁺, or HLA-DR⁺. Mature dendritic cells are typically CD11c+, while precursors of dendritic cells include those having the phenotype CD11c, IL-3Rα^{low}; and those that are CD11c- IL-3Rα^{high}. Treatment with GM-CSF *in vivo* preferentially expands CD11b^{high}, CD11c^{high} DC, while Flt-3 ligand has been shown to expand CD11c⁺ IL-3Rα^{low} DC, and CD11c⁻ IL-3Rα^{high} DC precursors.

"Tolerogenic dendritic cell" means a dendritic cell that is derived from an immature dendritic cell exposed to a differentiation stimulus, which can be a combination of cytokines, hormones, vitamins and other biological agents whereby the dendritic cell acquires the ability of inducing tolerance. A tolerogenic dendritic cell has low ability to activate effector T cells but high ability to induce and activate regulatory T cells. A tolerogenic dendritic cell can be seen as a maturation-resistant cell that acts as "an immature DC" with a stable phenotype that is preserved, even in the presence of pro-inflammatory signals. Tolerogenic dendritic cells secrete anti-inflammatory cytokines.

In a first step, the method for obtaining a population of tolerogenic dendritic cells comprises incubating a population of dendritic precursors cells under conditions adequate for the formation of a population of immature dendritic cells.

The term "dendritic cell precursor", as used herein, refers to any cell capable of differentiating into an immature dendritic cell in the presence of an appropriate cytokine (specifically, G-CSF, GM-CSF, TNF-a, IL-4, IL-13, SCF (c-kit ligand), Flt-3 ligand, or a combination thereof), and preferably is a cell that can differentiate into an immature dendritic cell in four weeks or less, more preferably in 20 days or less, even more preferably in 18 days or less, and still more preferably in 16 days or less. Examples of dendritic precursor cells include, but are not limited to, myeloid dendritic precursor cells, lymphoid dendritic precursor cells and plasmacytoid dendritic precursor cells. Phenotypic surface markers expressed by various subsets of dendritic precursor cells are well known in the art and may be used for the purpose of identification, for example, by flow cytometry or using immunohistochemical techniques.

In a preferred embodiment, the population of dendritic precursor cells is a population of monocytic dendritic precursor cells. "Monocytic dendritic cell precursors" as used herein, comprise monocytes that have the GM-CSF receptor on their surface and other myeloid precursor cells that are responsive to GM-CSF. The cells can be obtained from any tissue where they reside, particularly lymphoid tissues such as the spleen, bone marrow, lymph nodes and thymus. Monocytic dendritic cell precursors also can be isolated from the circulatory system. Peripheral blood is a readily accessible source of monocytic dendritic cell precursors. Umbilical cord blood is another source of monocytic dendritic cell precursors.

Monocytic dendritic cell precursors may be obtained from peripheral blood mononuclear cells (PBMCs), which can be obtained either from whole blood diluted 1:1 with buffered saline or from leukocyte concentrates ("buffy coat" fractions, MSKCC Blood Bank) by standard centrifugation over Ficoll-Paque PLUS (endotoxin-free, #17-1440-03, Amersham Pharmacia Biotech AB, Uppsala, Sweden). MoDC precursors are tissue culture plastic-adherent (#35-3003; Falcon, Becton-Dickinson Labware, Franklin Lakes, NJ) PBMCs, and can be cultured in complete RPMI 1640 plus 1% normal human serum (NHS) in the presence of GM-CSF (1000 IU/ml) and IL-4 (500 IU/ml) with replenishment every 2 days as described (Thurner B et al., 1999, J. Immunol. Meth. 223:1-15 and Ratzinger G. et al., 2004, J. Immunol. 173:2780-2791).

In general, monocytic dendritic cell precursors may be identified by the expression of markers such as CD13 and CD33. Myeloid dendritic precursors may differentiate into dendritic cells via CD14 or CD1a pathways. Accordingly, a dendritic precursor cell of the invention may be a CD14⁺CD1a⁻ dendritic precursor cell or a CD14⁻CD1a⁺ dendritic precursor cell. In certain embodiments of the invention, a myeloid dendritic precursor cell may be characterised by a CD34⁺CD33⁺CD7-CD10⁻ phenotype. In a preferred embodiment, the myeloid dendritic precursor cell is a CD14⁺ monocyte. The CD14⁺ monocyte may also express the GM-CSF receptor.

The dendritic precursor cells that are being used as starting material for the method of the invention can be autologous to the subject that is to be treated. In other embodiments, the dendritic cells that are being used as starting material for the methods of the invention are heterologous dendritic cells. For example, if graft-versus-host disease is to be treated, the dendritic cells that are being used as starting material are dendritic cells that were obtained from the donor. The subject can be, e.g., a mouse, a rat, a dog, a chicken, a horse, a goat, a donkey, or a primate. Most preferably, the subject is a human.

The expression "conditions adequate for the formation of a population of immature dendritic cells", as used herein, refers to conditions which result in the differentiation of the dendritic precursor cells into immature precursor cells. Suitable conditions include, for example, by culturing for about three days in the presence of SCF (50 ng/ml), GM-CSF (500 U/ml), and TNF-a (50 ng/ml) followed by culture in the presence of SCF (50 ng/ml), GM-CSF (500 U/ml), IL-4 (250 U/ml), and TNF-α (50 ng/ml), more preferably, in the presence of GM-CSF (20 ng/ml) and IL-4 (20 ng/ml), or in the presence of GM-CSF (20 ng/ml) and SCF (10 ng/ml). In a preferred embodiment, the cells are cultured in the presence of GM-CSF (800 UI/ml) and IL-4 (500 UI/ml).

This treatment leads to the generation of immature dendritic cells. "Immature dendritic cells" refers to dendritic cells having significantly low T cell-activating ability as compared with in a mature state. Specifically, the immature dendritic cells may have an antigen-presenting ability that is lower than 1/2, preferably lower than 1/4 of that of dendritic cells which maturation had been induced by adding LPS (1 µg/ml) and culturing for two days. The antigen-presenting ability can be quantified, for example, using the allo T cell-activating ability (mixed lymphocyte test: allo T cells and dendritic cells are co-cultured at a dendritic cell: T cell ratio of 1:10, 1:40, 1:80, 1:160; preferably at a dendritic cell: T cell ratio of 1:40, 1:80 or 1:160; 3H-thymidine is added 8 hours before terminating cultivation, and the T cell growth capacity is assessed based on the amount of 3H-thymidine incorporated into the DNA of the T cells (see Gene Therapy 7; 249-254 (2000)). Alternatively, it can be assessed by testing the ability to induce specific cytotoxic T cells (CTLs) using a peptide, in which a known class I-restricted peptide of a certain antigen is added to dendritic cells; the dendritic cells are co-cultured with T cells obtained from peripheral blood of the same healthy donor from whom the dendritic cells had been collected (with 25 U/ml or preferably 100 U/ml of IL-2 on day 3 or later). The T cells are preferably stimulated with dendritic cells three times during 21 days, more preferably stimulated with dendritic cells twice during 14 days. The resulting effector cells are co-cultured for four hours with 51Cr-labeled target cells (peptide-restricted class I positive tumor cells) at a ratio of 100:1 to 2.5:1 (100:1, 50:1, 25:1, 20:1, 12.5:1, 10:1, 5:1, or 2.5:1), preferably at a ratio of 10:1; and 51Cr released from the target cells is quantified (see Arch Dermatol Res 292:325-332 (2000)). Furthermore, the immature dendritic cells preferably have phagocytic ability for antigens, and more preferably show low (for example, significantly low as compared to mature DCs induced by LPS as described above) or negative expression of receptors that induce the costimulation for T cell activation.

In a preferred embodiment, the first step of the method is carried out in the presence of a composition of matter selected from the group consisting of:
(i) a polypeptide according to the invention,
(ii) an homooligomer according to the invention,
(iii) a polynucleotide according to the invention, and
(iv) a vector according to the invention.

In a more preferred embodiment, the composition of matter is present during the first step of the method of the invention but not during the second step. Preferably, the composition of matter is an homooligomer according to the invention.

The terms "polypeptide according to the invention", "homooligomer according to the invention", "polynucleotide according to the invention" and "vector according to the invention" have been described in detail above.

The step carried out in the presence of a polypeptide of the invention, an homooligomer of the invention, the polynucleotide encoding said molecule or the vector comprising said polynucleotide may be performed *in vivo* or *ex vivo.* Generally, in these methods, immature dendritic cells may be exposed to the polypeptide or homooligomer of the invention within a range having: a lower end of 0.01, 0.05, 0.1, 0.5, 1, 2, 3, 4, 5, 6, 7, 8, 9, 10, 12, 15, 20, 50, or 100 micrograms per ml of media; and an upper end of 0.05, 0.1, 0.5, 1, 2, 3, 4, 5, 6, 7, 8, 9, 10, 12, 15, 20, 50, 100, or 200 micrograms per ml of media. Most preferably, DC are matured in the presence of 1-10 µg/ml of the homooligomer of the invention and, most preferably, at 3, 5 and 10 µg/ml, even most preferably at 3 µg/ml.

In a second step, the immature dendritic cells isolated according to the first step are then incubated under conditions adequate for the maturation of said immature dendritic cells into tolerogenic mature dendritic cells. Compositions highly enriched for tolerogenic dendritic cells are achieved in this manner.

Mature human dendritic cells are cells that are positive for the expression of CD40, CD80, CD83, CD86, and HLA-class II. An immature dendritic cell can be distinguished from a mature dendritic cell, for example, based on markers selected from the group consisting of CD83 and CD86. An immature dendritic cell is weakly positive and preferably negative for the markers CD40, CD80, CD83, and CD86, while a mature dendritic cell is positive.

Mature DCs lose the ability to take up antigen and the cells display up-regulated expression of co-stimulatory cell surface molecules and secrete various cytokines. Specifically, mature DCs usually express higher levels of MHC class I and II antigens and are generally identified as CD80⁺, CD83⁺, and CD86⁺. Greater MHC expression leads to an increase in antigen density on the DC surface, while up regulation of co-stimulatory molecules CD80 and CD86 strengthens the T cell activation signal through the counterparts of the co-stimulatory molecules, such as CD28 on the T cells.

The expression "conditions adequate for the maturation of said immature dendritic cells into tolerogenic mature dendritic cells", as used herein, refers to methods which allow the maturation of an immature dendritic cell into a tolerogenic mature dendritic cell. Mature dendritic cells can be prepared (*i.e.,* matured) by contacting the immature dendritic cells with effective amounts or concentrations of a dendritic cell maturation agent. Dendritic cell maturation agents can include, for example, BCG; IFNγ; TLR4 ligands such as LPS; TNFα; or TLR7 ligands such as gardiquimod and the like. In a preferred embodiment the dendritic cell maturation agent is a TLR4 ligand, preferably LPS. In another embodiment the dendritic cell maturation agent is a TLR7 ligand, preferably gardiquimod. Effective amounts of BCG typically range from about 10⁵ to 10⁷ cfu per milliliter of tissue culture media. Effective amounts of IFNγ typically range from about 100-1000 U per milliliter of tissue culture media. Bacillus Calmette-Guerin (BCG) is an avirulent strain of *M. bovis.* As used herein, BCG refers to whole BCG as well as cell wall constituents, BCG-derived lipoarabidomannans, and other BCG components that are associated with induction of a type 2 immune response. BCG is optionally inactivated, such as heat-inactivated BCG, formalin-treated BCG, and the like. In a preferred embodiment, the LPS is added at 5 µg/ml of tissue culture media. In another embodiment, the gardiquimod is added at 10 µg/ml of tissue culture media.

The immature DCs are typically contacted with effective amounts of BCG and IFNγ, or with LPS, or with gardiquimod, for about one hour to about 48 hours. The immature dendritic cells can be cultured and matured in suitable maturation culture conditions. Suitable tissue culture media include AIM-V, RPMI 1640, DMEM, X-VIVO 15™, and the like. The tissue culture media can be supplemented with amino acids, vitamins, cytokines, such as GM-CSF, divalent cations, and the like, to promote maturation of the cells. Typically, about 500 units/ml of GM-CSF is used.

In a preferred embodiment, the second step of the method is carried out in the presence of a composition of matter selected from the group consisting of:
(i) a polypeptide according to the invention,
(ii) an homooligomer according to the invention,
(iii) a polynucleotide according to the invention, and
(iv) a vector according to the invention.

Maturation of dendritic cells can be monitored by methods known in the art for dendritic cells. Cell surface markers can be detected in assays familiar to the art, such as flow cytometry, immunohistochemistry, and the like. The cells can also be monitored for cytokine production (e.g., by ELISA, another immune assay, or by use of an oligonucleotide array). Mature DCs of the present invention also lose the ability to uptake antigen, which can be analyzed by uptake assays familiar to one of ordinary skill in the art.

The term "mature dendritic cells" refers to dendritic cells that have significantly strong antigen-presenting ability for T cell or the like as compared with in the immature state. Specifically, the mature dendritic cells may have an antigen-presenting ability that is half or stronger, preferably equivalent to or stronger than the antigen-presenting ability of dendritic cells in which maturation has been induced by adding LPS (1 µg/ml) and culturing for two days. Furthermore, the mature dendritic cells preferably have weak or no phagocytic ability for antigen, and more preferably are positive for the expression of receptors that induce the costimulation for T cell activation. The activation of dendritic cells refers to the transition from immature to mature dendritic cell; and the activated dendritic cells encompass mature dendritic cells and dendritic cells in the process of the transition, wherein the expression of CD80 and CD86 that induce costimulatory signals are elevated by the activating stimuli.

The population of tolerogenic cells may comprise at or about 50% or more of the cell composition, and preferably be at or about 75% or more of the cell composition, and may be 90% or more. The desired cells are identified by their surface phenotype, by the ability to induce tolerance, etc. The enriched cell population may be used immediately, or may be frozen at liquid nitrogen temperatures and stored for long periods of time, being thawed and capable of being reused. The cells will usually be stored in 10% DMSO, 50% FCS, 40% RPMI 1640 medium. The population of cells enriched for tolerogenic dendritic cells may be used in a variety of screening assays and cultures, as described below.

DCs may optionally be further purified by sorting of fluorescence-labeled cells using antibodies against DC markers. DCs may also be isolated using antibodies against DCs, wherein the antibodies are linked to magnetic beads. In a specific embodiment, DCs that co-express CD32a and CD32b are isolated using FACS.

The separated cells may be collected in any appropriate medium that maintains the viability of the cells, usually having a cushion of serum at the bottom of the collection tube. Various media are commercially available and may be used according to the nature of the cells. Culture medium may be liquid or semi-solid, e.g. containing agar, methylcellulose, etc. The cell population may be conveniently suspended in an appropriate nutrient medium, such as Iscove's modified dMEM, HBSS, dPBS, RPMI, Iscove's medium, DMEM or RPMI-1640 and the like, normally supplemented with fetal calf serum (about 5-10%), L-glutamine, a thiol, particularly 2-mercaptoethanol, and antibiotics, e.g. penicillin and streptomycin.

Optionally, standard techniques such as morphological observation and immunochemical staining, can be used to verify the presence of dendritic cells. For example, the purity of dendritic cells can be assessed by flow cytometry using fluorochrome-labeled antibodies directed against one or more of the characteristic cell surface markers.

In some embodiments, the tolerogenic dendritic cells are used to produce regulatory T cells (see, e.g., U.S. Pat. App. No. 10/661,804). Briefly, regulatory T cells may be produced from a population comprising CD4+ T cells and/or CD8+ T cells. These T cell populations may be isolated from a subject or may be cultured. Subpopulations of T cells may also be used, such as, for example, populations sorted by cell surface marker so as to comprise enriched populations of particular cells (e.g., CD4+ CD25+ T cells or CD4+ CD25⁻ T cells). The T cells are then cultured or incubated with tolerogenic dendritic cells of the invention. If the regulatory T cells are being produced *in vitro,* the tolerogenic dendritic cells may be allogeneic to or syngeneic with the T cells. In some embodiments, the tolerogenic DCs are loaded with antigen (e.g., pulsed with antigen or transfected with antigen-encoding RNA). In such embodiments, the tolerogenic DCs may present the antigen to T cells. During production of regulatory T cells, the T cells may be exposed to and/or cultured with the tolerogenic DCs once or more than once. For example, the T cells may be cultured with DCs for days or weeks; the DCs in the mixed culture may be replenished if necessary. In some embodiments, culture will continue until a therapeutic amount of regulatory T cells has been obtained. Other culture techniques and/or additives may be used to improve the results obtained; for example, the culture media may also contain cytokines such as IL-2.

Generally, regulatory T cells secrete IL-10 and/or TGF-beta (see, e.g., Walsh et al., 2004, J. Clin. Invest. 114: 1398-1403); assays for confirming secretion of these cytokines are known in the art. In some embodiments, regulatory T cells will inhibit a mixed lymphocyte reaction by at least 10%, 20%, 30%, 40%, 50%, 75%, 90%, 95%, or 100%. Mixed lymphocyte reaction assays are well-known in the art. The regulatory T cells of the invention may be antigen-specific. CD4+ CD25+ regulatory T cells generally express characteristic cell surface markers including CD4, and CD25, and specific intracellular markers such as Foxp3; assays for cell surface and intracellular markers are well-known in the art.

All the embodiments disclosed in the context of the previous aspects of the invention are also applicable to this aspect.

### Methods for the generation of a population of tolerogenic macrophages obtained using the polypeptides, homooligomers, polynucleotides or vectors of the invention

The authors of the present invention have observed that macrophages contacted with either a polypeptide according to the invention, an homooligomer according to the invention, a polynucleotide according to the invention or a vector according to the invention show a tolerogenic phenotype.

Thus, in another aspect, the invention relates to an *in vitro* method for the generation of a population of tolerogenic macrophages comprising the steps of:
(i) incubating a population of macrophage precursor cells under conditions adequate for the formation of a population of immature macrophages, and
(ii) incubating the population of immature macrophages obtained in step (i) under conditions adequate for the formation of mature macrophages
wherein steps (i) and/or (ii) are carried out in the presence of a composition of matter selected from the group consisting of:
(a) a polypeptide of the invention,
(b) an homooligomer of the invention,
(c) a polynucleotide of the invention, and
(d) a vector of the invention.

Therefore, the polypeptide of the invention, the homooligomer of the invention, the polynucleotide of the invention or the vector of the invention can be contacted with the cells during the differentiation stage (i.e., during the time wherein the macrophage precursor cells differentiate into immature macrophages), during the maturation step (i.e., during the time wherein the immature macrophages mature into mature macrophages) or during both stages.

The term "macrophage", as used herein, refers to a mononuclear phagocytic cell which is a type of white blood cell of the immune system that engulfs and digests cellular debris, foreign substances, microbes, cancer cells, and others in a process called phagocytosis. These large phagocytes are found in essentially all tissues. They take various forms with various names throughout the body (e.g., histiocytes, Kupffer cells, alveolar macrophages, microglia, and others), but all are part of the mononuclear phagocyte system. The term macrophage, as used herein, encompasses any of the types of macrophages known. Besides phagocytosis, they play a critical role in nonspecific defence (innate immunity) and also help initiate specific defence mechanisms (adaptative immunity) by recruiting other immune cells such as lymphocytes. Macrophages also play an important anti-inflammatory role and can decrease immune reactions through the release of cytokines. Macrophages are a class of "professional" antigen presenting cells. Macrophages may be recognized by function, or by phenotype, particularly by the cell surface phenotype. The macrophages affected by the methods of the invention may be selected to be immature or mature macrophages.

Macrophages include groups of cells distributed in various tissues and organs in the body that result from *in vitro* differentiation using cytokines or such from bone marrow- or blood-derived stem cells and equivalent cells. Each type of macrophage, determined by its location, has a specific name. Specifically, the macrophages include, for example, adipose tissue macrophages, Kupffer cells (located in the liver), sinus histiocytes (located in the lymph nodes), alveolar macrophages (located in the pulmonary alveoli of lungs), tissue macrophages (histiocytes) leading to giant cells (located in the connective tissue), microglia (located in the central nervous system), Hofbauer cells (located in the placenta), intraglomerular mesangial cells (located in kidneys), osteoclasts (located in bones), epithelioid cells (located in granulomas), red pulp macrophages (located in red pulp of spleen), white-pulp macrophages (located in white-pulp of spleen), marginal-zone macrophages and metallophilic macrophages (located in zones of the spleen), peritoneal macrophages (located in the peritoneal cavity), and LysoMac (located in Peyer's patch). There are a large number of commonly used macrophage markers such as CD14, CD16, CD64, CD68, CD71 and CCR5; the exact marker to be used will be dependent upon the subset of macrophage and the conditions of their local environment. Mature M1 macrophages are typically identified by markers CD86, CD80, CD68, MHC II, IL-1R, TLR2, TLR4, iNOS and/or SOCS3. M2 macrophages, particularly M2 macrophages activated by IL-4, are typically identified by markers CD163, MHC II, SR, MMR/CD206, CD200R, TGM2, DecoyR and/or IL-1R II. Precursors of macrophages include those having the phenotype IL3Ra^{low} CD11b⁻ CD34⁺ c-KIT⁺ FLT3⁺ and IL3Ra^{high} CD11b⁻ CD34⁺ c-KIT⁺ FLT3⁺ (Xiao et al. (2015) Stem Cell Reports 4: 984-994).

The term "tolerogenic macrophage", as used herein, is used interchangeably with "regulatory macrophage" or "Mreg", and "deactivated macrophage". Tolerogenic, regulatory or deactivated macrophages are macrophages that are derived from an immature macrophage exposed to a differentiation stimulus, which can be a combination of cytokines, hormones, vitamins and other biological agents whereby the macrophage acquires the ability of inducing tolerance. A tolerogenic macrophage has low ability to activate effector T cells but high ability to induce and activate regulatory T cells. A tolerogenic macrophage can be seen as a maturation-resistant cell that acts as an "immature macrophage" with a stable phenotype that is preserved, even in the presence of pro-inflammatory signals. Tolerogenic macrophages are capable of modulating the immune system toward a regulatory T_{H}2 response through the production of IL-10 and a limited or absent secretion of IL-12. In addition, tolerogenic macrophages present an increased antigen-presenting functionality with an elevated expression of HLA class II and B7 co-stimulatory molecules.

In a first step, the method for obtaining a population of tolerogenic macrophages comprises incubating a population of macrophage precursor cells under conditions adequate for the formation of a population of immature macrophages.

The term "macrophage precursor cell", as used herein, refers to any cell capable of differentiating into an immature macrophage in the presence of an appropriate cytokine (specifically, G-CSF, GM-CSF, M-CSF, TNF-a, IL-4, IL-13, SCF (c-kit ligand), Flt-3 ligand, or a combination thereof), and preferably is a cell that can differentiate into an immature macrophage in four weeks or less, more preferably in 20 days or less, even more preferably in 18 days or less, and still more preferably in 16 days or less. Examples of macrophage precursor cells include, but are not limited to, myeloid monocyte-macrophage precursor cells; or proliferative, conditional developmentally-arrested, primary macrophage progenitors obtained by overexpression of Hoxb8 in bone marrow progenitors, in media supplemented with GM-CSF or Flt3L. Phenotypic surface markers expressed by various subsets of macrophage precursor cells are well known in the art and may be used for the purpose of identification, for example, by flow cytometry or using immunohistochemical techniques.

In a preferred embodiment, the population of macrophage precursor cells is a population of monocytic macrophage precursor cells. "Monocytic macrophage precursor cells", as used herein, comprise monocytes that have the GM-CSF receptor on their surface and other myeloid precursor cells that are responsive to GM-CSF. The cells can be obtained from any tissue where they reside, particularly lymphoid tissues such as the spleen, bone marrow, lymph nodes and thymus. Monocytic macrophage precursor cells also can be isolated from the circulatory system. Peripheral blood is a readily accessible source of monocytic macrophage precursor cells. Umbilical cord blood is another source of monocytic macrophage precursor cells.

Monocytic macrophage precursor cells may be obtained from peripheral blood mononuclear cells (PBMCs), which can be obtained either from whole blood diluted 1:1 with buffered saline or from leukocyte concentrates ("buffy coat" fractions, MSKCC Blood Bank) by standard centrifugation over Ficoll-Paque PLUS (endotoxin-free, #17-1440-03, Amersham Pharmacia Biotech AB, Uppsala, Sweden). Monocytic macrophage precursors are tissue culture plastic-adherent (#35-3003; Falcon, Becton-Dickinson Labware, Franklin Lakes, NJ) PBMCs, and can be cultured in complete RPMI 1640 plus 1% normal human serum (NHS) in the presence of GM-CSF (1000 IU/ml) and IL-4 (500 IU/ml) with replenishment every 2 days as described. (Thurner B et al., 1999, J. Immunol. Meth. 223:1-15 and Ratzinger G. et al., 2004, J. Immunol. 173:2780-2791).

In general, monocytic macrophage precursor cells may be identified by the expression of markers such as CD13 and CD33. Myeloid macrophage precursors may differentiate into macrophage cells via CD14 or CD16 pathways. Accordingly, a macrophage precursor cell of the invention may be a CD14⁺CD16⁻ macrophage precursor cell, a CD14⁺CD16⁺ macrophage precursor cell, or a CD14^{dim}CD16⁺ macrophage precursor cell. In certain embodiments of the invention, a myeloid macrophage precursor cell may be characterised by a CD34⁺CD33⁺CD7⁻CD10⁻ phenotype. In a preferred embodiment, the myeloid macrophage precursor cell is a CD14⁺ monocyte. The CD14⁺ monocyte may also express the GM-CSF receptor.

The macrophage precursor cells that are being used as starting material for the method of the invention can be autologous to the subject that is to be treated. In other embodiments, the macrophage precursor cells that are being used as starting material for the methods of the invention are heterologous macrophage cells. For example, if graft-versus-host disease is to be treated, the macrophage cells that are being used as starting material are macrophage cells that were obtained from the donor. The subject can be, e.g., a mouse, a rat, a dog, a chicken, a horse, a goat, a donkey, or a primate. Most preferably, the subject is a human.
The expression "conditions adequate for the formation of a population of immature macrophages", as used herein, refers to conditions which result in the differentiation of the macrophage precursor cells into immature macrophages. Suitable conditions include, for example, by culturing for about six days in the presence of GM-CSF and/or M-CSF, optionally including IL-3. In a preferred embodiment, the cells are cultured in the presence of GM-CSF (50 ng/ml) or M-CSF (50 ng/ml).

This treatment leads to the generation of immature macrophages. The term "immature macrophage", as used herein, is interchangeable with the term "uncommitted macrophage", "naive macrophage" or "M0". Immature macrophage, or uncommitted macrophage, or naive macrophage or M0 refers to macrophages having significantly low T-cell activating ability as compared with in a mature state. Specifically, the immature macrophages may have an antigen-presenting ability that is lower than ½, preferably lower than ¼ of that of macrophages which maturation had been induced by adding LPS (1 µ/ml) and culturing for two days. The antigen-presenting ability can be quantified, for example, using the allo T cell-activating ability (mixed lymphocyte test: allo T cells and macrophages are co-cultured at a macrophage: T cell ratio of 1:10, 1:40, 1:80, 1:160; preferably at a macrophage: T cell ratio of 1:40, 1:80, 1:160; 3H-thymidine is added 8 hours before terminating cultivation, and the T cell growth capacity is assessed based on the amount of 3H-thymidine incorporated into the DNA of the T cells (see Gene Therapy 7; 249-254 (2000)). Alternatively, it can be assessed by testing the ability to induce specific cytotoxic T cells (CTLs) using a peptide, in which a known class I-restricted peptide of a certain antigen is added to macrophages; the macrophages are co-cultured with T cells obtained from peripheral blood of the same healthy donor from whom the macrophages had been collected (with 25 U/ml or preferably 100 U/ml of IL-2 on day 3 or later). The T cells are preferably stimulated with macrophages three times during 21 days, more preferably stimulated with macrophages twice during 14 days. The resulting effector cells are co-cultured for four hours with 51Cr-labeled target cells (peptide-restricted class I positive tumor cells) at a ratio of 100:1 to 2.5:1 (100:1, 50:1, 25:1, 20:1, 12.5:1, 10:1, 5:1, or 2.5:1), preferably at a ratio of 10:1; and 51Cr released from the target cells is quantified (see Arch Dermatol Res 292:325-332 (2000)). Furthermore, the immature macrophages preferably have phagocytic ability for antigens, and more preferably show low (for example, significantly low as compared to mature macrophages induced by LPS as described above) or negative expression of receptors that induce the costimulation for T cell activation.

In a preferred embodiment, the first step of the method is carried out in the presence of a composition of matter selected from the group consisting of:
(i) a polypeptide according to the invention,
(ii) an homooligomer according to the invention,
(iii) a polynucleotide according to the invention, and
(iv) a vector according to the invention.

The terms "polypeptide according to the invention", "homooligomer according to the invention", "polynucleotide according to the invention" and "vector according to the invention" have been described in detail above.

The step carried out in the presence of a polypeptide of the invention, an homooligomer of the invention, the polynucleotide encoding said molecule or the vector comprising said polynucleotide may be performed *in vivo* or *ex vivo.* Generally, in these methods, immature macrophages may be exposed to the homooligomer of the invention within a range having: a lower end of 0.01, 0.05, 0.1, 0.5, 1, 2, 3, 4, 5, 6, 7, 8, 9, 10, 12, 15, 20, 50, or 100 micrograms per ml of media; and an upper end of 0.05, 0.1, 0.5, 1, 2, 3, 4, 5, 6, 7, 8, 9, 10, 12, 15, 20, 50, 100, or 200 micrograms per ml of media. Most preferably, macrophages are matured in the presence of 1-10 µg/ml of the homooligomer of the invention and, most preferably, at 3, 5 and 10 µg/ml, even most preferably at 3 µg/ml.

In a second step, the immature macrophages isolated according to the first step are then incubated under conditions adequate for the maturation of said immature macrophages into tolerogenic mature macrophages. Compositions highly enriched for tolerogenic macrophages are achieved in this manner.

Mature human macrophages are cells that are positive for the expression of one or more of these markers depending on the specific type of macrophage: CD86, CD80, CD68, MHC II, IL-1R, TLR2, TLR4, iNOS, SOCS3, CD163, SR, MMR/CD206, CD200R, TGM2, DecoyR, IL-1R II, TLR1, TLR8 and/or VEGF. An immature macrophage has an intermediate or low expression of these markers, preferably is weakly positive for these markers, while a mature macrophage cell is positive. In a preferred embodiment, the marker of immature macrophages is CD64^{high} since upon differentiation into M1 and M2 its expression is CD64^{low} and the expression is CD64⁻ for tolerogenic macrophages. In a preferred embodiment, immature macrophages are CD64^{high}, CD80⁻, CD209⁻, CD11b⁺. In a preferred embodiment, M1 are CD64^{low}, CD80⁺. In a preferred embodiment, M2 are CD209⁺, CD11b⁺.

The term "mature macrophage" encompasses, without limitation, both classically-activated macrophages (M1), and alternatively-activated macrophages (M2).

M1 are referred to herein as "classically-activated macrophages" and are also known as "inflammatory macrophages" or "pro-inflammatory macrophages". M1 are activated by toll-like receptors (TLR) and IFNγ, for example by LPS and IFNγ, and secrete high levels of IL-12 and low levels of IL-10. M1 macrophages exhibit a functional phenotype that is pro-inflammatory, anti-microbial (mediating resistance to intracellular pathogens), tissue destructive, anti-tumoral and phagocytic. Upon activation, these M1 macrophages express a plethora of pro-inflammatory cytokines (TNFα, IL-1β, IL-6, IL-12, IL-18, IL-23), chemokines (CXCL-1, 2, 3, 5, 8, 9, 10, CCL-2, 3, 4, 5, 11, 17 and 22), proteases and ROS/RNS. Therefore, M1 play a pivotal role, not only in innate responses, but also adaptative, antigen-specific responses. M1 macrophages are cells that are positive for the expression of CD64 and CD80.

M2 are referred to herein as "alternatively-activated macrophages" but they are also known as "anti-inflammatory macrophages" or "wound-healing macrophages". M1 are activated by IL-4 or IL-13. M2 exhibit a functionally distinct phenotype to that of M1. M2 functionality is generally described as an anti-inflammatory phenotype by producing anti-inflammatory cytokines, and also mediates tissue remodeling and repair and resistance to parasites. M2 macrophages produce high levels of IL-10, TGF-beta and low levels of IL-12. M2 macrophages are cells that are positive for the expression of CD209 and CD11b markers. M2 have a limited ability to induce antigen-specific responses due to their low HLA expression.

Mature macrophages lose the ability to take up antigen and the cells display up-regulated expression of co-stimulatory cell surface molecules and secrete various cytokines.

The expression "conditions adequate for the maturation of said immature macrophages into tolerogenic mature macrophages", as used herein, refers to methods which allow the maturation of an immature macrophage into a tolerogenic mature macrophage. Mature macrophages can be prepared (i.e., matured) by contacting the immature macrophages with effective amounts or concentrations of a macrophage maturation agent. Macrophage maturation agents are different depending on the mature macrophage to be obtained (M1 or M2). Macrophage maturation agents for obtaining M1 from M0 can include, for example, IPNγ in combination with inflammatory stimuli such as LPS or TNFα; or GM-CSF and further polarising signals such as anoxic environments, β-chemokines and phorbol myristate acetate (PMA) (Foey A.D. Chapter 5: Macrophages: Masters of immune activation, suppression and deviation. In book: Immune response activation. Publisher: InTech, Editors: Guy Huynh Thien Duc, pp. 121-149); or certain TLR agonists that induce both TNF and IPNβ (Mosser D.M. and Edwards J.P. 2008. Nat Rev Immunol, 8(12):958-969). In a preferred embodiment, the macrophage maturation agent for M1 is LPS, preferably LPS at a concentration of 40 ng/ml of tissue culture media. In a more preferred embodiment, the macrophage maturation agent for M1 is a combination of LPS and IFNγ, preferably a combination of LPS at 40 ng/ml and IPNγ at 40 ng/ml. Macrophage maturation agents for obtaining M2 from M0 are, without limitation, IL-4/IL-13, or M-CSF and IL-14. In a preferred embodiment, the macrophage maturation agent for M2 is IL-4, preferably IL-4 at 40 ng/ml.

The immature macrophages are typically contacted with effective amounts the macrophage maturation agents for about one hour to about 48 hours. The immature macrophages can be cultured and matured in suitable maturation culture conditions. Suitable tissue culture media include AIM-V, RPMI 1640, DMEM, X-VIVO 15™, and the like. The tissue culture media can be supplemented with amino acids, vitamins, cytokines, divalent cations, and the like, to promote maturation of cells.

In a preferred embodiment, the second step of the method is carried out in the presence of a composition of matter selected from the group consisting of:
(i) a polypeptide according to the invention,
(ii) an homooligomer according to the invention,
(iii) a polynucleotide according to the invention, and
(iv) a vector according to the invention.

Maturation of macrophages can be monitored by methods known in the art for macrophages. Cell surface markers can be detected in assays familiar to the art, such as flow cytometry, immunohistochemistry, and the like. The cells can also be monitored for cytokine production (*e.g.,* by ELISA, another immune assay, or by use of an oligonucleotide array). Mature macrophages of the present invention also lose the ability to uptake antigen, which can be analyzed by uptake assays familiar to one of ordinary skill in the art.

The term "mature macrophages" refers to macrophages that have significantly strong antigen-presenting ability for T cell or the like as compared with in the immature state. Specifically, the mature macrophages may have an antigen-presenting ability that is half or stronger, preferably equivalent to or stronger than the antigen-presenting ability of macrophages in which maturation has been induced by adding LPS (1 µg/ml) and culturing for two days. Furthermore, the mature macrophages preferably have weak or no phagocytic ability for antigen, and more preferably are positive for the expression of receptors that induce the costimulation for T cell activation. The activation of macrophages refers to the transition from immature to mature macrophages; and the activated macrophages encompass mature macrophages and macrophages in the process of transition.

The population of tolerogenic macrophages may comprise at or about 50% or more of the cell composition, and preferably be at or about 75% or more of the cell composition, and may be 90% or more. The desired cells are identified by their surface phenotype, by the ability to induce tolerance, etc. The enriched cell population may be used immediately, or may be frozen at liquid nitrogen temperatures and stored for long periods of time, being thawed and capable of being reused. The cells will usually be stored in 10% DMSO, 50% FCS, 40% RPMI 1640 medium. The population of cells enriched for tolerogenic macrophages may be used in a variety of screening assays and cultures, as described below.

Macrophages may optionally be further purified by sorting of fluorescence-labeled cells using antibodies against macrophage markers. Macrophages may also be isolated using antibodies against macrophages, wherein the antibodies are linked to magnetic beads.

The separated cells may be collected in any appropriate medium that maintains the viability of the cells, usually having a cushion of serum at the bottom of the collection tube. Various media are commercially available and may be used according to the nature of the cells. Culture medium may be liquid or semi-solid, e.g. containing agar, methylcellulose, etc. The cell population may be conveniently suspended in an appropriate nutrient medium, such as Iscove's modified dMEM, HBSS, dPBS, RPMI, Iscove's medium, DMEM or RPMI-1640 and the like, normally supplemented with fetal calf serum (about 5-10%), L-glutamine, a thiol, particularly 2-mercaptoethanol, and antibiotics, e.g. penicillin and streptomycin.

Optionally, standard techniques such as morphological observation and immunochemical staining, can be used to verify the presence of macrophages. For example, the purity of macrophages can be assessed by flow cytometry using fluorochrome-labeled antibodies directed against one or more of the characteristic cell surface markers.

All the embodiments disclosed in the context of the previous aspects of the invention are also applicable to this aspect.

### Tolerogenic dendritic cells of the invention obtained using polypeptides, homooligomers, polynucleotides and vectors of the invention

It is known that the transcriptomic profile of tolerogenic dendritic cells strongly varies depending on the approach used to generate them (Navarro-Barriuso et al. 2018. Frontiers in Immunology, 9: 2062).

Thus, in another aspect, the invention relates to tolerogenic dendritic cells of the invention, obtained by differentiating and/or maturing dendritic cells by a method of the invention in the presence of a polypeptide of the invention, an homooligomer of the invention, a polynucleotide encoding any of said polypeptides or homooligomers, or a vector comprising said polynucleotide. Preferably, relates to tolerogenic dendritic cells of the invention obtained by a method of the invention in the presence of an homooligomer of the invention, a polynucleotide encoding said homooligomer or a vector comprising said polynucleotide, preferably in the presence of a homooligomer of the invention.

The tolerogenic dendritic cells according to the invention are characterized by showing one or more of the following features:
- The cell is CD83⁻, CD86⁻, CD80⁻, CD40⁻ and/or CCR7⁻. The term "negative", when applied to a given marker, indicates that the level of expression of a particular cell surface marker on a tolerogenic DC produced by a method of the invention is decreased by 10%, 20%, 30%, 40%, 50%, 60%, 70%, 80%, 90%, 95%, 97%, 98%, 99%, or 100%, or is undetectable, in comparison to the level of expression of the same cell surface marker on an appropriate control cell (e.g., a native mature immunostimulatory DC, or a mature immunostimulatory DC obtained via *in vitro* maturation, preferably LPS-matured).
- The cell shows an increased antigen internalization capacity or endocytic activity when immature with respect to immature dendritic cells. The term "increased antigen internalization capacity" or "increased endocytic activity", as used herein, indicates that the level of antigen internalization or endocytic activity of an immature tolerogenic DC produced by a method of the invention is increased by 1%, 5%, 10%, 20%, 30%, 40%, 50%, 60%, 70%, 80%, 90%, 95%, 97%, 98%, 99%, 100%, 200%, 300% or more, in comparison to the level of antigen internalization or endocytic activity on an appropriate control cell (e.g., a native immature DC, or an immature DC obtained via *in vitro* differentiation). The antigen internalization capacity or endocytic activity of a tolerogenic dendritic cell can be determined, for instance, as described in materials and methods, example 2 and Figure 5 of the present invention, by measuring the fluorescent DQ-OVA internalization and processing at the differentiation stage.
- The cell does not secrete or secretes reduced amounts of inflammatory cytokines such as IL-12p70 and/or TNF-α with respect to mature dendritic cells. The term "secrete reduced amounts", when applied to a given cytokine, indicates that the level of secretion of a particular cytokine on a tolerogenic DC produced by a method of the invention is decreased by 10%, 20%, 30%, 40%, 50%, 60%, 70%, 80%, 90%, 95%, 97%, 98%, 99%, or 100%, or is undetectable, in comparison to the level of secretion of the same cytokine on an appropriate control cell (e.g., a native mature immunostimulatory DC, or a mature immunostimulatory DC obtained via *in vitro* maturation, preferably LPS-matured).
- The cell is HLA-DR⁺ and/or CD14⁺. The term "positive", when applied to a given marker, indicates that the level of expression of a particular cell surface marker on a tolerogenic DC produced by a method of the invention is substantially the same as in an appropriate control cell (e.g., a native mature immunostimulatory DC, or a mature immunostimulatory DC obtained via *in vitro* maturation, preferably LPS-matured).
- The cell shows reduced chemotactic behaviour towards CCL21 with respect to mature dendritic cells. The term "reduced chemotactic behaviour", as used herein, indicates that the level of chemotaxis of a tolerogenic DC produced by a method of the invention towards CCL21 is decreased by 10%, 20%, 30%, 40%, 50%, 60%, 70%, 80%, 90%, 95%, 97%, 98%, 99%, or 100%, or is undetectable, in comparison to the level of chemotaxis towards the same cytokine on an appropriate control cell (e.g., a native mature immunostimulatory DC, or a mature immunostimulatory DC obtained via *in vitro* maturation, preferably LPS-matured). The chemotactic behaviour of a tolerogenic dendritic cell towards CCL21 can be determined, for instance, as described in materials and methods, example 7 and Figure 12 of the present invention.
- The cell shows reduced expression at the transcriptional level of PTGER3 and/or EGLN3 when immature with respect to immature DCs. The term "reduced expression", when applied to a given gene, indicates that the level of expression of a particular gene on a tolerogenic immature DC produced by a method of the invention is decreased by 10%, 20%, 30%, 40%, 50%, 60%, 70%, 80%, 90%, 95%, 97%, 98%, 99%, or 100%, or is undetectable, in comparison to the level of expression of the same gene on an appropriate control cell (e.g., a native immature DC, or an immature DC obtained via *in vitro* differentiation). The level of expression of a gene can be determined, for instance, as described in materials and methods, Example 8 and Figure 13 of the present invention. In a preferred embodiment, the expression is measured at days 4 or 5 of differentiation.
- The cell shows increased expression at the transcriptional level of "find me" or *smell* receptors such as FPR2, P2RY2 and/or S1PR1 when immature with respect to immature dendritic cells. The term "increased expression", when applied to a given gene, indicates that the level of expression of a particular gene on a tolerogenic immature DC produced by a method of the invention is increased by 1%, 5%, 10%, 20%, 30%, 40%, 50%, 60%, 70%, 80%, 90%, 95%, 97%, 98%, 99%, 100%, 200%, 300% or more, in comparison to the level of expression of the same gene on an appropriate control cell (e.g., a native immature DC, or an immature DC obtained via *in vitro* differentiation). The level of expression of a gene can be determined, for instance, as described in materials and methods, Example 8 and Figure 14 of the present invention. In a preferred embodiment, the expression is measured at day 5 of differentiation, and/or
- The cell remains viable after the differentiation/maturation process, i.e., they do not undergo apoptosis. The term "viable", as used herein, refers to populations wherein less than 50%, less than 40%, less than 30%, less than 20%, less than 10%, less than 5%, less than 4%, less than 3%, less than 2%, or less than 1% undergo apoptosis after the treatment with a maturation stimuli (e.g. LPS). Apoptosis can be determined by any method commonly known in the art such as Annexin V/7-ADD staining, caspase-3 activation assay, TUNEL and DNA fragmentation assay, determination of the mitochondrial membrane potential and the like. The viability of the cells can be determined, for example, as described in materials and methods and Example 2 of the present invention.

Therefore, in a preferred embodiment, the tolerogenic dendritic cells according to the invention are characterized by showing one or more of the above features.

The inventors have found that some "find me" receptors, particularly FPR2, P2RY2, and S1PR1 have increased expression at the transcriptional level in the immature tolerogenic dendritic cells obtained by the method of the invention with respect to immature dendritic cells. However, said genes are not overexpressed when tolerogenic dendritic cells are obtained by other methods, for example, by incubation with the physiological isoform of C4BP formed by 7 alpha chains and lacking the beta chain (C4BP(a7β0)) (named PRP-HO7) (see figure 14).

Therefore, in a preferred embodiment, the tolerogenic dendritic cells according to the invention are characterized by showing overexpression of one or more of the genes FPR2, P2RY2 and/or S1PR1 when compared with immature dendritic cells.

Therefore, in a preferred embodiment, the tolerogenic dendritic cells according to the invention are FPR2^{high}, P2RY2^{high}, and/or S1PR1^{high}. In an embodiment, the tolerogenic dendritic cells of the invention are FPR2^{high}. In another embodiment, the tolerogenic dendritic cells of the invention are P2RY2^{high}. In another embodiment, the tolerogenic dendritic cells of the invention are S1PR1^{high}. In another embodiment, the tolerogenic dendritic cells of the invention are FPR2^{high}, and P2RY2^{high}. In another embodiment, the tolerogenic dendritic cells of the invention are FPR2^{high} and S1PR1^{high}. In another embodiment, the tolerogenic dendritic cells of the invention are P2RY2^{high}, and S1PR1^{high}. In a more preferred embodiment, the tolerogenic dendritic cells of the invention are FPR2^{high}, P2RY2^{high}, and S1PR1^{high}.

The term "high" when applied to a given gene, indicates that the level of expression of a particular gene on a tolerogenic DC produced by a method of the invention is increased by 1%, 5%, 10%, 20%, 30%, 40%, 50%, 60%, 70%, 80%, 90%, 95%, 97%, 98%, 99%, 100%, 200%, 300% or more, in comparison to the level of expression of the same gene on an appropriate control cell (i.e., native immature DC, or an immature DC obtained via *in vitro* differentiation), i.e., that the gene is overexpressed in comparison to immature DC.

In another embodiment, the invention relates to a cell population comprising tolerogenic dendritic cells of the invention. In a preferred embodiment, the cell population comprises at least 5%, at least 10%, at least 20%, at least 30%, at least 40%, at least 50%, at least 60%, at least 70%, at least 80%, at least 85%, at least 90%, at least 95%, at least 96%, at least 97%, at least 98%, at least 99% or 100% of tolerogenic dendritic cells of the invention. In a preferred embodiment the cell population comprises at least 80% of tolerogenic dendritic cells of the invention.

All the embodiments disclosed in the context of the previous aspects of the invention are also applicable to this aspect.

### Tolerogenic macrophages of the invention obtained using polypeptides, homooligomers, polynucleotides and vectors of the invention

It is known that the transcriptomic profile of tolerogenic macrophages strongly varies depending on the approach used to generate them (Navarro-Barriuso et al. 2018. Frontiers in Immunology, 9: 2062).

Thus, in another aspect, the invention relates to tolerogenic macrophages of the invention, obtained by differentiating and/or maturing macrophages by a method of the invention in the presence of a polypeptide of the invention, a homooligomer of the invention, a polynucleotide encoding any of said polypeptides or homooligomers, or a vector comprising said polynucleotide. Preferably, relates to tolerogenic macrophages of the invention obtained by a method of the invention in the presence of a homooligomer of the invention, a polynucleotide encoding said homooligomer or a vector comprising said polynucleotide; preferably in the presence of a homooligomer of the invention.

The tolerogenic macrophages according to the invention are characterized by showing one or more of the following features:
- The cell is CD64⁻ and/or CD80⁻. The term "negative", when applied to CD64 and CD80 markers, indicates that the level of expression of these particular cell surface markers on a tolerogenic macrophage produced by a method of the invention is decreased by 10%, 20%, 30%, 40%, 50%, 60%, 70%, 80%, 90%, 95%, 97%, 98%, 99%, or 100%, or is undetectable, in comparison to the level of expression of the same cell surface marker on an appropriate control cell (e.g., a native mature M1 macrophage, or a mature M1 macrophage obtained via *in vitro* maturation).
- The cell is CD209⁻ and/or CD11b⁻. The term "negative", when applied to CD209 and CD11b markers, indicates that the level of expression of these particular cell surface markers on a tolerogenic macrophage produced by a method of the invention is decreased by 10%, 20%, 30%, 40%, 50%, 60%, 70%, 80%, 90%, 95%, 97%, 98%, 99%, or 100%, or is undetectable, in comparison to the level of expression of the same cell surface marker on an appropriate control cell (e.g., a native mature M2 macrophage, or a mature M2 macrophage obtained via *in vitro* maturation).
- The cell shows reduced expression at the transcriptional level of ALCAM, SLC16A1 and/or LMNB1 when immature with respect to immature macrophages M0. The term "reduced expression", when applied to a given gene, indicates that the level of expression of a particular gene on a tolerogenic macrophage produced by a method of the invention is decreased by 10%, 20%, 30%, 40%, 50%, 60%, 70%, 80%, 90%, 95%, 97%, 98%, 99%, or 100%, or is undetectable, in comparison to the level of expression of the same gene on an appropriate control cell (e.g., a native immature M0 macrophage, or an immature M0 macrophage obtained via *in vitro* differentiation). The level of expression of a gene can be determined, for instance, as described in materials and methods, Example 10 and Figure 16. In a preferred embodiment, the expression is measured at days 2, 4 or 6 of differentiation.
- The cell shows increased expression at the transcriptional level of "find me" or *smell* receptors such as CCR2, CX3CR1, CXCR1, FPR2 and/or P2RY2 when immature with respect to immature macrophages M0. The term "increased expression", when applied to a given gene, indicates that the level of expression of a particular gene on a tolerogenic macrophage produced by a method of the invention is increased by 1%, 5%, 10%, 20%, 30%, 40%, 50%, 60%, 70%, 80%, 90%, 95%, 97%, 98%, 99%, 100%, 200%, 300% or more, in comparison to the level of expression of the same gene on an appropriate control cell (e.g., a native immature M0 macrophage, or an immature M0 macrophage obtained via *in vitro* differentiation). The level of expression of a gene can be determined, for instance, as described in materials and methods, Example 10 and Figure 17. In a preferred embodiment, the expression is measured at day 6 of differentiation.
- The cell secretes reduced amounts of inflammatory cytokines, and chemokines such as CCL1, CCL2, MIP-1, CCL5, CXCL1, IL-6 and/or TNF-α with respect to mature M1 macrophages. The term "secretes reduced amounts", when applied to a given cytokine, chemokine or acute phase protein indicates that the level of secretion of a particular cytokine or chemokine on a tolerogenic macrophage produced by a method of the invention is decreased by 10%, 20%, 30%, 40%, 50%, 60%, 70%, 80%, 90%, 95%, 97%, 98%, 99%, or 100%, or is undetectable, in comparison to the level of secretion of the same cytokine or chemokine on an appropriate control cell (e.g., a native mature M1 macrophage, or a mature M1 macrophage obtained via *in vitro* maturation).

Therefore, in a preferred embodiment, the tolerogenic macrophages according to the invention are characterized by showing one or more of the above features.

In a preferred embodiment, the tolerogenic macrophage of the invention shows reduced expression at the transcriptional level of ALCAM, SLC16A1 and/or LMNB1 with respect to immature macrophages M0. In a more preferred embodiment, the tolerogenic macrophage of the invention shows reduced expression of ALCAM at the transcriptional level with respect to immature macrophages M0, preferably at days 2, 4 or 6 of differentiation, more preferably at day 2 of differentiation. In another embodiment, the tolerogenic macrophage of the invention shows reduced expression of SLC16A1 at the transcriptional level with respect to immature macrophages M0, preferably at 4 or 6 days of differentiation. In another embodiment, the tolerogenic macrophage of the invention shows reduced expression of LMNB1 at the transcriptional level with respect to immature macrophages M0, preferably at 2 or 4 days of differentiation.

In a preferred embodiment, the tolerogenic macrophage of the invention is CD64⁻ and/or CD80⁻. In a preferred embodiment, the tolerogenic macrophage obtained by differentiating monocytes to M0 in the presence of the homooligomers of the invention and further maturing M0 with LPS and IPNγ is CD64⁻ and/or CD80⁻. In another preferred embodiment, the tolerogenic macrophage of the invention secretes reduced amounts of CCL1, CCL2, MIP-1, CCL5, CXCL1, IL-6 and/or TNF-α with respect to mature M1 macrophages. In a preferred embodiment, the tolerogenic macrophage of the invention secretes reduced amounts of CCL1, CCL2 and/or MIP-1. In a more preferred embodiment, the tolerogenic macrophage of the invention secretes reduced amounts of CCL2 with respect to mature M1 macrophages.

In a preferred embodiment, the tolerogenic macrophage of the invention is CD209⁻ and/or CD11b⁻. In a preferred embodiment, the tolerogenic macrophage obtained by differentiating monocytes to M0 in the presence of the homooligomers of the invention and further maturing M0 with IL-4 is CD209⁻ and/or CD11b⁻.

The CD64 and CD11b cell surface markers are even more underexpressed when the tolerogenic macrophages are obtained with the method of the invention than when the tolerogenic macrophages are obtained by treatment with the physiological isoform C4BP(α7β0) (see Figure 15). Therefore, in an embodiment, the tolerogenic macrophage of the invention is CD64⁻, preferably wherein the level of expression of CD64 is decreased to 25% in comparison to the level of expression in a M1 macrophage. In another embodiment, the tolerogenic macrophage of the invention is CD11b⁻, preferably wherein the level of expression of CD11b is decreased to 18% in comparison to the level of expression in a M2 macrophage.

The tolerogenic macrophages of the invention secrete even less CCL1 and MIP-1 than when the tolerogenic macrophages are obtained by treatment with the physiological isoform C4BP(α7β0) (see Figure 18). Therefore, in an embodiment, the tolerogenic macrophage of the invention secretes reduced amounts of CCL1 with respect to mature M1 macrophages, preferably the level of expression of CCL1 is decreased to 70% in comparison to the level of expression in a M1 macrophage. In another embodiment, the tolerogenic macrophage of the invention secretes reduced amounts of MIP-1 with respect to mature M1 macrophages, preferably the level of expression of MIP-1 is decreased to 63% in comparison to the level of expression in a M1 macrophage.

The inventors have found that some "find me" receptors, particularly CCR2, CX3CR1, CXCR1, FPR2 and P2RY2 have increased expression at the transcriptional level in the tolerogenic macrophages obtained by the method of the invention with respect to immature M0 macrophages. However, said genes are not overexpressed when tolerogenic macrophages are obtained by other methods, for example, by incubation with the physiological isoform of C4BP formed by 7 alpha chains and lacking the beta chain (C4BP(α7β0)) (named PRP-HO7) (see figure 17).

Therefore, in a preferred embodiment, the tolerogenic macrophages according to the invention are characterized by showing overexpression of one or more of the genes CCR2, CX3CR1, CXCR1, FPR2 and/or P2RY2 when compared with immature macrophages M0.

Therefore, in a preferred embodiment, the tolerogenic macrophages according to the invention are CCR2^{high}, CX3CR1^{high}, CXCR1^{high}, FPR2^{high} and/or P2RY2^{high}. In an embodiment, the tolerogenic macrophages of the invention are CCR2^{high}. In another embodiment, the tolerogenic macrophages of the invention are CX3CR1^{high}. In another embodiment, the tolerogenic macrophages of the invention are CXCR1^{high}. In another embodiment, the tolerogenic macrophages of the invention are FPR2^{high}. In another embodiment, the tolerogenic macrophages of the invention are P2RY2^{high}. In another embodiment, the tolerogenic macrophages of the invention are CCR2^{high} and CX3CR1^{high}. In another embodiment, the tolerogenic macrophages of the invention are CCR2^{high} and CXCR1^{high}. In another embodiment, the tolerogenic macrophages of the invention are CCR2^{high} and FPR2^{high}. In another embodiment, the tolerogenic macrophages of the invention are CCR2^{high} and P2RY2^{high}. In another embodiment, the tolerogenic macrophages of the invention are CX3CR1^{high} and CXCR1^{high}. In another embodiment, the tolerogenic macrophages of the invention are CX3CR1^{high} and FPR2^{high}. In another embodiment, the tolerogenic macrophages of the invention are CX3CR1^{high} and P2RY2^{high}. In another embodiment, the tolerogenic macrophages of the invention are CXCR1^{high} and FPR2^{high}. In another embodiment, the tolerogenic macrophages of the invention are CXCR1^{high} and P2RY2^{high}. In another embodiment, the tolerogenic macrophages of the invention are FPR2^{high}, and P2RY2^{high}. In another embodiment, the tolerogenic macrophages of the invention are CCR2^{high}, CX3CR1^{high}, CXCR1^{high}. In another embodiment, the tolerogenic macrophages of the invention are CCR2^{high}, CX3CR1^{high} and FPR2^{high}. In another embodiment, the tolerogenic macrophages of the invention are CCR2^{high}, CX3CR1^{high} and P2RY2^{high}. In another embodiment, the tolerogenic macrophages of the invention are CCR2^{high}, CXCR1^{high}, FPR2^{high}. In another embodiment, the tolerogenic macrophages of the invention are CCR2^{high}, CXCR1^{high} and P2RY2^{high}. In another embodiment, the tolerogenic macrophages of the invention are CCR2^{high}, FPR2^{high} and/or P2RY2^{high}. In another embodiment, the tolerogenic macrophages of the invention are CX3CR1^{high}, CXCR1^{high} and FPR2^{high}, In another embodiment, the tolerogenic macrophages of the invention are CX3CR1^{high}, CXCR1^{high} and P2RY2^{high}. In another embodiment, the tolerogenic macrophages of the invention are CX3CR1^{high}, FPR2^{high} and P2RY2^{high}. In another embodiment, the tolerogenic macrophages of the invention are CXCR1^{high}, FPR2^{high} and P2RY2^{high} In another embodiment, the tolerogenic macrophages of the invention are CCR2^{high}, CX3CR1^{high}, CXCR1^{high} and FPR2^{high}. In another embodiment, the tolerogenic macrophages of the invention are CCR2^{high}, CX3CR1^{high}, CXCR1^{high} and P2RY2^{high}. In another embodiment, the tolerogenic macrophages of the invention are CX3CR1^{high}, CXCR1^{high}, FPR2^{high} and P2RY2^{high}. In another embodiment, the tolerogenic macrophages of the invention are CCR2^{high}, CX3CR1^{high} , FPR2^{high} and P2RY2^{high}. In a more preferred embodiment, the tolerogenic macrophages of the invention are CCR2^{high}, CX3CR1^{high}, CXCR1^{high}, FPR2^{high} and P2RY2^{high}.

The term "high" when applied to a given gene, indicates that the level of expression of a particular gene on a tolerogenic macrophage produced by a method of the invention is increased by 1%, 5%, 10%, 20%, 30%, 40%, 50%, 60%, 70%, 80%, 90%, 95%, 97%, 98%, 99%, 100%, 200%, 300% or more, in comparison to the level of expression of the same gene on an appropriate control cell (e.g., a native immature M0 macrophage, or an immature M0 macrophage obtained via *in vitro* differentiation), i.e., that the gene is overexpressed in comparison to immature M0 macrophages.

In another embodiment, the invention relates to a cell population comprising tolerogenic macrophages of the invention. In a preferred embodiment, the cell population comprises at least 5%, at least 10%, at least 20%, at least 30%, at least 40%, at least 50%, at least 60%, at least 70%, at least 80%, at least 85%, at least 90%, at least 95%, at least 96%, at least 97%, at least 98%, at least 99% or 100% of tolerogenic macrophages of the invention. In a preferred embodiment the cell population comprises at least 80% of tolerogenic macrophages of the invention.

All the embodiments disclosed in the context of the previous aspects of the invention are also applicable to this aspect.

### Therapeutic uses of the tolerogenic dendritic cells and tolerogenic macrophages of the invention

Tolerogenic dendritic cells and tolerogenic macrophages are useful in the treatment of patients with immunological diseases, particularly in the treatment of autoimmune diseases such as type I diabetes, rheumatoid arthritis or Crohn's disease, as well as kidney transplantation (Navarro-Barriuso et al. 2018. Frontiers in Immunology, 9: 2062).

Therefore, in another aspect, the invention relates to a pharmaceutical composition comprising a tolerogenic dendritic cell of the invention, or a tolerogenic macrophage according to the invention, or a cell population according to the invention, and a pharmaceutically acceptable carrier.

All the embodiments disclosed in the context of the previous aspects of the invention are also applicable to this aspect, particularly all the embodiments and definitions disclosed in the context of the pharmaceutical compositions of the invention.

In another aspect, the invention relates to a tolerogenic dendritic cell of the invention, a tolerogenic macrophage of the invention, a cell population of the invention, or a pharmaceutical composition comprising thereof for use in medicine.

In another aspect, the invention relates to the use of a tolerogenic dendritic cell of the invention, a tolerogenic macrophage of the invention, a cell population of the invention, or a pharmaceutical composition comprising thereof for the manufacture of a medicament.

In another aspect, the invention relates to a tolerogenic dendritic cell of the invention, a tolerogenic macrophage of the invention, a cell population of the invention, or a pharmaceutical composition comprising thereof for use in the prevention and/or treatment of an immunological disease caused by an undesired activation of the immune system.

In another aspect, the invention relates to the use of a tolerogenic dendritic cell of the invention, a tolerogenic macrophage of the invention, a cell population of the invention, or a pharmaceutical composition comprising thereof for the manufacture of a medicament for the prevention and/or treatment of an immunological disease caused by an undesired activation of the immune system.

In another aspect, the invention relates to a method for the prevention and/or treatment of an immunological disease caused by an undesired activation of the immune system, in a subject in need thereof comprising the administration to said subject of a tolerogenic dendritic cell of the invention, a tolerogenic macrophage of the invention, a cell population of the invention, or a pharmaceutical composition comprising thereof.

In a preferred embodiment, the immunological disease is selected from the group consisting of an immunoinflammatory disease, sepsis, an autoimmune disease, transplant rejection, graft-versus-host disease, and a hypersensitivity disease.

The dose of the composition for treating an immunological disease or disorder may be determined according to parameters understood by a person skilled in the medical art. Accordingly, the appropriate dose may depend upon the patient's (e.g., human) condition, that is, stage of the disease, general health status, as well as age, gender, and weight, and other factors familiar to a person skilled in the medical art.

The terms "treatment", "prevention", "subject", "immunological disease caused by an undesired activation of the immune system", "immunoinflammatory disease", "sepsis", "autoimmune disease", "transplant rejection", "graft-versus-host disease" and "hypersensitivity disease" have been described in detail above and are used with the same meaning in the context of the therapeutic methods of the invention.

All the embodiments disclosed in the context of the previous aspects of the invention are also applicable to these aspects, particularly all the embodiments and definitions disclosed in the context of therapeutic methods of the invention.

The invention also relates to the following:
[1]. A recombinant polypeptide comprising the CCP6 domain of the C4BP alpha chain or a functionally equivalent variant thereof and an oligomerization domain, wherein said polypeptide does not comprise CCP1, CCP2, CCP3, CCP4, CCP5, CCP7 nor CCP8 domains of the C4BP alpha chain.
[2]. The recombinant polypeptide according to [1], wherein the CCP6 domain is the CCP6 domain of the human C4BP alpha chain.
[3]. The recombinant polypeptide according to any one of [1] or [2], wherein the CCP6 domain of the C4BP alpha chain is SEQ ID NO: 1.
[4]. The recombinant polypeptide according to any one of [1] to [3], wherein the oligomerization domain is the oligomerization domain of C4BP alpha chain or a functionally equivalent variant thereof.
[5]. The recombinant polypeptide according to any one of [1] to [4], wherein the oligomerization domain is the oligomerization domain of human C4BP alpha chain or a functionally equivalent variant thereof.
[6]. The recombinant polypeptide according to any one of [1] to [5], wherein the oligomerization domain is SEQ ID NO: 2 or a functionally equivalent variant thereof, preferably SEQ ID NO: 2.
[7]. The recombinant polypeptide according to any one of [1] to [6], wherein the polypeptide comprises in an amino terminal to carboxy terminal direction, (a) the CCP6 domain, and (b) the oligomerization domain.
[8]. The recombinant polypeptide according to any one of [1] to [7], wherein the polypeptide consists of SEQ ID NO: 3 or a functionally equivalent variant thereof, preferably SEQ ID NO: 3.
[9]. The recombinant polypeptide according to any one of [1] to [8], wherein the polypeptide further comprises a signal peptide.
[10]. The recombinant polypeptide according to [9], wherein the signal peptide is the signal peptide of the C4BP alpha chain, preferably the peptide with SEQ ID NO: 4 or a functionally equivalent variant thereof, preferably SEQ ID NO: 4.
[11]. The recombinant polypeptide according to [10], wherein the polypeptide consists of SEQ ID NO: 5 or a functionally equivalent variant thereof, preferably SEQ ID NO: 5.
[12]. The recombinant polypeptide according to any one of [1] to [11], wherein the polypeptide further comprises a peptide that is not part of the C4BP alpha chain, preferably a tag peptide.
[13]. The recombinant polypeptide according to [12], wherein the tag peptide is linked to the N-terminal end of the CCP6 domain.
[14]. The recombinant polypeptide according to any one of [12] or [13], wherein the tag peptide is a poly-histidine tag.
[15]. The recombinant polypeptide according to [14], wherein the polypeptide consists of SEQ ID NO: 6 or a functionally equivalent variant thereof, preferably SEQ ID NO: 6.
[16]. The recombinant polypeptide according to [14], wherein the polypeptide consists of SEQ ID NO: 7 or a functionally equivalent variant thereof, preferably SEQ ID NO: 7.
[17]. An homooligomer of at least six recombinant polypeptides according to any one of [1] to [16].
[18]. The homooligomer according to [17], wherein the homooligomer is formed by seven recombinant polypeptides according to any one of [1] to [16].
[19]. The homooligomer according to [17], wherein the homooligomer consists of seven recombinant polypeptides according to any one of [1] to [16].
[20]. The homooligomer according to any one of [17] to [19], wherein the polypeptide is the polypeptide according to [15].
[21]. A polynucleotide encoding a recombinant polypeptide according to any one of [1] to [16].
[22]. A vector comprising a polynucleotide according to [21].
[23]. A host cell comprising a vector according to [22].
[24]. A pharmaceutical composition comprising a recombinant polypeptide according to any one of [1] to [16], an homooligomer according to any one of [17] to [20], a polynucleotide according to [21], a vector according to [22], or a host cell according to [23] and a pharmaceutically acceptable carrier.
[25]. A recombinant polypeptide according to any one of [1] to [16], an homooligomer according to any one of [17] to [20], a polynucleotide according to [21], a vector according to [22], a host cell according to [23] or a pharmaceutical composition according to [24] for use in medicine.
[26]. A recombinant polypeptide according to any one of [1] to [16], an homooligomer according to any one of [17] to [20], a polynucleotide according to [21], a vector according to [22], a host cell according to [23], or a pharmaceutical composition according to [24] for use in the prevention and/or treatment of an immunological disease caused by an undesired activation of the immune system.
[27]. The recombinant polypeptide according to any one of [1] to [16], the homooligomer according to any one of [17] to [20], the polynucleotide according to [21], the vector according to [22], the host cell according to [23], or the pharmaceutical composition according to [24] for use according to [26], wherein the immunological disease is selected from the group consisting of an immunoinflammatory disease, sepsis, an autoimmune disease, transplant rejection, graft-versus-host disease and a hypersensitivity disease.
[28]. The recombinant polypeptide according to any one of [1] to [16], the homooligomer according to any one of [17] to [20], the polynucleotide according to [21], the vector according to [22], the host cell according to [23], or the pharmaceutical composition according to [24] for use according to [27], wherein the autoimmune disease is selected from the group consisting of lupus nephritis, systemic lupus erythematosus, inflammatory bowel disease and rheumatoid arthritis.
[29]. The recombinant polypeptide according to any one of [1] to [16], the homooligomer according to any one of [17] to [20], the polynucleotide according to [21], the vector according to [22], the host cell according to [23], or the pharmaceutical composition according to [24] for use according to [28], wherein the autoimmune disease is inflammatory bowel disease.
[30]. An *in vitro* method for the generation of a population of tolerogenic dendritic cells comprising the steps of:
   (i) incubating a population of dendritic precursor cells under conditions adequate for the formation of a population of immature dendritic cells, and
   (ii) incubating the population of immature dendritic cells obtained in step (i) under conditions adequate for the formation of mature dendrite cells
   wherein steps (i) and/or (ii) are carried out in the presence of a composition of matter selected from the group consisting of:
   (a) a polypeptide according to any one of [1] to [16],
   (b) an homooligomer according to any one of [17] to [20],
   (c) a polynucleotide according to [21], and
   (d) a vector according to [22].
[31]. The method according to [30], wherein the population of dendritic precursor cells is a monocyte population.
[32]. An *in vitro* method for the generation of a population of tolerogenic macrophages comprising the steps of:
   (i) incubating a population of macrophage precursor cells under conditions adequate for the formation of a population of immature macrophages, and
   (ii) incubating the population of immature macrophages obtained in step (i) under conditions adequate for the formation of mature macrophages
   wherein steps (i) and/or (ii) are carried out in the presence of a composition of matter selected from the group consisting of:
   (a) a polypeptide according to any one of [1] to [16],
   (b) an homooligomer according to any one of [17] to [20],
   (c) a polynucleotide according to [21], and
   (d) a vector according to [22].
[33]. The method according to [32], wherein the population of macrophage precursor cells is a monocyte population.
[34]. A tolerogenic dendritic cell obtained by the method according to any one of [30] or [31], or a tolerogenic macrophage obtained by the method according to any one of [32] or [33].
[35]. The tolerogenic dendritic cell according to [34], wherein said cell is FPR2^{high}, P2RY2^{high}, and/or S1PR1^{high}.
[36]. The tolerogenic macrophage according to [34], wherein said macrophage is CCR2^{high}, CX3CR1^{high}, CXCR1^{high}, FPR2^{high} and/or P2RY2^{high}.
[37]. A cell population comprising at least 80% of tolerogenic dendritic cells according to [34] or [35], or at least 80% of tolerogenic macrophages according to [34] or [36].
[38]. A pharmaceutical composition comprising a tolerogenic dendritic cell according to [34] or [35], a tolerogenic macrophage according to [34] or [36] or a cell population according to [37], and a pharmaceutically acceptable carrier.
[39]. A tolerogenic dendritic cell according to [34] or [35], a tolerogenic macrophage according to [34] or [36], a cell population according to [37], or a pharmaceutical composition according to [38] for use in medicine.
[40]. A tolerogenic dendritic cell according to [34] or [35], a tolerogenic macrophage according to [34] or [36], a cell population according to [37], or a pharmaceutical composition according to [38] for use in the prevention and/or treatment of an immunological disease caused by an undesired activation of the immune system.
[41]. A tolerogenic dendritic cell according to [34] or [35], a tolerogenic macrophage according to [34] or [36], a cell population according to [37], or a pharmaceutical composition according to [38] for use according to [40], wherein the immunological disease is selected from the group consisting of an immunoinflammatory disease, sepsis, an autoimmune disease, transplant rejection, graft-versus-host disease and a hypersensitivity disease.
[42]. A method for increasing tolerogenic dendritic cell populations and/or tolerogenic macrophage populations in a subject in need thereof which comprises the administration to said subject of a polypeptide according to any one of [1] to [16], an homooligomer according to any one of [17] to [20], a polynucleotide according to [21], a vector according to [22], a host cell according to [23], or a pharmaceutical composition according to [24], or combinations thereof.
The invention is described in detail by way of the following examples which are to be considered as merely illustrative and not limitative of the scope of the invention.

### EXAMPLES

### MATERIALS AND METHODS

### Obtention and purification of PRP-HE8, PRP-HO7, PRP6-HO7 and PRP6-NO

PRP-HE8 corresponds to the physiological isoform (C4BP(β⁺)) also named C4BP(α₇β₁) formed by 7 α-chains and 1 β-chain which was purified from human plasma (Olivar et al. 2013. J Immunol, 190(6): 2857-2872).

PRP-HO7 corresponds to the physiological isoform (C4BP(β⁻)) also named C4BP(α₇β₀) formed by 7 α-chains but lacking the β-chain which was also purified from human plasma (Olivar et al. 2013. J Immunol, 190(6): 2857-2872).

PRP6-HO7 is an homooligomer of seven recombinant polypeptides, each of them formed by the CCP6 domain of C4BP(β⁻) fused to its C-terminus to the full oligomerization domain of C4BP(β⁻) which was engineered by the inventors. The sequence of the immature form of the recombinant polypeptide forming PRP6-HO7 is SEQ ID NO: 7 and is shown in Figure 1A. The immature form of the recombinant polypeptide forming PRP6-HO7 also contains the signal peptide of C4BP, which is eliminated in the mature form. The sequence of the mature form of PRP6-HO7 is SEQ ID NO: 6 and has a theoretical pI of 5.81 and a theoretical Mw of 14,287.98 Da. Sequence SEQ ID NO: 6 is shown below wherein the first 6 amino acids are underlined and correspond to a histidine tag, followed by the sequence of the CCP6 domain of C4BP(β⁻) that is fused to the full oligomerization domain of C4BP(β⁻) which is shown double underlined.

HHHHHHLCCPEPKLNNGEITQHRKSRPANHCVYFYGDEISFSCHETSRFSAICQG

DGTWSPRTPSCGDETPEGCEQVLTGKRLMQCLPNPEDVKMALEVYKLSLEIEQ

LELQRDSARQSTLDKEL (SEQ ID NO: 6)

PRP6-NO is a recombinant polypeptide formed by the CCP6 domain of C4BP(β⁻ ) fused to its C-terminus to a mutated C4BP(β⁻) oligomerization domain unable to oligomerize by replacement of two cysteine residues by alanine and by deletion of its last 13 C-terminal amino acids (Δ537-549/C498A/C510A) (Kask et al. 2002. Biochemistry, 41:9349-9357). This recombinant polypeptide was engineered by the inventors. The sequence of the immature form of PRP6-NO is SEQ ID NO: 10 and is shown in Figure 1B. The immature form of PRP6-NO also contains the signal peptide of C4BP, which is eliminated in the mature form. The sequence of the mature form of PRP6-NO is SEQ ID NO: 8 and has a theoretical pI of 5.80 and a theoretical Mw of 12,723.23 Da. Sequence SEQ ID NO: 8 is shown below wherein the first 6 amino acids are underlined and correspond to a histidine tag, followed by the sequence of the CCP6 domain of C4BP(β⁻) that is fused to a truncated mutant of the oligomerization domain of C4BP(β⁻) which is shown double underlined. The two residues of the oligomerization domain mutated to alanine are shown in bold. 
MHPPKTPSGALHRKRKMAAWPFSRLWKVSDPILFQMTLIAALLPAVLGHHHH
HHLCCPEPKLNNGEITQHRKSRPANHCVYFYGDEISFSCHETSRFSAICQGDGT
WSPRTPSCGDETPEGAEQVLTGKRLMQALPNPEDVKMALEVYKLSLEIEQLEL
Q (SEQ ID NO: 10)
 In a preferred embodiment, the polynucleotide encoding SEQ ID NO: 10 is SEQ ID NO: 11.

HHHHHHLCCPEPKLNNGEITQHRKSRPANHCVYFYGDEISFSCHETSRFSAICQG
DGTWSPRTPSCGDETPEGAEQVLTGKRLMQALPNPEDVKMALEVYKLSLEIEQ
LELQ (SEQ ID NO: 8)

A six-histidine tag has been added to the N-terminus of PRP6-HO7 and PRP6-NO to improve purification and detection. Their production have been confirmed by SDS-PAGE, and by peptide mapping analysis (mass spectrometry).

PRP-HE8 (C4BP(β⁺)) and PRP-HO7 (C4BP(β⁻)) purification from pooled human plasma supplied by the local blood bank involved BaCl₂ precipitation (Dahlbäck. (1983) Biochem. J. 209: 847-856; Blom et al. (2009) Ann. Rheum. Dis. 68: 136-142), followed by four sequential chromatography steps including heparin chromatography, hydrophobic interaction (butyl) chromatography, anion exchange (Q Sepharose) chromatography and, finally, size exclusion (Superdex) chromatography. Both plasma-purified PRP-HE8 and PRP-HO7 were concentrated, dialyzed and recovered in PBS buffer, pH 7.4. The purity of both glycoproteins was higher than 85%, as assessed by Tris-Acetate 3-8% SDS-PAGE (NuPAGE precast protein gels; ThermoFisher, Waltham, MA, USA) from 6 µg protein/lane, and further Coomassie Blue staining.

PRP6-HO7 and PRP6-NO DNAs (SEQ ID NOs: 9 and 11, respectively) were cloned into pCDNA3.1(+) expression vector. Plasmid DNAs were amplified and purified using a Qiafilter Plasmid MegaKit, Qiagen. Expi293 cells were grown in suspension cultures until reaching the desired volume at a cell density of 2.5x10⁶ cells/ml and 98% viability. Cells were transiently transfected with 1 mg DNA per liter of culture and then, incubated for 7 days at 37°C, shaking at 125 rpm, with 8% CO₂ supply. Finally, culture media was collected and centrifuged 30 min, 6000g at 4°C. Supernatant was used for protein purification by nickel affinity chromatography (HisTrap FF) according to standard procedures. Both recombinant polypeptides were concentrated, dialyzed and recovered in PBS buffer, pH 7.4. Its purity was assessed by SDS-PAGE and Coomassie Blue staining.

ATGCACCCCCAAAAACTCCATCTGGGGCTCTTCATAGAAAAGGAAAATG
GCAGCCTGGCCCTTCTCCAGGCTGTGGAAAGTCTCTGATCCAATTCTCTTCC
AAATGACCTTGATCGCTGCTCTGTTGCCTGCTGTTCTTGGCCACCACCACCA
CCACCACTTATGTTGCCCTGAACCAAAGCTAAATAATGGTGAAATCACTCA
ACACAGGAAAAGTCGTCCTGCCAATCACTGTGTTTATTTCTATGGAGATGAG
ATTTCATTTTCATGTCATGAGACCAGTAGGTTTTCAGCTATATGCCAAGGAG
ATGGCACGTGGAGTCCCCGAACACCATCATGTGGAGACGAGACCCCCGAAG
GCTGTGAACAAGTGCTCACAGGCAAAAGACTCATGCAGTGTCTCCCAAACC
CAGAGGATGTGAAAATGGCCCTGGAGGTATATAAGCTGTCTCTGGAAATTG
AACAACTGGAACTACAGAGAGACAGCGCAAGACAATCCACTTTGGATAAA
GAACTATAA (SEQ ID NO: 9)

ATGCACCCCCAAAAACTCCATCTGGGGCTCTTCATAGAAAAGGAAAATG
GCAGCCTGGCCCTTCTCCAGGCTGTGGAAAGTCTCTGATCCAATTCTCTTCC
AAATGACCTTGATCGCTGCTCTGTTGCCTGCTGTTCTTGGCCACCACCACCA
CCACCACTTATGTTGCCCTGAACCAAAGCTAAATAATGGTGAAATCACTCA
ACACAGGAAAAGTCGTCCTGCCAATCACTGTGTTTATTTCTATGGAGATGAG
ATTTCATTTTCATGTCATGAGACCAGTAGGTTTTCAGCTATATGCCAAGGAG
ATGGCACGTGGAGTCCCCGAACACCATCATGTGGAGACGAGACCCCCGAAG
GCGCTGAACAAGTGCTCACAGGCAAAAGACTCATGCAGGCTCTCCCAAACC
CAGAGGATGTGAAAATGGCCCTGGAGGTATATAAGCTGTCTCTGGAAATTG
AACAACTGGAACTACAGTAA (SEQ ID NO: 11)

### Cell culture and PRP-based treatment

Total blood from healthy donors was acquired in the Blood and Tissue Bank (Barcelona, Spain) and their PBMCs were isolated after Ficoll-Paque density centrifugation (GE Healthcare Bio-Sciences AB, Uppsala, Sweden). Lupus nephritis patients experiencing a flare and derived to the Nephrology Unit from Bellvitge University Hospital underwent blood extraction at hospital admission, and their PBMCs were obtained as described for healthy donors. This study was approved by the IDIBELL's ethics committee in accordance with institutional guidelines and the Declaration of Helsinki, and the patients' written informed consent was obtained.

Monocytes (Mo) were purified using colloidal super-paramagnetic microbeads conjugated with monoclonal mouse anti-human CD14 antibodies (MACS, Miltenyi Biotec, Auburn, CA, or EasySep® Human CD14 Positive Selection Kit, StemCell Technologies, Grenoble, France), and counted using Perfect Count microspheres (Cytognos SL, Salamanca, Spain). The purity of CD14+ cells was tested by CD14 staining and flow cytometry analysis (FACSCanto II flow cytometer equipped with FACSDiva software (Becton Dickinson, Franklin Lakes, NJ)), allowing the assessment of the number of PBMCs and the number of total CD14+ cells (> 90% CD14+).

Monocytes were plated at 1x10⁶ cells/500 µl in 24 well culture plates (Jet Biofil, Guangzhou, China), in RPMI 1640 (Gibco, ThermoFisher, Waltham, MA, USA) supplemented with 100 mg/ml streptomycin, 100 IU/ml penicillin, 2 mM L-glutamine (all from Invitrogen, Carlsbad, CA) and 10% heat-inactivated FBS (Life technologies, ThermoFischer, Carlsbad, CA, USA) (complete medium) at 37°C under 5% CO₂. Monocyte-derived DCs (MoDCs) were generated supplementing the monocyte cultures with complete RPMI 1640 medium plus GM-CSF (800 IU/ml) and IL-4 (500 IU/ml) both from Gentaur, Kampenhout, Belgium. Monocyte-derived uncommitted macrophages (M0) were generated by incubation with GM-CSF (50 ng/ml) (Gentaur) or M-CSF (50 ng/ml) (MACS, Miltenyi Biotec, Auburn, CA) for 6 days.

PRP-based proteins were added at day 0 to differentiating monocytes at the indicated concentrations. For DC maturation iDCs, either untreated or treated with the PRP-based molecules, were further stimulated for 48 h with 5 µg/ml LPS (*Escherichia coli* 055.B5, Sigma Aldrich, Merck, Darmstadt, Germany) or 10 µg/ml Gardiquimod (Imidazoquinoline compound; TLR7 ligand) (Invivogen, San Diego, CA) at day 5. For M1 (classically activated) and M2 (alternatively activated) macrophage polarization, M0 cells at day 6 were further incubated with LPS (40 ng/ml) (Sigma-Aldrich) + γ-IFN (40 ng/ml) (Invitrogen, Carlsbad, CA) (M1), or with IL-4 (40 ng/ml) (Invitrogen) (M2) for 2 days.

Moreover, to assess the influence of human serum in PRP-HO7 and PRP6-HO7 immunomodulatory activity, human MoDCs were treated with the respective PRP-based proteins at the indicated concentrations, and co-incubated with 50% of heat-inactivated human serum (56°C, 1h) through their differentiation and maturation process.

### Abs and flow cytometry

Cell-surface phenotypes were analyzed using the following mAbs: FITC-conjugated anti-HLA-DR (Immu-357), FITC-conjugated anti-CD83 (HB15a) (Becton Dickinson, Franklin Lakes, NJ), FITC-conjugated anti-CD14 (TÜK4), PE-conjugated anti-CD40 (HB14), PE-conjugated anti-CD80 (2D10), PE-conjugated anti-CD86 (FM95), PE-conjugated anti-CDllb (M1/70.15.11.5), APC-conjugated anti-CD64 (10.1.1) and APC-conjugated anti-CD209 (REA617) (Miltenyi Biotec, Bergisch Gladbach, Germany), and Alexa Fluor 488-conjugated anti-CCR7 (G043H7) (Biolegend, San Diego, CA). FITC-conjugated anti-IgG1 (IS5-21F5), FITC-conjugated anti-IgG2b (IS6-11E5.11), FITC-conjugated anti-IgG2a (S43.10), PE-conjugated anti-IgG2b (IS6-11E5.11) and APC-conjugated IgG1 (IS5-21F5) (Miltenyi Biotec), PE-conjugated anti-IgG1 (PPV-06) (EuroBioSciences GmbH, Friesoythe, Germany), and Alexa Fluor 488-conjugated anti-IgG2aκ (MOPC-173) (Biolegend) were used as the respective isotype controls.

After washing with PBS, cells were subsequently stained with 3 µl MoAb/10⁵ cells in 100 ml FACS buffer (PBS containing 1% BSA and 0.1% sodium azide) for 15 min at room temperature. To exclude debris, we gated the cells according to forward scatter (FSC) and side scatter (SSC) parameters. Staining with 7-aminoactinomycin D (ThermoFischer, Carlsbad, CA) was also employed to assess their viability status. Stained cells were analyzed using a FACSCanto II flow cytometer (Becton Dickinson). Subsequent analyses used FlowJo software (Flowjo LLC, Ashland, OR).

### Endocytic activity

To measure endocytosis of iDCs, 2x10⁵ cells/ml were resuspended in 100 µl of PBS and incubated with 4 µl of BODIPY FL-conjugated DQ-Ovalbumin (1 mg/ml, DQ-OVA, Molecular Probes, Leiden, Netherlands) at 37°C or at 0°C for 30 min (receptor-dependent endocytosis). The incubations were stopped by adding 1 ml of cold FACS buffer. The cells were washed two times with cold FACS buffer and their fluorescence analyzed using flow cytometry.

### Chemotaxis

DCs differentiated and matured (LPS for 48 h) in presence of PRP-HE8, PRP-HO7 or PRP6-HO7, were tested for migration toward the CCL21 chemokine using transwell assays. Briefly, the lower chambers of transwell plates (polycarbonate filters of 8.0 µm pore size; Costar, Corning, NY) were filled with 400 µl of complete RPMI 1640 medium with or without CCL21 (200 ng/ml). A total of 1.6x10⁴ DCs in 100 µl of complete RPMI 1640 medium were added into the upper chamber, and cells were incubated at 37°C for 2 h. Cells migrated into the lower chambers were harvested and counted with a flow cytometer, acquiring events for a fixed time period of 1.5 min. The migration assays for all stimulation conditions were performed in duplicate wells. Values are given as total number of migrated cells.

### Differential gene expression analysis

Total RNA from untreated and PRP-HE8-, PRP-HO7-, and PRP6-HO7-treated iDCs / M0 macrophages was extracted using the RNeasy Mini Kit (Qiagen, Hilden, Germany). Reverse transcription was performed using the High-capacity cDNA archive kit (Applied BioSystems, Carlsbad, CA). Selected gene transcripts were analyzed in individual samples by RT-qPCR using the corresponding inventoried TaqMan Gene Expression Assays (Applied BioSystems). Quantification was achieved through the ΔΔCt method. A relative fold change in mRNA abundance was calculated with the equation 2^{-ΔΔCt}, employing PPIA as endogenous reference transcript.

### DC cytokine secretion

Concentrations of human IL-12p70 and TNF-α were determined from DC supernatants treated with the C4BP isoforms or PRP-based proteins using the respective DuoSet Elisa kits (R&D Systems, Minneapolis, MN, USA) according to the manufacturer's instructions.

### SDS-PAGE and Western Blot analysis

PRP-based proteins were resolved by 12% SDS-PAGE under reducing and non-reducing conditions. Gels were immersed in Blue Safe (NZYTech, Lisboa, Portugal) during 15 min and destained in water.

For Western blot analysis, PRP-based proteins were resolved on 4-12% gradient SDS-PAGE (NuPAGE Bis-Tris 4-12% Mini Gels, Invitrogen) under reducing and non-reducing conditions and transferred to PVDF membrane. After blocking, the membrane was probed with primary antibodies: 1:2000 dil. polyclonal PK9008 rabbit anti-C4BP (a gift from Anna Blom), and 1:4000 dil. 6xHis Monoclonal Antibody (Clontech, Mountain View, CA, USA), followed by the addition of 1:2000 dil. polyclonal Goat Anti-Rabbit IgG HRP (P0448, Dako, Agilent, Santa Clara, CA, USA), and 1:2000 dil. polyclonal Goat Anti-Mouse IgG HRP (P0447, Dako, Agilent, Santa Clara, CA, USA), respectively. Detection was performed with enhanced chemiluminescence (ECL) (Pierce, ThermoFischer, Carlsbad, CA, USA) using ChemiDoc Imager (Bio-Rad, Hercules, CA, USA).

### C4b cofactor activity assay

Complement C4b (8.9 µg/ml) and Factor I (4.4 µg/ml) (Merck, Darmstadt, Germany) were mixed with C4BP isoforms (0.6 nM or 6 nM) in low-salt buffer (25 mM phosphate buffer pH 7.4 and 25 mM NaCl) in a total volume of 60 µl and incubated at 37°C for 30 min. Reducing SDS sample buffer was added, C4b fragments were separated on a 4-12% gradient SDS-PAGE (NuPAGE Bis-Tris 4-12% Mini Gels, Invitrogen, ThermoFischer, Carlsbad, CA, USA), and Western blot analysis of C4b fragments was performed using a 1:2000 dilution of Anti-Human C4d MoAb (Quidel, San Diego, CA, USA) followed by a 1:2000 dilution of polyclonal Goat Anti-Mouse IgG HRP (Dako, Agilent technologies, Santa Clara, CA, USA), in TBS-Tween 0.05%, 1% BSA, 0.02% NaN₃.

### Cytokine array

A representative sample from untreated, PRP-HO7-, and PRP6-HO7-treated M1 macrophages (100 µl cell supernatants) was analyzed using the Proteome Profiler Array "Human Cytokine Array Panel A" kit (R&D Systems), according to the manufacturer's instructions, to determine the relative levels of 36 cytokines, chemokines and acute phase proteins. The density of each dot was quantified with Quantity One® software and displayed as normalized mean pixel density for each relevant cytokine and treatment.

### Statistical analysis

Statistical analyses and scientific graphing were performed using the GraphPad Prism 5 software (GraphPad software, Inc, La Jolla, CA). Repeated measures one-way ANOVA, corrected for multiple comparisons using the Dunnett's method was performed to contrast MFI and cell number data under different experimental conditions with respect to a reference condition. To compare cytokine levels, the paired t test was employed. Relative gene expression levels between treated and untreated cells were analyzed using one-sample t-test. Two-way ANOVA, corrected for multiple comparisons using the Holm-Sidak method, was applied to evaluate the time course experiments. Data are expressed as mean values + SD. In all cases, a P value < 0.05 was considered significant.

### EXAMPLE 1: Physico-chemical features of PRP6-HO7

PRP6-HO7 is derived from the physiological protein PRP-HO7, the minor isoform of the soluble complement regulator human C4BP, by joining the CCP6 domain of its α-chain with its oligomerization domain in a spatial arrangement analogous to PRP-HO7 (**Figures 1** **and** **2**). This confers this novel recombinant protein a reduced size (100 kDa compact symmetrical unit composed by 7 CCP6 domain chains joined in their C-terminus to the C4BP oligomerization domain and forming a radial spider-like homooligomer, smaller than an antibody) and similar or even superior stability (enhanced plasma half-life) than antibodies in body fluids; and like IgM- and IgA-type antibodies, allowing a cooperative, high avidity interaction with its potential surface receptors with a Kd in the low nanomolar range (data not shown) that probably involves nanoclustering of specific receptor(s) for immunomodulatory signaling. The C-terminal oligomerization scaffold is stabilized by intermolecular disulphide bonds and by layers of electrostatic interactions that contribute to the extraordinary thermodynamic stability of the complex (Hofmeyer et al. (2013) J. Mol. Biol. 425: 1302-1317). Furthermore, PRP6-HO7, contrary to its precursor PRP-HO7, is not glycosylated, which has allowed its production and further purification in a variety of expression systems, ranging from eukaryotic cells (shown below), to yeast, or bacterial cells (data not shown).

Finally, because of its simplified structure including only the CCP6 domain, PRP6-HO7 administration would not increase infection susceptibility compared to PRP-HO7. PRP-HO7 α-chains bind to surface proteins from a variety of bacterial pathogens (Blom et al. (2004) Mol. Immunol. 40: 1333-1346), which hijack host's complement regulators and subsequently down regulate complement activation. Thus, an essential aspect of the fact that PRP-HO7 binds to pathogens (mainly through CCP1 to CCP4) (Blom et al. (2004) Mol. Immunol. 40: 1333-1346) and to plasminogen (through CCP8) (Agarwal et al. (2015) J. Biol. Chem. 290: 18333-18342) as bacterial virulence factor is that one has to consider it while planning to use complement inhibitors as immunomodulatory agents for autoimmune diseases.

In this regard, and to assess whether the C4BP CCP6 domain alone as a monomer or, conversely, in an heptavalent oligomerized form analogous to its physiological origin, would preserve the anti-inflammatory and immunomodulatory activities ascribed to PRP-HO7 (Olivar et al. (2013) J. Immunol. 190: 2857-2872), PRP6-HO7 and PRP6-NO were produced in HEK293 cells and were purified from its supernatants (**Figure 3**). As expected and shown through PAGE and both Coomassie Blue staining and Western blot analysis, PRP6-HO7 folded in a homooligomeric 100 kDa structure consisting of 7 monomer chains of 14.3 kDa, which included the CCP6 domain and the C-terminal oligomerization domain of C4BP. On the other hand, PRP6-NO was not able to oligomerize under non-reducing conditions and remained as a single monomer of 12.7 kDa because of the substitution of two essential folding Cys residues (C498A/C510A) and truncation of the 13 C-terminal amino acids from the C-terminus of the C4BP oligomerization domain (Kask et al. (2002) Biochemistry 41: 9349-9357).

### EXAMPLE 2: PRP6-HO7 down-regulates the activation phenotype of DCs

Both PRP-HE8 and PRP6-NO had no effect on the expression of the DC activation marker CD83 and the DC co-stimulatory molecule CD86 on LPS-matured DCs. Conversely, PRP6-HO7, analogously to PRP-HO7, significantly down-regulated these markers (**Figure 4**).

On the other hand, the antigen internalization capacity of DCs was assessed by flow cytometry of both self-quenching DQ-OVA (mannose receptor-mediated endocytosis marker). Thus, the endocytic activity of iDCs was significantly increased by treatment with either PRP-HO7 or PRP6-HO7 (**Figure 5**) but neither by monomeric PRP6-NO nor by the inactive molecule PRP-HE8.

The inventors next assessed whether the effect of the different PRP proteins on the DC phenotype was accompanied by changes in their release of cytokines (TNF-α and IL-12p70). Compared to untreated iDCs, secretion of each of the pro-inflammatory cytokines was up-regulated when iDCs were matured with LPS. DCs pre-treated with both PRP-HE8 and PRP6-NO secreted the same cytokine levels than untreated DCs upon maturation. In contrast, pre-treatment with both PRP-HO7 and PRP6-HO7 prevented the release of IL-12p70 and significantly decreased the release of TNF-α (**Figure 6**). Thus, Th1 pro-inflammatory cytokine production upon LPS-mediated DC stimulation was significantly diminished by PRP6-HO7-treated DCs.

DCs treated with the different PRP molecules remained highly viable throughout the differentiation/maturation process, as assessed by annexin V / 7-ADD staining, with less than 10% of apoptotic cells evidenced at 48 h after LPS-mediated DC maturation (data not shown).

Together, these data are evidence that oligomeric PRP6-HO7, but not monomeric PRP6-NO, has the potential to modify pro-inflammatory DC differentiation/maturation towards an anti-inflammatory and tolerogenic phenotype.

### EXAMPLE 3: PRP6-HO7 lacks complement inhibitory activity

One of the major regulatory functions of C4BP is to serve as a cofactor for the serine protease factor I to inactivate C4b, also termed cofactor activity. To confirm that PRP6-HO7 effectively was devoid of complement inhibitory activity, assigned to the CCP1-CCP3 domains of the PRP-HO7 α-chain, the inventors performed a comparative C4BP cofactor activity assay assessing its contribution on Factor I-mediated cleavage of C4b in solution. Thus, both PRP-HE8 and PRP-HO7, but not PRP6-HO7 at any of the concentrations tested, were able to accelerate Factor I cleavage of the α'-chain of C4b yielding the 70 kDa partial cleavage fragment, α3-C4d, and the small fragment C4d (45 kDa) (**Figure 7**).

### EXAMPLE 4: PRP6-HO7 affects the expression of surface activation markers on DCs

The inventors assessed whether PRP6-HO7 was able to influence the expression of different monocyte and DC surface markers, including CD14, HLA-DR, CD40, CD80, CD83, and CD86. While PRP-HE8 had no effect on the expression of any of the above markers on LPS-matured DCs, both PRP-HO7 and PRP6-HO7 significantly down-regulated not only CD83 and CD86, as previously stated, but also the co-stimulatory molecules CD80 and CD40. Conversely, expression of HLA-DR and CD14 on DCs was not significantly altered by both PRP-HO7 and PRP6-HO7 treatment (**Figure 8**).

These data confirm that PRP6-HO7 has the potential to modify pro-inflammatory DC differentiation/maturation as judged by the expression pattern of various cell surface markers. In contrast, neither PRP6-HO7 alone nor PRP6-HO7 plus LPS incubated from day 5 to day 7 (maturation) had any effect on DC surface marker expression, i.e., when DC are differentiated (data not shown).

### EXAMPLE 5: Human serum does not interfere with the PRP6-HO7 immunomodulatory activity

To infer the behavior of PRP6-HO7 in a more complex environment the inventors analyzed CD83 and CD86 surface marker expression on DCs differentiated and LPS-matured in presence of 50% human serum. Analogously to PRP-HO7, PRP6-HO7 was able to significantly downregulate the above markers in a dose-dependent manner (**Figure 9**). Thus, under near physiological conditions, PRP6-HO7 was at least as active as its physiological counterpart PRP-HO7 regarding immunomodulation.

### EXAMPLE 6: PRP6-HO7 modulates the expression of TLRs on DCs from both normal individuals and lupus nephritis patients

An analogous cell surface marker behavior that in DCs incubated with the TLR4 ligand LPS (e.g., significant CD83 and CD86 downregulation) was observed when both PRP-HO7- and PRP6-HO7-treated DCs where matured by gardiquimod exposure (a TLR7 analogue) (**Figure 10**). TLR7 has a critical role in the acceleration of disease in systemic lupus erythematosus (SLE). Importantly, the same trend was perceived using DCs isolated from SLE patients experiencing a lupus nephritis flare, although for some DC markers statistical significance could not be achieved due to the limited number of samples available (**Figure 11**). Together, these data suggest that PRP6-HO7 is active regulating the expression of TLR4 and TLR7 from both normal individuals and SLE patients with active disease.

### EXAMPLE 7: PRP6-HO7 alters the chemotaxis of DCs

Maturation signals determine the expression of distinct DC functions, such as migration towards lymph node-directing chemokines. Both PRP-HO7 and PRP6-HO7 treatments down-regulated the expression of the chemokine receptor CCR7. Reduced surface CCR7 expression, in turn, significantly decreased the migration of LPS-matured DCs towards the chemokine CCL21 (**Figure 12**). In contrast, LPS maturation of both untreated and PRP-HE8-treated DCs induced maximal migration in response to CCL21.

### EXAMPLE 8: DCs exposed to the PRP6-HO7 up-regulate "find me" receptors and down-regulate PTGER3 and EGLN3 at the transcriptional level

To further assess the effect of PRP6-H07 on several key transcripts conforming the molecular signature of inflammatory monocyte-derived iDCs, the inventors analyzed by RT-qPCR the expression of the genes coding for the PGE₂ receptor (EP3; PTGER3), an important mediator of acute inflammation (Kawahara et al. (2015) Biochim. Biophys. Acta 1851: 414-421); and for prolyl-hydroxylase 3 (EGLN3; PHD3). It has been recently shown that loss of PHD3 in myeloid cells dampens the inflammatory response (Beneke et al. (2017) Cell Death Dis. 8: e2976). Accordingly, both *PTGER3* and *EGLN3* transcripts were preferentially downregulated by PRP6-HO7 (**Figure 13**). In contrast, a transcriptional profiling of all known "find me" receptors in iDCs revealed significant upregulation of *FPR2, P2RY2* and *S1PR1* under PRP6-HO7, but not PRP-HO7, treatment, suggesting increased efferocytosis capacity mediating a tolerant response from PRP6-HO7-treated DCs (Kolb et al. (2017) Trends Immunol. 38: 705-718) (**Figure 14**).

### EXAMPLE 9: PRP6-HO7 down-regulates the polarization phenotype of monocyte-derived macrophages

Macrophages are widely distributed innate immune cells that play central roles in host defense against invading pathogens and in maintaining the immune homeostasis, contributing to inflammation, and promoting wound healing and tissue repair. These are dynamic cells that mature under the influence of signals from the local microenvironment.

The inventors differentiated human peripheral blood monocytes into uncommitted macrophages (M0) and then polarized them to M1 (classically activated macrophages) using LPS and IFN-γ, and to M2 (alternatively activated macrophages) using IL-4. M1 and M2 macrophages are identified by flow cytometry as CD80⁺ CD64⁺ and CD11b⁺ CD209⁺, respectively. PRP-HO7 and, particularly, PRP6-HO7 preincubation were able to significantly downregulate the expression of both M1 (CD80 and CD64) and M2 (CD11b and CD209) polarization markers (**Figure 15**). Thus PRP6-HO7, and in a lesser extent PRP-HO7, but not PRP-HE8 immunomodulatory activity prevents macrophage polarization/activation towards M1 or M2 phenotypes, maintaining macrophages in an uncommitted-like phenotype.

### EXAMPLE 10: PRP6-HO7 up-regulates "find me" receptors and down-regulates ALCAM, SLC16A1 and LMNB1 expression in M0 uncommitted macrophages

Analogously than for DCs, to inquire the effect of PRP6-HO7 on several key transcripts conforming the molecular signature of differentiating monocyte-derived macrophages towards M0, the inventors performed a time-course assay sequentially analyzing by RT-qPCR the expression of the genes coding for activated leukocyte cell adhesion molecule (ALCAM; CD166), which is upregulated in activated monocytes (Levesque et al. (1998) Arthritis Rheum. 41: 2221-2229); SLC16A1, a lactate transporter that is induced by autophagy (Bao et al. (2017) Theriogenology 87: 339-348); and LMNB1, an important component of the nuclear lamina that interacts with the nuclear autophagy protein LC3 (Dou et al. (2015) Nature 527: 105-109). These 3 transcripts were found significantly downregulated trough the monocyte-to-macrophage differentiation process mainly by PRP6-HO7 action (**Figure 16**). Thus, downregulation of both SLC16A1 and LMNB1 suggests a block in autophagy mediated by PRP6-HO7. In contrast, transcriptional profiling of all known "find me" or *smell* receptors in M0 macrophages revealed significant upregulation of *CCR2, CX3CR1, CXCR1, FPR2* and *P2RY2* under PRP6-HO7, but not PRP-HO7 or PRP-HE8, treatment (**Figure 17**). A recent study revealed another interaction between the pathways leading to autophagy and phagocytosis. ATG7-deficient macrophages were found to have increased levels of class A scavenger receptors because of the accumulation of p62 (Cadwell and Philips (2013) Immunity 39: 425-427). The upregulation of these receptors led to higher phagocytic uptake rates and increased bacterial uptake revealing that the loss of autophagy can enhance phagocytosis (Bonilla et al. (2013) Immunity 39: 537-547).

### EXAMPLE 11: PRP6-HO7 modulates the cytokine signature of M1 inflammatory macrophages

Finally, the inventors examined the induction of cytokines in the cell supernatant upon macrophage polarization from M0 to M1, and the consequences of PRP-H07 and PRP6-HO7 treatment on the inflammatory cytokine profile released by the M1 macrophages. Remarkably, a cytokine array including 36 cytokines, chemokines and acute phase proteins evidenced differential downregulation of CCL1, CCL2, MIP-1, CCL5, CXCL1, IL-6 and TNF-α through PRP-HO7 and PRP6-HO7 action respect to untreated M1 cells. Moreover, CCL1 and MIP-1 evidenced stronger downregulation under the presence of PRP6-HO7 (**Figure 18**). Thus, upregulation of these inflammatory chemokines upon M1 macrophage polarization (Sokol and Luster (2015) Cold Spring Harb. Perspect. Biol. 7: a016303) is prevented by PRP6-HO7 treatment and, in a lesser extent, by PRP-HO7 treatment.

### EXAMPLE 12: Yeast purification of PRP6-HO7

The corresponding DNA sequence of PRP6-HO7 was codon-optimized for *P. pastoris.* The synthetic gene was cloned in pBGZα vector (Bioingenium, Barcelona Science Park, Baldiri Reixac, 15-21 (Helix building) 08028 Barcelona) using BsaI cloning sites. The expression vector pBGZα contained the GAP promoter and the AOX transcription terminator. Moreover, it encoded a zeocin resistance cassette for clone selection, flanked by loxP sites for future resistant phenotype reversion if required. Furthermore, the vector had an ampicillin resistance gene and a pUC replication origin, for plasmid selection and propagation in *E. coli,* respectively. Finally, the α-mating factor secretion signal of *S.cerevisiae* between the promoter and the gene coding sequence, allowed addressing the protein expression towards the secretory pathway.

Aiming to avoid the use of methanol in an eventual production process at industrial scale, it was decided to control the gene expression under the constitutive GAP promoter. Taking advantage of the *P. pastoris* ability to secrete heterologous proteins, the genes of interest were cloned in frame with the α-mating factor of *S.cerevisiae* (SEQ ID NO: 62).

MRFPSIFTAVLFAASSALAAPVNTTTEDETAQIPAEAVIGYLDLEGDFDVAVLPF
SNSTNNGLLFINTTIASIAAKEEGVSLDKREAEA (SEQ ID NO: 62)

The sequence of the recombinant polypeptide including the signal peptide was SEQ ID NO: 63.

MRFPSIFTAVLFAASSALAAPVNTTTEDETAQIPAEAVIGYLDLEGDFDVAVLPF
SNSTNNGLLFINTTIASIAAKEEGVSLDKREAEAHHHHHHLCCPEPKLNNGEITQ
HRKSRPANHCVYFYGDEISFSCHETSRFSAICQGDGTWSPRTPSCGDETPEGCEQ
VLTGKRLMQCLPNPEDVKMALEVYKLSLEIEQLELQRDSARQSTLDKEL (SEQ
ID NO: 63)

Then, the yeast was cultured and the protein was purified from *Pichia pastoris.* Cultivation parameters are shown in Table 4. Figure 19 shows visualization of the purified protein.

**Table 4. Cultivation parameters**

| | |
|---|---|
| **Bioreactor** | Applikon 5 L |
| **Strain** | *P. pastoris* NRRLY-11430 pBGZα_PRP6-HO7 G9 |
| **Medium** | Fermentation Basal Salts Medium (as described in "Pichia Fermentation Process Guidelines" by Invitrogen) |
| **pH control (base/acid)** | 28% Ammonia / 4 M ortho-phosphoric acid |
| **Antifoam** | Antifoam 204, 1 % v/v solution |
| **Cultivation strategy** | Glucose fed-batch (exponential feeding rate, constant µ) |
| **DO control (*DO setpoint*/control)** | Glycerol batch: 25% (Cascade. 1st: 800 to 1100 rpm, 1 vvm air. 2nd: pure O2, 1 L/min) Glycerol fed-batch: 25% (Cascade, same as batch phase) |
| **Temperature** | Glycerol batch: 28 °C Glycerol fed-batch: 28 °C |
| **pH** | 4.0 |
| **Inoculum preparation** | Seeding from agar plate 300 mL of BMGY (in 2x150 mL in 1L shake flasks) Overnight, 200 rpm, 28 °C |
| **Sampling** | 15 h, 18 h, 20 h, 23 h, 37 h, 40.5 h, 43.5 h, 46 h |
| **Processing of final broth** | Centrifugation at 6000 g, 30 min, 4 °C Supernatants stored at -20 °C |

### EXAMPLE 13: Bacterial production of PRP6-HO7

PRP6-HO7 nucleotide sequence was codon-optimized for *E.coli* expression, subcloned in pUC57 plasmid and cloned into the expression vector pET32a. The sequence SEQ ID NO: 61 was expressed in strain BL-21(D3). Figure 20 shows visualization of the purified protein.

## Claims

1. A recombinant polypeptide comprising the CCP6 domain of the C4BP alpha chain or a functionally equivalent variant thereof and an oligomerization domain, wherein said polypeptide does not comprise CCP1, CCP2, CCP3, CCP4, CCP5, CCP7 nor CCP8 domains of the C4BP alpha chain.

2. The recombinant polypeptide according to claim 1, wherein the CCP6 domain of the C4BP alpha chain is SEQ ID NO: 1.

3. The recombinant polypeptide according to any one of claims 1 or 2, wherein the oligomerization domain is SEQ ID NO: 2 or a functionally equivalent variant thereof, preferably SEQ ID NO: 2.

4. The recombinant polypeptide according to any one of claims 1 to 3, wherein the polypeptide consists of SEQ ID NO: 3 or a functionally equivalent variant thereof, preferably SEQ ID NO: 3.

5. The recombinant polypeptide according to any one of claims 1 to 3, wherein the polypeptide consists of a sequence selected from the group consisting of SEQ ID NO: 5, SEQ ID NO: 7 and a functionally equivalent variant thereof.

6. The recombinant polypeptide according to any one of claims 1 to 3, wherein the polypeptide consists of SEQ ID NO: 6 or a functionally equivalent variant thereof, preferably SEQ ID NO: 6.

7. An homooligomer of at least six recombinant polypeptides according to any one of claims 1 to 6.

8. The homooligomer according to claim 7, wherein the homooligomer is formed by seven recombinant polypeptides according to any one of claims 1 to 6.

9. The homooligomer according to any one of claims 7 or 8, wherein the polypeptide is the polypeptide consisting of SEQ ID NO: 6.

10. A recombinant polypeptide according to any one of claims 1 to 6, an homooligomer according to any one of claims 7 to 9, a polynucleotide encoding a recombinant polypeptide according to any one of claims 1 to 6, a vector comprising said polynucleotide, a host cell comprising said vector or a pharmaceutical composition comprising said recombinant polypeptide, said homooligomer, said polynucleotide, said vector or said host cell and a pharmaceutically acceptable carrier for use in medicine.

11. A recombinant polypeptide according to any one of claims 1 to 6, an homooligomer according to any one of claims 7 to 9, a polynucleotide encoding a recombinant polypeptide according to any one of claims 1 to 6, a vector comprising said polynucleotide, a host cell comprising said vector, or a pharmaceutical composition comprising said recombinant polypeptide, said homooligomer, said polynucleotide, said vector or said host cell and a pharmaceutically acceptable carrier for use in the prevention and/or treatment of an immunological disease caused by an undesired activation of the immune system.

12. An *in vitro* method for the generation of a population of tolerogenic dendritic cells comprising the steps of:
(i) incubating a population of dendritic precursor cells under conditions adequate for the formation of a population of immature dendritic cells, and
(ii) incubating the population of immature dendritic cells obtained in step (i) under conditions adequate for the formation of mature dendrite cells
wherein steps (i) and/or (ii) are carried out in the presence of a composition of matter selected from the group consisting of:
(a) a polypeptide according to any one of claims 1 to 6,
(b) an homooligomer according to any one of claims 7 to 9,
(c) a polynucleotide encoding a recombinant polypeptide according to any one of claims 1 to 6, and
(d) a vector comprising a polynucleotide according to (c).

13. An *in vitro* method for the generation of a population of tolerogenic macrophages comprising the steps of:
(i) incubating a population of macrophage precursor cells under conditions adequate for the formation of a population of immature macrophages, and
(ii) incubating the population of immature macrophages obtained in step (i) under conditions adequate for the formation of mature macrophages
wherein steps (i) and/or (ii) are carried out in the presence of a composition of matter selected from the group consisting of:
(a) a polypeptide according to any one of claims 1 to 6,
(b) an homooligomer according to any one of claims 7 to 9,
(c) a polynucleotide encoding a recombinant polypeptide according to any one of claims 1 to 6, and
(d) a vector comprising a polynucleotide according to (c).

14. A tolerogenic dendritic cell obtained by the method according to claim 12, or a tolerogenic macrophage obtained by the method according to claim 13, preferably the tolerogenic dendritic cell is FPR2^{high}, P2RY2^{high}, and/or S1PR1^{high}, and the macrophage is CCR2^{high}, CX3CR1^{high}, CXCR1^{high}, FPR2^{high} and/or P2RY2^{high}.

15. A tolerogenic dendritic cell or a tolerogenic macrophage according to claim 14 for use in the prevention and/or treatment of an immunological disease caused by an undesired activation of the immune system.


SEQUENCE LISTING

<110>  FUNDACIÓ INSTITUT D'INVESTIGACIÓ BIOMÈDICA DE BELLVITGE
       (IDIBELL)

<120>  COMPOUNDS FOR IMMUNOMODULATION

<130>  P18233EP00

<160>  63

<170>  PatentIn version 3.5

<210>  1
<211>  62
<212>  PRT
<213>  Homo sapiens

<400>  1

Leu Cys Cys Pro Glu Pro Lys Leu Asn Asn Gly Glu Ile Thr Gln His
1               5                   10                  15

Arg Lys Ser Arg Pro Ala Asn His Cys Val Tyr Phe Tyr Gly Asp Glu
            20                  25                  30

Ile Ser Phe Ser Cys His Glu Thr Ser Arg Phe Ser Ala Ile Cys Gln
        35                  40                  45

Gly Asp Gly Thr Trp Ser Pro Arg Thr Pro Ser Cys Gly Asp
    50                  55                  60

<210>  2
<211>  57
<212>  PRT
<213>  Homo sapiens

<400>  2

Glu Thr Pro Glu Gly Cys Glu Gln Val Leu Thr Gly Lys Arg Leu Met
1               5                   10                  15

Gln Cys Leu Pro Asn Pro Glu Asp Val Lys Met Ala Leu Glu Val Tyr
            20                  25                  30

Lys Leu Ser Leu Glu Ile Glu Gln Leu Glu Leu Gln Arg Asp Ser Ala
        35                  40                  45

Arg Gln Ser Thr Leu Asp Lys Glu Leu
    50                  55

<210>  3
<211>  119
<212>  PRT
<213>  Artificial Sequence

<220>
<223>  Recombinant polypeptide

<400>  3

Leu Cys Cys Pro Glu Pro Lys Leu Asn Asn Gly Glu Ile Thr Gln His
1               5                   10                  15

Arg Lys Ser Arg Pro Ala Asn His Cys Val Tyr Phe Tyr Gly Asp Glu
            20                  25                  30

Ile Ser Phe Ser Cys His Glu Thr Ser Arg Phe Ser Ala Ile Cys Gln
        35                  40                  45

Gly Asp Gly Thr Trp Ser Pro Arg Thr Pro Ser Cys Gly Asp Glu Thr
    50                  55                  60

Pro Glu Gly Cys Glu Gln Val Leu Thr Gly Lys Arg Leu Met Gln Cys
65                  70                  75                  80

Leu Pro Asn Pro Glu Asp Val Lys Met Ala Leu Glu Val Tyr Lys Leu
                85                  90                  95

Ser Leu Glu Ile Glu Gln Leu Glu Leu Gln Arg Asp Ser Ala Arg Gln
            100                 105                 110

Ser Thr Leu Asp Lys Glu Leu
        115


<210>  4
<211>  48
<212>  PRT
<213>  Homo sapiens

<400>  4

Met His Pro Pro Lys Thr Pro Ser Gly Ala Leu His Arg Lys Arg Lys
1               5                   10                  15

Met Ala Ala Trp Pro Phe Ser Arg Leu Trp Lys Val Ser Asp Pro Ile
            20                  25                  30

Leu Phe Gln Met Thr Leu Ile Ala Ala Leu Leu Pro Ala Val Leu Gly
        35                  40                  45


<210>  5
<211>  167
<212>  PRT
<213>  Artificial Sequence

<220>

<223>  Recombinant polypeptide

<400>  5

Met His Pro Pro Lys Thr Pro Ser Gly Ala Leu His Arg Lys Arg Lys
1               5                   10                  15

Met Ala Ala Trp Pro Phe Ser Arg Leu Trp Lys Val Ser Asp Pro Ile
            20                  25                  30

Leu Phe Gln Met Thr Leu Ile Ala Ala Leu Leu Pro Ala Val Leu Gly
        35                  40                  45

Leu Cys Cys Pro Glu Pro Lys Leu Asn Asn Gly Glu Ile Thr Gln His
    50                  55                  60

Arg Lys Ser Arg Pro Ala Asn His Cys Val Tyr Phe Tyr Gly Asp Glu
65                  70                  75                  80

Ile Ser Phe Ser Cys His Glu Thr Ser Arg Phe Ser Ala Ile Cys Gln
                85                  90                  95

Gly Asp Gly Thr Trp Ser Pro Arg Thr Pro Ser Cys Gly Asp Glu Thr
            100                 105                 110

Pro Glu Gly Cys Glu Gln Val Leu Thr Gly Lys Arg Leu Met Gln Cys
        115                 120                 125

Leu Pro Asn Pro Glu Asp Val Lys Met Ala Leu Glu Val Tyr Lys Leu
    130                 135                 140

Ser Leu Glu Ile Glu Gln Leu Glu Leu Gln Arg Asp Ser Ala Arg Gln
145                 150                 155                 160

Ser Thr Leu Asp Lys Glu Leu
                165


<210>  6
<211>  125
<212>  PRT
<213>  Artificial Sequence

<220>
<223>  Recombinant polypeptide

<400>  6

His His His His His His Leu Cys Cys Pro Glu Pro Lys Leu Asn Asn
1               5                   10                  15

Gly Glu Ile Thr Gln His Arg Lys Ser Arg Pro Ala Asn His Cys Val

20                  25                  30

Tyr Phe Tyr Gly Asp Glu Ile Ser Phe Ser Cys His Glu Thr Ser Arg
        35                  40                  45

Phe Ser Ala Ile Cys Gln Gly Asp Gly Thr Trp Ser Pro Arg Thr Pro
    50                  55                  60

Ser Cys Gly Asp Glu Thr Pro Glu Gly Cys Glu Gln Val Leu Thr Gly
65                  70                  75                  80

Lys Arg Leu Met Gln Cys Leu Pro Asn Pro Glu Asp Val Lys Met Ala
                85                  90                  95

Leu Glu Val Tyr Lys Leu Ser Leu Glu Ile Glu Gln Leu Glu Leu Gln
            100                 105                 110

Arg Asp Ser Ala Arg Gln Ser Thr Leu Asp Lys Glu Leu
        115                 120                 125

<210>  7
<211>  173
<212>  PRT
<213>  Artificial Sequence

<220>
<223>  Recombinant polypeptide

<400>  7

Met His Pro Pro Lys Thr Pro Ser Gly Ala Leu His Arg Lys Arg Lys
1               5                   10                  15

Met Ala Ala Trp Pro Phe Ser Arg Leu Trp Lys Val Ser Asp Pro Ile
            20                  25                  30

Leu Phe Gln Met Thr Leu Ile Ala Ala Leu Leu Pro Ala Val Leu Gly
        35                  40                  45

His His His His His His Leu Cys Cys Pro Glu Pro Lys Leu Asn Asn
    50                  55                  60

Gly Glu Ile Thr Gln His Arg Lys Ser Arg Pro Ala Asn His Cys Val
65                  70                  75                  80

Tyr Phe Tyr Gly Asp Glu Ile Ser Phe Ser Cys His Glu Thr Ser Arg
                85                  90                  95

Phe Ser Ala Ile Cys Gln Gly Asp Gly Thr Trp Ser Pro Arg Thr Pro
            100                 105                 110

Ser Cys Gly Asp Glu Thr Pro Glu Gly Cys Glu Gln Val Leu Thr Gly
        115                 120                 125

Lys Arg Leu Met Gln Cys Leu Pro Asn Pro Glu Asp Val Lys Met Ala
    130                 135                 140

Leu Glu Val Tyr Lys Leu Ser Leu Glu Ile Glu Gln Leu Glu Leu Gln
145                 150                 155                 160

Arg Asp Ser Ala Arg Gln Ser Thr Leu Asp Lys Glu Leu
                165                 170

<210>  8
<211>  112
<212>  PRT
<213>  Artificial Sequence

<220>
<223>  Mature form of PRP6-NO

<400>  8

His His His His His His Leu Cys Cys Pro Glu Pro Lys Leu Asn Asn
1               5                   10                  15

Gly Glu Ile Thr Gln His Arg Lys Ser Arg Pro Ala Asn His Cys Val
            20                  25                  30

Tyr Phe Tyr Gly Asp Glu Ile Ser Phe Ser Cys His Glu Thr Ser Arg
        35                  40                  45

Phe Ser Ala Ile Cys Gln Gly Asp Gly Thr Trp Ser Pro Arg Thr Pro
    50                  55                  60

Ser Cys Gly Asp Glu Thr Pro Glu Gly Ala Glu Gln Val Leu Thr Gly
65                  70                  75                  80

Lys Arg Leu Met Gln Ala Leu Pro Asn Pro Glu Asp Val Lys Met Ala
                85                  90                  95

Leu Glu Val Tyr Lys Leu Ser Leu Glu Ile Glu Gln Leu Glu Leu Gln
            100                 105                 110

<210>  9
<211>  522
<212>  DNA
<213>  Artificial Sequence

<220>
<223>  PRP6-HO7 DNA

<400>  9
atgcaccccc caaaaactcc atctggggct cttcatagaa aaaggaaaat ggcagcctgg        60

cccttctcca ggctgtggaa agtctctgat ccaattctct tccaaatgac cttgatcgct       120

gctctgttgc ctgctgttct tggccaccac caccaccacc acttatgttg ccctgaacca       180

aagctaaata atggtgaaat cactcaacac aggaaaagtc gtcctgccaa tcactgtgtt       240

tatttctatg gagatgagat ttcattttca tgtcatgaga ccagtaggtt ttcagctata       300

tgccaaggag atggcacgtg gagtccccga acaccatcat gtggagacga gacccccgaa       360

ggctgtgaac aagtgctcac aggcaaaaga ctcatgcagt gtctcccaaa cccagaggat       420

gtgaaaatgg ccctggaggt atataagctg tctctggaaa ttgaacaact ggaactacag       480

agagacagcg caagacaatc cactttggat aaagaactat aa                          522


<210>  10
<211>  160
<212>  PRT
<213>  Artificial Sequence

<220>
<223>  Immature form of PRP6-NO

<400>  10

Met His Pro Pro Lys Thr Pro Ser Gly Ala Leu His Arg Lys Arg Lys
1               5                   10                  15


Met Ala Ala Trp Pro Phe Ser Arg Leu Trp Lys Val Ser Asp Pro Ile
            20                  25                  30


Leu Phe Gln Met Thr Leu Ile Ala Ala Leu Leu Pro Ala Val Leu Gly
        35                  40                  45


His His His His His His Leu Cys Cys Pro Glu Pro Lys Leu Asn Asn
    50                  55                  60


Gly Glu Ile Thr Gln His Arg Lys Ser Arg Pro Ala Asn His Cys Val
65                  70                  75                  80


Tyr Phe Tyr Gly Asp Glu Ile Ser Phe Ser Cys His Glu Thr Ser Arg
                85                  90                  95


Phe Ser Ala Ile Cys Gln Gly Asp Gly Thr Trp Ser Pro Arg Thr Pro
            100                 105                 110


Ser Cys Gly Asp Glu Thr Pro Glu Gly Ala Glu Gln Val Leu Thr Gly
        115                 120                 125

Lys Arg Leu Met Gln Ala Leu Pro Asn Pro Glu Asp Val Lys Met Ala
    130                 135                 140

Leu Glu Val Tyr Lys Leu Ser Leu Glu Ile Glu Gln Leu Glu Leu Gln
145                 150                 155                 160

<210>  11
<211>  483
<212>  DNA
<213>  Artificial Sequence

<220>
<223>  PRP6-NO DNA

<400>  11
atgcaccccc caaaaactcc atctggggct cttcatagaa aaaggaaaat ggcagcctgg        60

cccttctcca ggctgtggaa agtctctgat ccaattctct tccaaatgac cttgatcgct       120

gctctgttgc ctgctgttct tggccaccac caccaccacc acttatgttg ccctgaacca       180

aagctaaata atggtgaaat cactcaacac aggaaaagtc gtcctgccaa tcactgtgtt       240

tatttctatg gagatgagat ttcattttca tgtcatgaga ccagtaggtt ttcagctata       300

tgccaaggag atggcacgtg gagtccccga acaccatcat gtggagacga gacccccgaa       360

ggcgctgaac aagtgctcac aggcaaaaga ctcatgcagg ctctcccaaa cccagaggat       420

gtgaaaatgg ccctggaggt atataagctg tctctggaaa ttgaacaact ggaactacag       480

taa                                                                     483

<210>  12
<211>  60
<212>  PRT
<213>  Oryctolagus cuniculus

<400>  12

Ile Cys Cys Pro Pro Pro Gln Leu Asp Asn Gly Ala Ile Thr Glu His
1               5                   10                  15

Ser Arg Asn Pro Arg Ala Asn Asn Cys Thr Tyr Phe Phe Gly Asp Ile
            20                  25                  30

Val Val Tyr Ser Cys Tyr Gln Lys Gln Arg Pro Pro Val Thr Cys Arg
        35                  40                  45

Ala Asp Gly Thr Trp Ser Pro His Thr Pro Leu Cys
    50                  55                  60

<210>  13
<211>  62
<212>  PRT
<213>  Rattus norvegicus

<400>  13

Ile Cys Cys Pro Val Pro Asp Pro Lys Ser Val Arg Val Ile Gln His
1               5                   10                  15

Glu Lys Ala His Pro Asp Asn Asp Cys Thr Tyr Phe Phe Gly Asp Glu
            20                  25                  30

Val Ser Tyr Thr Cys Gln Asn Asp Ile Met Leu Thr Ala Thr Cys Lys
        35                  40                  45

Ser Asp Gly Thr Trp His Pro Arg Thr Pro Ser Cys His Gln
    50                  55                  60

<210>  14
<211>  63
<212>  PRT
<213>  Bos taurus

<400>  14

Val Cys Cys Pro Glu Pro Asn Leu Asn Asn Tyr Gly Ser Ile Thr Leu
1               5                   10                  15

His Arg Arg Pro Ser Thr Ser Thr His Cys Thr Tyr Ile Ser Gly Asp
            20                  25                  30

Lys Ile Ser Tyr Glu Cys His Ser Lys Tyr Met Phe Asp Ala Leu Cys
        35                  40                  45

Thr Lys His Gly Thr Trp Ser Pro Arg Thr Pro Glu Cys Arg Pro
    50                  55                  60

<210>  15
<211>  54
<212>  PRT
<213>  Mus musculus

<400>  15

Glu Ala Ser Glu Asp Leu Lys Pro Ala Leu Thr Gly Asn Lys Thr Met
1               5                   10                  15

Gln Tyr Val Pro Asn Ser His Asp Val Lys Met Ala Leu Glu Ile Tyr
            20                  25                  30

Lys Leu Thr Leu Glu Val Glu Leu Leu Gln Leu Gln Ile Gln Lys Glu
        35                  40                  45

Lys His Thr Glu Ala His
    50

<210>  16
<211>  55
<212>  PRT
<213>  Rattus norvegicus

<400>  16

Glu Val Pro Lys Asp Cys Glu His Val Phe Ala Gly Lys Lys Leu Met
1               5                   10                  15

Gln Cys Leu Pro Asn Ser Asn Asp Val Lys Met Ala Leu Glu Val Tyr
            20                  25                  30

Lys Leu Thr Leu Glu Ile Lys Gln Leu Gln Leu Gln Ile Asp Lys Ala
        35                  40                  45

Lys His Val Asp Arg Glu Leu
    50                  55

<210>  17
<211>  67
<212>  PRT
<213>  Bos taurus

<400>  17

Glu Tyr Pro Glu Gly Cys Glu Gln Val Val Thr Gly Arg Lys Leu Leu
1               5                   10                  15

Gln Cys Leu Ser Arg Pro Glu Glu Val Lys Leu Ala Leu Glu Val Tyr
            20                  25                  30

Lys Leu Ser Leu Glu Ile Glu Ile Leu Gln Thr Asn Lys Leu Lys Lys
        35                  40                  45

Glu Ala Phe Leu Leu Arg Glu Arg Glu Lys Asn Val Thr Cys Asp Phe
    50                  55                  60

Asn Pro Glu
65

<210>  18
<211>  57
<212>  PRT
<213>  Artificial Sequence

<220>
<223>  Oligomerization domain of C4BP alpha chain of mammalilan ortholog

<400>  18

Glu Thr Pro Glu Gly Cys Glu Gln Val Leu Thr Gly Lys Arg Leu Met

1               5                   10                  15

Gln Cys Leu Pro Asn Pro Glu Asp Val Lys Met Ala Leu Glu Val Tyr
            20                  25                  30

Lys Leu Ser Leu Glu Ile Glu Gln Leu Glu Leu Gln Arg Asp Ser Ala
        35                  40                  45

Arg Gln Tyr Thr Leu Asp Lys Glu Leu
    50                  55

<210>  19
<211>  57
<212>  PRT
<213>  Artificial Sequence

<220>
<223>  Oligomerization domain of C4BP alpha chain of mammalian ortholog

<400>  19

Glu Thr Pro Glu Gly Cys Glu Gln Val Leu Ala Gly Lys Arg Leu Met
1               5                   10                  15

Gln Cys Leu Pro Asn Pro Glu Asp Val Lys Met Ala Leu Glu Val Tyr
            20                  25                  30

Lys Leu Ser Leu Glu Ile Glu Gln Leu Glu Leu Gln Arg Asp Arg Ala
        35                  40                  45

Arg Gln Ser Thr Leu Asp Lys Glu Leu
    50                  55

<210>  20
<211>  57
<212>  PRT
<213>  Artificial Sequence

<220>
<223>  Oligomerization domain of C4BP alpha chain of mammalian ortholog

<400>  20

Glu Thr Pro Glu Gly Cys Glu Gln Val Leu Ala Gly Lys Arg Leu Met
1               5                   10                  15

Gln Cys Leu Pro Asn Pro Glu Asp Val Lys Met Ala Leu Glu Val Tyr
            20                  25                  30

Lys Leu Ser Leu Glu Ile Glu Gln Leu Glu Leu Gln Arg Asp Arg Ala
        35                  40                  45

Arg Gln Ser Thr Trp Asp Lys Glu Leu
    50                  55

<210>  21
<211>  57
<212>  PRT
<213>  Artificial Sequence

<220>
<223>  oligomerization domain of C4BP alpha chain of mammalian ortholog

<400>  21

Glu Val Pro Glu Gly Cys Glu Gln Val Gln Ala Gly Arg Arg Leu Met
1               5                   10                  15

Gln Cys Leu Ala Asp Pro Tyr Glu Val Lys Met Ala Leu Glu Val Tyr
            20                  25                  30

Lys Leu Ser Leu Glu Ile Glu Leu Leu Glu Leu Gln Arg Asp Lys Ala
        35                  40                  45

Arg Lys Ser Ser Val Leu Arg Gln Leu
    50                  55

<210>  22
<211>  57
<212>  PRT
<213>  Artificial Sequence

<220>
<223>  oligomerization domain of C4BP alpha chain of mammalian ortholog

<400>  22

Val Val Pro Glu Gly Cys Glu His Ile Leu Lys Gly Arg Lys Thr Met
1               5                   10                  15

Gln Cys Leu Pro Asn Pro Glu Asp Val Lys Met Ala Leu Glu Ile Tyr
            20                  25                  30

Lys Leu Ser Leu Asp Ile Glu Leu Leu Glu Leu Gln Arg Asp Arg Ala
        35                  40                  45

Lys Glu Ser Thr Val Gln Ser Pro Val
    50                  55

<210>  23
<211>  57
<212>  PRT
<213>  Artificial Sequence

<220>
<223>  oligomerization domain of C4BP alpha chain of mammalian ortholog

<400>  23

Glu Tyr Pro Glu Asp Cys Glu Gln Val His Glu Gly Lys Lys Leu Met
1               5                   10                  15

Gln Cys Leu Pro Asn Leu Glu Glu Ile Lys Leu Ala Leu Glu Leu Tyr
            20                  25                  30

Lys Leu Ser Leu Glu Thr Lys Leu Leu Glu Leu Gln Ile Asp Lys Glu
        35                  40                  45

Lys Lys Ala Lys Ala Lys Tyr Ser Ile
    50                  55

<210>  24
<211>  57
<212>  PRT
<213>  Artificial Sequence

<220>
<223>  oligomerization domain of C4BP alpha chain of mammalian ortholog

<400>  24

Glu Tyr Pro Glu Asp Cys Glu Gln Val His Glu Gly Lys Lys Leu Met
1               5                   10                  15

Glu Cys Leu Pro Thr Leu Glu Glu Ile Lys Leu Ala Leu Ala Leu Tyr
            20                  25                  30

Lys Leu Ser Leu Glu Thr Asn Leu Leu Glu Leu Gln Ile Asp Lys Glu
        35                  40                  45

Lys Lys Ala Lys Ala Lys Tyr Ser Thr
    50                  55

<210>  25
<211>  57
<212>  PRT
<213>  Artificial Sequence

<220>
<223>  oligomerization domain of C4BP alpha chain of mammalian ortholog

<400>  25

Glu Ile Ala Glu Gly Cys Glu Gln Val Leu Ala Gly Arg Lys Ile Met
1               5                   10                  15

Gln Cys Leu Pro Lys Pro Glu Asp Val Arg Thr Ala Leu Glu Leu Tyr
            20                  25                  30

Lys Leu Ser Leu Glu Ile Lys Gln Leu Glu Lys Lys Leu Glu Lys Glu
        35                  40                  45

Glu Lys Cys Thr Pro Glu Val Gln Glu
    50                  55

<210>  26
<211>  52
<212>  PRT
<213>  Artificial Sequence

<220>
<223>  oligomerization domain of C4BP alpha chain of mammalian ortholog

<400>  26

Glu Tyr Pro Glu Gly Cys Glu Gln Val Val Thr Gly Arg Lys Leu Leu
1               5                   10                  15

Lys Cys Leu Ser Arg Pro Glu Glu Val Lys Leu Ala Leu Glu Val Tyr
            20                  25                  30

Lys Leu Ser Leu Glu Ile Ala Leu Leu Glu Leu Gln Ile Asp Lys Pro
        35                  40                  45

Lys Asp Ala Ser
    50

<210>  27
<211>  52
<212>  PRT
<213>  Artificial Sequence

<220>
<223>  oligomerization domain of C4BP alpha chain of mammalian ortholog

<400>  27

Glu Val Pro Glu Asn Cys Glu Gln Val Ile Val Gly Lys Lys Leu Met
1               5                   10                  15

Lys Cys Leu Ser Asn Pro Asp Glu Ala Gln Met Ala Leu Gln Leu Tyr
            20                  25                  30

Lys Leu Ser Leu Glu Ala Glu Leu Leu Arg Leu Gln Ile Val Lys Ala
        35                  40                  45

Arg Gln Gly Ser
    50

<210>  28
<211>  51
<212>  PRT

<213>  Artificial Sequence

<220>
<223>  oligomerization domain of C4BP alpha chain of mammalian ortholog

<400>  28

Val Ser Ala Glu Val Cys Glu Ala Val Phe Lys Gly Gln Lys Leu Leu
1               5                   10                  15

Lys Cys Leu Pro Asn Ala Met Glu Val Lys Met Ala Leu Glu Val Tyr
            20                  25                  30

Lys Leu Ser Leu Glu Ile Glu Lys Leu Glu Gln Glu Lys Arg Lys Leu
        35                  40                  45

Glu Ile Ala
    50

<210>  29
<211>  66
<212>  PRT
<213>  Artificial Sequence

<220>
<223>  oligomerization domain of C4BP alpha chain of mammalian ortholog

<400>  29

Glu Val Pro Glu Glu Cys Lys Gln Val Ala Ala Gly Arg Lys Leu Leu
1               5                   10                  15

Glu Cys Leu Pro Asn Pro Ser Asp Val Lys Met Ala Leu Glu Val Tyr
            20                  25                  30

Lys Leu Ser Leu Glu Ile Glu Gln Leu Glu Lys Glu Lys Tyr Val Lys
        35                  40                  45

Ile Gln Glu Lys Phe Ser Lys Lys Glu Met Lys Gln Leu Thr Ser Ala
    50                  55                  60

Leu His
65

<210>  30
<211>  59
<212>  PRT
<213>  Artificial Sequence

<220>
<223>  oligomerization domain of C4BP alpha chain of mammalian ortholog

<400>  30

Glu Val Leu Glu Asp Cys Arg Ile Val Ser Arg Gly Ala Gln Leu Leu
1               5                   10                  15

His Cys Leu Ser Ser Pro Glu Asp Val His Arg Ala Leu Lys Val Tyr
            20                  25                  30

Lys Leu Phe Leu Glu Ile Glu Arg Leu Glu His Gln Lys Glu Lys Trp
        35                  40                  45

Ile Gln Leu His Arg Lys Pro Gln Ser Met Lys
    50                  55

<210>  31
<211>  63
<212>  PRT
<213>  Artificial Sequence

<220>
<223>  oligomerization domain of C4BP alpha chain of mammalian ortholog

<400>  31

Glu Gly Pro Glu Asp Cys Glu Ile Val Asn Lys Gly Arg Gln Leu Leu
1               5                   10                  15

Gln Cys Leu Ser Ser Pro Glu Asp Val Gln Arg Ala Leu Glu Val Tyr
            20                  25                  30

Lys Leu Ser Leu Glu Ile Glu Arg Leu Glu Gln Gln Arg Glu Lys Arg
        35                  40                  45

Thr Ser Val His Arg Lys Ala His Tyr Thr Lys Val Asp Gly Pro
    50                  55                  60

<210>  32
<211>  67
<212>  PRT
<213>  Artificial Sequence

<220>
<223>  oligomerization domain of C4BP alpha chain of mammalian ortholog

<400>  32

Glu Ala Pro Glu Gly Cys Glu Gln Val Leu Thr Gly Arg Lys Leu Met
1               5                   10                  15

Gln Cys Leu Pro Ser Pro Glu Asp Val Lys Val Ala Leu Glu Val Tyr
            20                  25                  30

Lys Leu Ser Leu Glu Ile Lys Gln Leu Glu Lys Glu Arg Asp Lys Leu
        35                  40                  45

Met Asn Thr His Gln Lys Phe Ser Glu Lys Glu Glu Met Lys Asp Leu
    50                  55                  60

Phe Phe Pro
65

<210>  33
<211>  52
<212>  PRT
<213>  Artificial Sequence

<220>
<223>  oligomerization domain of C4BP alpha chain of mammalian ortholog

<400>  33

Glu Val Pro Glu Gly Cys Glu Gln Val Leu Thr Gly Lys Lys Leu Met
1               5                   10                  15

Gln Cys Leu Pro Asn Pro Glu Asp Val Lys Met Ala Leu Glu Val Tyr
            20                  25                  30

Lys Leu Ser Leu Glu Ile Glu Leu Leu Glu Leu Gln Ile Asp Lys Ala
        35                  40                  45

Arg Gln Gly Ser
    50

<210>  34
<211>  47
<212>  PRT
<213>  Artificial Sequence

<220>
<223>  Functionally equivalent variant of the oligomerization domain of
       C4BP alpha chain

<400>  34

Gly Cys Glu Gln Val Leu Thr Gly Lys Arg Leu Met Gln Cys Leu Pro
1               5                   10                  15

Asn Pro Glu Asp Val Lys Met Ala Leu Glu Val Tyr Lys Leu Ser Leu
            20                  25                  30

Glu Ile Glu Gln Leu Glu Leu Gln Arg Asp Ser Ala Arg Gln Ser
        35                  40                  45

<210>  35
<211>  51
<212>  PRT
<213>  Artificial Sequence

<220>
<223>  Functionally equivalent variant of the oligomerization domain of
      C4BP alpha chain

<400>  35

Glu Thr Pro Glu Gly Cys Glu Gln Val Leu Thr Gly Lys Arg Leu Met
1               5                   10                  15

Gln Glu Leu Pro Asn Pro Glu Asp Val Lys Met Ala Leu Glu Val Tyr
            20                  25                  30

Lys Leu Ser Leu Glu Ile Glu Gln Leu Glu Leu Gln Arg Asp Ser Ala
        35                  40                  45

Arg Gln Ser
    50

<210>  36
<211>  53
<212>  PRT
<213>  Artificial Sequence

<220>
<223>  Functionally equivalent variant of the oligomerization domain of
      C4BP alpha chain

<400>  36

Gly Ser Glu Gln Val Leu Thr Gly Lys Arg Leu Met Gln Ser Leu Pro
1               5                   10                  15

Asn Pro Glu Asp Val Lys Met Ala Leu Glu Val Tyr Lys Leu Ser Leu
            20                  25                  30

Glu Ile Glu Gln Leu Glu Leu Gln Arg Asp Ser Ala Arg Gln Ser Thr
        35                  40                  45

Leu Asp Lys Glu Leu
    50

<210>  37
<211>  57
<212>  PRT
<213>  Artificial Sequence

<220>
<223>  Functionally equivalent variant of the oligomerization domain of
      C4BP alpha chain

<400>  37

Glu Thr Pro Glu Gly Cys Glu Gln Val Leu Thr Gly Lys Arg Leu Met
1               5                   10                  15

Gln Glu Leu Pro Asn Pro Glu Asp Val Lys Met Ala Leu Glu Ile Tyr
            20                  25                  30

Lys Leu Thr Leu Glu Ile Glu Gln Leu Glu Leu Gln Arg Asp Ser Ala
        35                  40                  45

Arg Gln Ser Thr Leu Asp Lys Glu Leu
    50                  55

<210>  38
<211>  57
<212>  PRT
<213>  Artificial Sequence

<220>
<223>  Functionally equivalent variant of the oligomerization domain of
       C4BP alpha chain

<400>  38

Glu Thr Pro Glu Gly Cys Glu Gln Val Leu Thr Gly Lys Arg Leu Met
1               5                   10                  15

Gln Glu Leu Pro Asn Pro Glu Asp Val Lys Met Ala Leu Glu Ile Tyr
            20                  25                  30

Lys Leu Ser Leu Glu Ile Lys Gln Leu Glu Leu Gln Arg Asp Ser Ala
        35                  40                  45

Arg Gln Ser Thr Leu Asp Lys Glu Leu
    50                  55

<210>  39
<211>  50
<212>  PRT
<213>  Artificial Sequence

<220>
<223>  Functionally equivalent variant of the oligomerization domain of
       C4BP alpha chain

<400>  39

Glu Gly Cys Glu Gln Ile Leu Thr Gly Lys Arg Leu Met Gln Glu Leu
1               5                   10                  15

Pro Asp Pro Glu Asp Val Lys Met Ala Leu Glu Ile Tyr Lys Leu Ser
            20                  25                  30

Leu Glu Ile Lys Gln Leu Glu Leu Gln Arg Asp Arg Ala Arg Gln Ser
        35                  40                  45

Thr Leu
    50

<210>  40
<211>  57
<212>  PRT
<213>  Artificial Sequence

<220>
<223>  Functionally equivalent variant of the oligomerization domain of
       C4BP alpha chain

<400>  40

Glu Thr Pro Glu Gly Cys Glu Gln Val Leu Thr Gly Lys Arg Leu Met
1               5                   10                  15

Gln Glu Leu Pro Asn Pro Glu Asp Val Lys Met Ala Leu Glu Val Tyr
            20                  25                  30

Lys Leu Ser Leu Glu Ile Lys Gln Leu Glu Leu Gln Arg Asp Arg Ala
        35                  40                  45

Arg Gln Ser Thr Leu Asp Lys Glu Leu
    50                  55

<210>  41
<211>  52
<212>  PRT
<213>  Artificial Sequence

<220>
<223>  Functionally equivalent variant of the oligomerization domain of
       C4BP alpha chain

<400>  41

Glu Gly Cys Glu Gln Ile Leu Thr Gly Lys Arg Leu Met Gln Glu Leu
1               5                   10                  15

Pro Asn Pro Glu Asp Val Lys Met Ala Leu Glu Ile Tyr Lys Leu Ser
            20                  25                  30

Leu Glu Ile Glu Gln Leu Glu Leu Gln Arg Asp Arg Ala Arg Gln Ser
        35                  40                  45

Thr Leu Asp Lys
    50

<210>  42
<211>  62
<212>  PRT
<213>  Gallus gallus

<400>  42

Lys Lys Gln Gly Asp Ala Asp Val Cys Gly Glu Val Ala Tyr Ile Gln
1               5                   10                  15

Ser Val Val Ser Asp Cys His Val Pro Thr Glu Asp Val Lys Thr Leu
            20                  25                  30

Leu Glu Ile Arg Lys Leu Phe Leu Glu Ile Gln Lys Leu Lys Val Glu
        35                  40                  45

Leu Gln Gly Leu Ser Lys Glu Phe Leu Glu His Ile Leu His
    50                  55                  60

<210>  43
<211>  50
<212>  PRT
<213>  Taeniopygia guttata

<400>  43

Met Lys Glu Gly Asp Gly Asp Val Cys Gln Glu Val His Tyr Ile Lys
1               5                   10                  15

Ser Thr Phe Glu Cys Gly Val Pro Val Glu Glu Val Lys Ile Leu Leu
            20                  25                  30

Glu Ile Gln Lys Leu Leu Leu Glu Ile Asn Lys Leu Glu Met Glu Leu
        35                  40                  45

Glu Asn
    50

<210>  44
<211>  62
<212>  PRT
<213>  Artificial Sequence

<220>
<223>  Functionally equivalent variant of the oligomerization domain of
       C4BP

<400>  44

Lys Lys Gln Gly Asp Ala Asp Val Cys Gly Glu Val Ala Tyr Ile Gln
1               5                   10                  15

Ser Val Val Ser Asp Cys His Val Pro Thr Glu Asp Val Lys Thr Leu
            20                  25                  30

Leu Glu Val Arg Lys Leu Phe Leu Glu Ile Gln Lys Leu Lys Val Glu
        35                  40                  45

Leu Gln Gly Leu Ser Lys Glu Phe Leu Glu His Ile Leu His
    50                  55                  60

<210>  45
<211>  62
<212>  PRT
<213>  Artificial Sequence

<220>
<223>  Functionally equivalent variant of the oligomerization domain of
       C4BP

<400>  45

Lys Lys Gln Gly Asp Ala Asp Val Cys Gly Glu Val Ala Tyr Ile Gln
1               5                   10                  15

Ser Val Val Ser Asp Cys His Val Pro Thr Glu Asp Val Lys Met Leu
            20                  25                  30

Leu Glu Val Arg Lys Leu Phe Leu Glu Ile Gln Lys Leu Lys Val Glu
        35                  40                  45

Leu Gln Gly Leu Ser Lys Glu Phe Leu Glu His Ile Leu His
    50                  55                  60

<210>  46
<211>  62
<212>  PRT
<213>  Artificial Sequence

<220>
<223>  Functionally equivalent variant of the oligomerization domain of
       C4BP

<400>  46

Lys Lys Gln Gly Asp Ala Asp Val Cys Gly Glu Val Ile Tyr Ile Gln
1               5                   10                  15

Ser Val Val Ser Asp Cys His Val Pro Thr Glu Asp Val Arg Thr Leu
            20                  25                  30

Leu Glu Ile Arg Lys Leu Phe Leu Glu Ile Gln Lys Leu Lys Val Glu
        35                  40                  45

Leu Gln Gly Leu Ser Lys Glu Phe Leu Glu His Ile Leu His
    50                  55                  60

<210>  47
<211>  62
<212>  PRT
<213>  Artificial Sequence

<220>
<223>  Functionally equivalent variant of the oligomerization domain of
      C4BP

<400>  47

Lys Lys Gln Gly Asp Ala Asp Val Cys Gly Glu Val Ile Tyr Ile Gln
1               5                   10                  15

Ser Val Val Ser Asp Cys His Val Pro Thr Glu Asp Val Lys Thr Leu
            20                  25                  30

Leu Glu Ile Arg Lys Leu Phe Leu Glu Ile Gln Lys Leu Lys Val Glu
        35                  40                  45

Leu Asn Gly Leu Ser Lys Glu Phe Leu Glu His Ile Leu His
    50                  55                  60

<210>  48
<211>  62
<212>  PRT
<213>  Artificial Sequence

<220>
<223>  Functionally equivalent variant of the oligomerization domain of
      C4BP

<400>  48

Lys Lys Gln Gly Asp Ala Asp Val Cys Gly Glu Val Ile Tyr Ile Gln
1               5                   10                  15

Ser Val Val Ser Asp Cys His Val Pro Thr Glu Asp Val Lys Met Leu
            20                  25                  30

Leu Glu Ile Arg Lys Leu Phe Leu Glu Ile Gln Lys Leu Lys Val Glu
        35                  40                  45

Leu Asn Gly Leu Ser Lys Glu Phe Leu Glu His Ile Leu His
    50                  55                  60

<210>  49
<211>  62
<212>  PRT
<213>  Artificial Sequence

<220>
<223>  Functionally equivalent variant of the oligomerization domain of
      C4BP

<400>  49

Lys Lys Gln Gly Asp Ala Asp Val Cys Gly Glu Val Ile Tyr Ile Gln
1               5                   10                  15

Ser Val Val Ser Asp Cys Leu Pro Asn Thr Glu Asp Val Lys Thr Leu
            20                  25                  30

Leu Glu Val Arg Lys Leu Phe Leu Glu Ile Gln Lys Leu Lys Val Glu
        35                  40                  45

Leu Gln Gly Leu Ser Lys Glu Phe Leu Glu His Ile Leu His
    50                  55                  60

<210>  50
<211>  54
<212>  PRT
<213>  Artificial Sequence

<220>
<223>  Functionally equivalent variant of the oligomerization domain of
       C4BP

<400>  50

Asp Ala Asp Val Cys Gly Glu Val Ala Tyr Ile Gln Ser Val Val Ser
1               5                   10                  15

Asp Cys His Val Pro Thr Glu Asp Val Lys Thr Leu Leu Glu Ile Arg
            20                  25                  30

Lys Leu Phe Leu Glu Ile Gln Lys Leu Lys Val Glu Leu Gln Gly Leu
        35                  40                  45

Ser Lys Glu Phe Leu Glu
    50

<210>  51
<211>  51
<212>  PRT
<213>  Artificial Sequence

<220>
<223>  Functionally equivalent variant of the oligomerization domain of
       C4BP

<400>  51

Ala Asp Val Cys Gly Glu Val Ala Tyr Ile Gln Ser Val Val Ser Asp
1               5                   10                  15

Cys His Val Pro Thr Glu Asp Val Lys Thr Leu Leu Glu Ile Arg Lys
            20                  25                  30

Leu Phe Leu Glu Ile Gln Lys Leu Lys Val Glu Leu Gln Gly Leu Ser
        35                  40                  45

Lys Glu Phe
    50

<210>  52
<211>  51
<212>  PRT
<213>  Artificial Sequence

<220>
<223>  Functionally equivalent variant of the oligomerization domain of
       C4BP

<400>  52

Ala Asp Val Cys Gly Glu Val Ile Tyr Ile Gln Ser Val Val Ser Asp
1               5                   10                  15

Cys His Val Pro Thr Glu Asp Val Lys Thr Leu Leu Glu Val Arg Lys
            20                  25                  30

Leu Phe Leu Glu Ile Gln Lys Leu Lys Val Glu Leu Gln Gly Leu Ser
        35                  40                  45

Lys Glu Phe
    50

<210>  53
<211>  48
<212>  PRT
<213>  Artificial Sequence

<220>
<223>  Functionally equivalent variant of the oligomerization domain of
       C4BP

<400>  53

Ala Asp Val Cys Gly Glu Val Ala Tyr Ile Gln Ser Val Val Ser Asp
1               5                   10                  15

Cys His Val Pro Thr Glu Asp Val Lys Thr Leu Leu Glu Ile Arg Lys
            20                  25                  30

Leu Phe Leu Glu Ile Gln Lys Leu Lys Val Glu Leu Asn Gly Leu Ser
        35                  40                  45

<210>  54
<211>  55
<212>  PRT
<213>  Artificial Sequence

<220>
<223>  Functionally equivalent variant of the oligomerization domain of
       C4BP

<400>  54

Lys Lys Gln Gly Asp Ala Asp Val Cys Gly Glu Val Ala Tyr Ile Gln
1               5                   10                  15

Ser Val Val Ser Asp Cys His Val Pro Thr Glu Asp Val Lys Thr Leu
            20                  25                  30

Leu Glu Ile Arg Lys Leu Phe Leu Glu Ile Gln Lys Leu Lys Val Glu
        35                  40                  45

Leu Gln Gly Leu Ser Lys Glu
    50                  55

<210>  55
<211>  55
<212>  PRT
<213>  Artificial Sequence

<220>
<223>  Functionally equivalent variant of the oligomerization domain of
       C4BP

<400>  55

Lys Lys Gln Gly Asp Ala Asp Val Cys Gly Glu Val Ala Tyr Ile Gln
1               5                   10                  15

Ser Val Val Ser Asp Cys His Val Pro Thr Ala Glu Leu Arg Thr Leu
            20                  25                  30

Leu Glu Ile Arg Lys Leu Phe Leu Glu Ile Gln Lys Leu Lys Val Glu
        35                  40                  45

Leu Gln Gly Leu Ser Lys Glu
    50                  55

<210>  56
<211>  180
<212>  PRT
<213>  Artificial Sequence

<220>
<223>  Recombinant polypeptide

<400>  56

Met His Pro Pro Lys Thr Pro Ser Gly Ala Leu His Arg Lys Arg Lys
1               5                   10                  15

Met Ala Ala Trp Pro Phe Ser Arg Leu Trp Lys Val Ser Asp Pro Ile
            20                  25                  30

Leu Phe Gln Met Thr Leu Ile Ala Ala Leu Leu Pro Ala Val Leu Gly
        35                  40                  45

His His His His His His Glu Asn Leu Tyr Phe Gln Gly Leu Cys Cys
    50                  55                  60

Pro Glu Pro Lys Leu Asn Asn Gly Glu Ile Thr Gln His Arg Lys Ser
65                  70                  75                  80

Arg Pro Ala Asn His Cys Val Tyr Phe Tyr Gly Asp Glu Ile Ser Phe
                85                  90                  95

Ser Cys His Glu Thr Ser Arg Phe Ser Ala Ile Cys Gln Gly Asp Gly
            100                 105                 110

Thr Trp Ser Pro Arg Thr Pro Ser Cys Gly Asp Glu Thr Pro Glu Gly
        115                 120                 125

Cys Glu Gln Val Leu Thr Gly Lys Arg Leu Met Gln Cys Leu Pro Asn
    130                 135                 140

Pro Glu Asp Val Lys Met Ala Leu Glu Val Tyr Lys Leu Ser Leu Glu
145                 150                 155                 160

Ile Glu Gln Leu Glu Leu Gln Arg Asp Ser Ala Arg Gln Ser Thr Leu
                165                 170                 175

Asp Lys Glu Leu
            180

<210>  57
<211>  132
<212>  PRT
<213>  Artificial Sequence

<220>
<223>  Recombinant polypeptide

<400>  57

His His His His His His Glu Asn Leu Tyr Phe Gln Gly Leu Cys Cys
1               5                   10                  15

Pro Glu Pro Lys Leu Asn Asn Gly Glu Ile Thr Gln His Arg Lys Ser
            20                  25                  30

Arg Pro Ala Asn His Cys Val Tyr Phe Tyr Gly Asp Glu Ile Ser Phe
        35                  40                  45

Ser Cys His Glu Thr Ser Arg Phe Ser Ala Ile Cys Gln Gly Asp Gly
    50                  55                  60

Thr Trp Ser Pro Arg Thr Pro Ser Cys Gly Asp Glu Thr Pro Glu Gly
65                  70                  75                  80

Cys Glu Gln Val Leu Thr Gly Lys Arg Leu Met Gln Cys Leu Pro Asn
                85                  90                  95

Pro Glu Asp Val Lys Met Ala Leu Glu Val Tyr Lys Leu Ser Leu Glu
            100                 105                 110

Ile Glu Gln Leu Glu Leu Gln Arg Asp Ser Ala Arg Gln Ser Thr Leu
        115                 120                 125

Asp Lys Glu Leu
    130

<210>  58
<211>  120
<212>  PRT
<213>  Artificial Sequence

<220>
<223>  Recombinant polypeptide

<400>  58

Gly Leu Cys Cys Pro Glu Pro Lys Leu Asn Asn Gly Glu Ile Thr Gln
1               5                   10                  15

His Arg Lys Ser Arg Pro Ala Asn His Cys Val Tyr Phe Tyr Gly Asp
            20                  25                  30

Glu Ile Ser Phe Ser Cys His Glu Thr Ser Arg Phe Ser Ala Ile Cys
        35                  40                  45

Gln Gly Asp Gly Thr Trp Ser Pro Arg Thr Pro Ser Cys Gly Asp Glu
    50                  55                  60

Thr Pro Glu Gly Cys Glu Gln Val Leu Thr Gly Lys Arg Leu Met Gln
65                  70                  75                  80

Cys Leu Pro Asn Pro Glu Asp Val Lys Met Ala Leu Glu Val Tyr Lys
                85                  90                  95

Leu Ser Leu Glu Ile Glu Gln Leu Glu Leu Gln Arg Asp Ser Ala Arg
            100                 105                 110

Gln Ser Thr Leu Asp Lys Glu Leu

115                 120


<210>  59
<211>  5
<212>  PRT
<213>  Artificial Sequence

<220>
<223>  Cleavable linker

<400>  59

Asp Asp Asp Asp Lys
1               5


<210>  60
<211>  8
<212>  PRT
<213>  Artificial Sequence

<220>
<223>  Strep-tag II peptide

<400>  60

Trp Ser His Pro Gln Phe Glu Lys
1               5


<210>  61
<211>  139
<212>  PRT
<213>  Artificial Sequence

<220>
<223>  Recombinant polypeptide

<400>  61

Met His His His His His His Trp Ser His Pro Gln Phe Glu Lys Asp
1               5                   10                  15


Asp Asp Asp Lys Leu Cys Cys Pro Glu Pro Lys Leu Asn Asn Gly Glu
            20                  25                  30


Ile Thr Gln His Arg Lys Ser Arg Pro Ala Asn His Cys Val Tyr Phe
        35                  40                  45


Tyr Gly Asp Glu Ile Ser Phe Ser Cys His Glu Thr Ser Arg Phe Ser
    50                  55                  60


Ala Ile Cys Gln Gly Asp Gly Thr Trp Ser Pro Arg Thr Pro Ser Cys
65                  70                  75                  80


Gly Asp Glu Thr Pro Glu Gly Cys Glu Gln Val Leu Thr Gly Lys Arg
                85                  90                  95

Leu Met Gln Cys Leu Pro Asn Pro Glu Asp Val Lys Met Ala Leu Glu
            100                 105                 110

Val Tyr Lys Leu Ser Leu Glu Ile Glu Gln Leu Glu Leu Gln Arg Asp
        115                 120                 125

Ser Ala Arg Gln Ser Thr Leu Asp Lys Glu Leu
    130                 135

<210>  62
<211>  89
<212>  PRT
<213>  Saccharomyces cerevisiae

<400>  62

Met Arg Phe Pro Ser Ile Phe Thr Ala Val Leu Phe Ala Ala Ser Ser
1               5                   10                  15

Ala Leu Ala Ala Pro Val Asn Thr Thr Thr Glu Asp Glu Thr Ala Gln
            20                  25                  30

Ile Pro Ala Glu Ala Val Ile Gly Tyr Leu Asp Leu Glu Gly Asp Phe
        35                  40                  45

Asp Val Ala Val Leu Pro Phe Ser Asn Ser Thr Asn Asn Gly Leu Leu
    50                  55                  60

Phe Ile Asn Thr Thr Ile Ala Ser Ile Ala Ala Lys Glu Glu Gly Val
65                  70                  75                  80

Ser Leu Asp Lys Arg Glu Ala Glu Ala
                85

<210>  63
<211>  214
<212>  PRT
<213>  Artificial Sequence

<220>
<223>  Recombinant polypeptide

<400>  63

Met Arg Phe Pro Ser Ile Phe Thr Ala Val Leu Phe Ala Ala Ser Ser
1               5                   10                  15

Ala Leu Ala Ala Pro Val Asn Thr Thr Thr Glu Asp Glu Thr Ala Gln
            20                  25                  30

Ile Pro Ala Glu Ala Val Ile Gly Tyr Leu Asp Leu Glu Gly Asp Phe
        35                  40                  45

Asp Val Ala Val Leu Pro Phe Ser Asn Ser Thr Asn Asn Gly Leu Leu
    50                  55                  60

Phe Ile Asn Thr Thr Ile Ala Ser Ile Ala Ala Lys Glu Glu Gly Val
65                  70                  75                  80

Ser Leu Asp Lys Arg Glu Ala Glu Ala His His His His His His Leu
                85                  90                  95

Cys Cys Pro Glu Pro Lys Leu Asn Asn Gly Glu Ile Thr Gln His Arg
            100                 105                 110

Lys Ser Arg Pro Ala Asn His Cys Val Tyr Phe Tyr Gly Asp Glu Ile
        115                 120                 125

Ser Phe Ser Cys His Glu Thr Ser Arg Phe Ser Ala Ile Cys Gln Gly
    130                 135                 140

Asp Gly Thr Trp Ser Pro Arg Thr Pro Ser Cys Gly Asp Glu Thr Pro
145                 150                 155                 160

Glu Gly Cys Glu Gln Val Leu Thr Gly Lys Arg Leu Met Gln Cys Leu
                165                 170                 175

Pro Asn Pro Glu Asp Val Lys Met Ala Leu Glu Val Tyr Lys Leu Ser
            180                 185                 190

Leu Glu Ile Glu Gln Leu Glu Leu Gln Arg Asp Ser Ala Arg Gln Ser
        195                 200                 205

Thr Leu Asp Lys Glu Leu
    210